**Europäisches Patentamt**

**European Patent Office**

**Office européen des brevets**

(19)

(11) Publication number: **0 080 821**

**A2**

(12)

# EUROPEAN PATENT APPLICATION

(21) Application number: **82306076.9**

(22) Date of filing: **15.11.82**

(51) Int. Cl.³: **G 06 F 15/42**

(30) Priority: **16.11.81 US 322001**

(43) Date of publication of application:
**08.06.83 Bulletin 83/23**

(84) Designated Contracting States:
**AT CH DE FR GB LI**

(71) Applicant: **DATAMEDIX, INC.**
**Arvida Park of Commerce 1001 N.W. 58th Street**
**Boca Raton Florida 33431(US)**

(72) Inventor: **Johnson, Robert Joel**
**2955 N. E. 19th Street**
**Pompano Beach Florida 33062(US)**

(72) Inventor: **Schell, James Joseph**
**400 S. E. 5th Terrace**
**Pompano Beach Florida 33060(US)**

(72) Inventor: **Van Campen, George**
**2841 N. E. 24th Street**
**Fort Lauderdale Florida 33305(US)**

(72) Inventor: **Mount, Joseph Franklin**
**2250 Cherry Palm Drive**
**Boca Raton Florida 33432(US)**

(74) Representative: **Caro, William Egerton et al,**
**J. MILLER & CO. Lincoln House 296-302 High Holborn**
**London WC1V 7JH(GB)**

(54) **Dual channel ambulatory monitoring unit and system.**

(57) A heart analyzing system for an ambulatory patient including a fixed base procedure control system and a dockable programmable dual channel ambulatory satellite monitoring unit (10) connectable to an ambulatory patient and connectable to the procedure control unit (30). The ambulatory monitoring unit includes a microprocessor with QRS classification criteria capabilities for analysis and classification of QRS events and including ST level analysis capabilities to provide ST level. The dual channel ambulatory monitoring unit also includes heart pacer spike detecting and control circuits for use of the ambulatory monitoring unit on heart pacer patients.

./...

Croydon Printing Company Ltd.

Fig. 1.

"DUAL CHANNEL AMBULATORY MONITORING UNIT AND SYSTEM"

This invention relates to a dual channel ambulatory monitoring unit and system with QRS classification method ST segment measurement and heart pacer spike detection and control.

In the past ambulatory monitoring units and systems have been sold utilizing a single channel approach. The advantages of a dual channel ambulatory monitoring unit and systems improvements will be apparent through the disclosure herebelow.

According to one aspect of the present invention there is provided a dual channel patient monitoring system for monitoring ECG signals of an ambulatory patient comprising: an ambulatory container connectable to a patient, a monitoring means connected to said container, said monitoring means for monitoring of ambulatory patient output signals, real-time analysis of said signals and storage of analyzed signals for indepth reporting of said monitored and analyzed ambulatory patient output signals, said monitoring means includes power means for supplying power to said monitoring means, acquisition and conditioning means for acquiring and conditioning the ambulatory patient output signals to provide conditioned output signals, said acquisition and conditioning means connected to said power means, said acquisition and conditioning means including dual channel connection means, connected to the ambulatory patient for providing improved analysis and output data, detection and management means for detecting and managing the conditioned output signals for identification, said detection and management means connected to said acquisition and conditioning means, identification means for identifying particular conditioned output signals, said identification means connected to said detection and management means, pattern classification means for classifying particular patterns and change in the particular conditioned output signals in real-time, said pattern classification means connected to said identification means, storage means for storing the classified patterns and changes, said storage means connected to said pattern classification means; output means for providing output signals from said storage means, said output means connected to said storage means.

According to another aspect of the present invention there is provided a patient monitoring system for monitoring ECG signals of an ambulatory patient comprising: an ambulatory container connectable to a patient, a monitoring means connected to said container, said monitoring means for monitoring of ambulatory patient output signals, real-time analysis of said signals and storage of analyzed signals for indepth reporting of said monitored and analyzed ambulatory patient output signals, said monitoring means includes power means for supplying power to said monitoring means, acquisition and conditioning means for acquiring and conditioning the ambulatory patient output signals to provide conditioned output signals, said acquisition and conditioning means connected to said power means, connectable to the ambulatory patient, detection and management means for detecting and managing the conditioned output signals for identification, said detection and management means connected to said acquisition and conditioning means, identification means for identifying particular conditioned output signals, said identification means connected to said detection and management means, pattern classification means for classifying particular patterns and change in the particular conditioned output signals in real-time, said pattern classification means connected to said pattern classification means; said pattern classification means including QRS classification criteria means for providing real time QRS classification output, said QRS classification criteria means connected to said identification means, and said storage means, and output means for providing output signals from said storage means, said output means connected to said storage means.

The invention is illustrated, merely by way of example in the accompanying drawings.

Figure 1 is a block drawing of the AMU.

Figure 2 is an illustration of the individual connected to the dual channel AMU by leads.

Figure 3 is an illustration of the AMU, PCU and Graphic Report Printer.

Figure 4 is a PCU block diagram.

Figures 5A, 5B, 5C, 5D and 5E are the front end of the AMU dual channel circuit.

Figures 6A, 6B, 6C, 6D and 6E are the dual channel circuit board in the AMU.

Figures 7A, 7B, and 7C are the PCU interface Analog Dual Channel Circuit.

Figures 8A through 8K are timing charts of the procedure control system of a one channel system.

Figures 9A through 9N are charts and reports of the dual channel system with heart pacer spike detection and control.

- 5 -

0080821

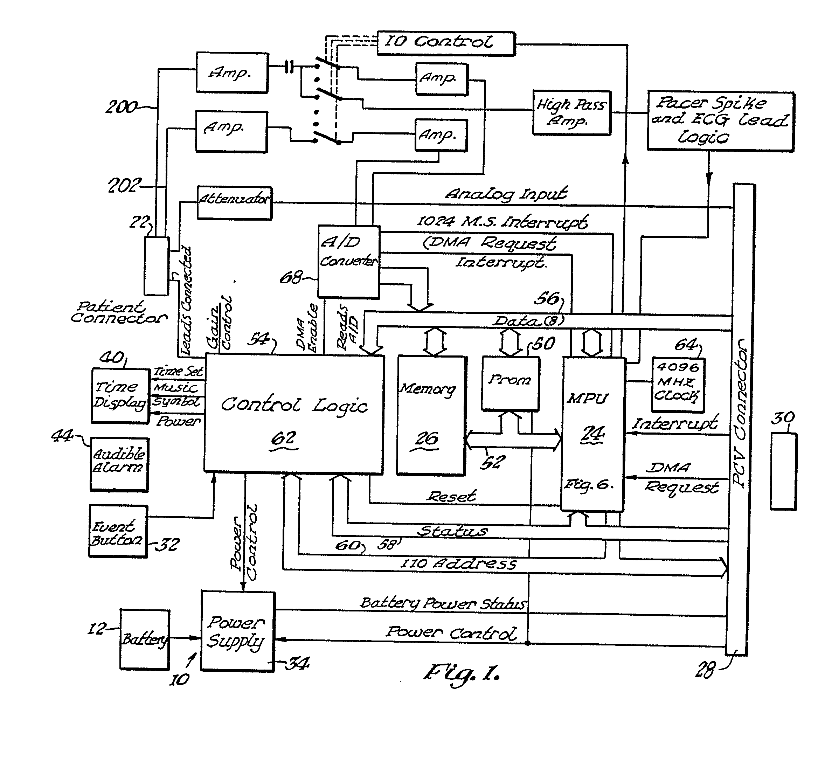

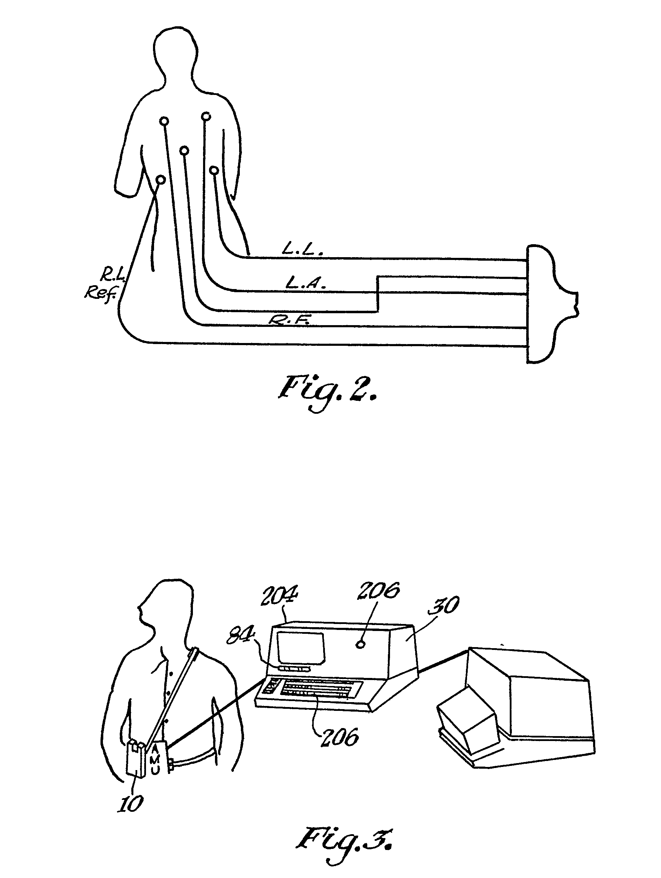

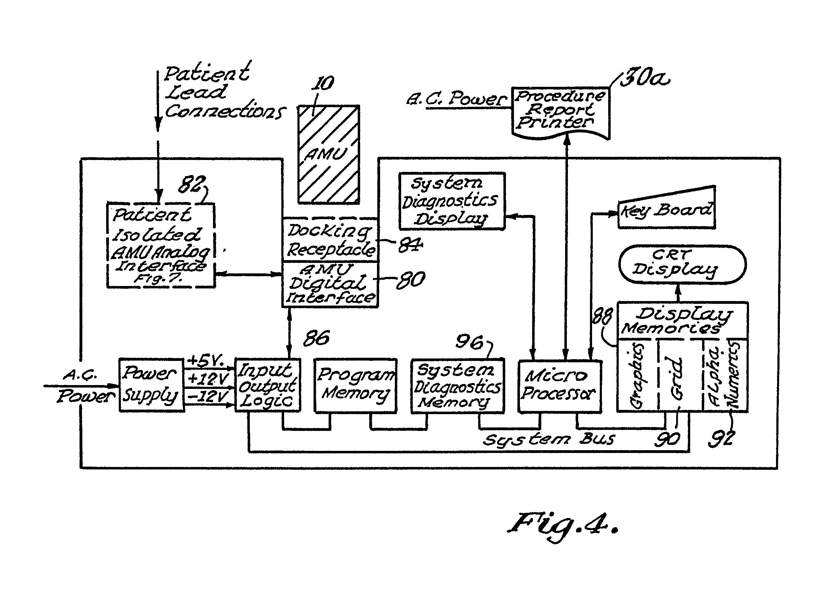

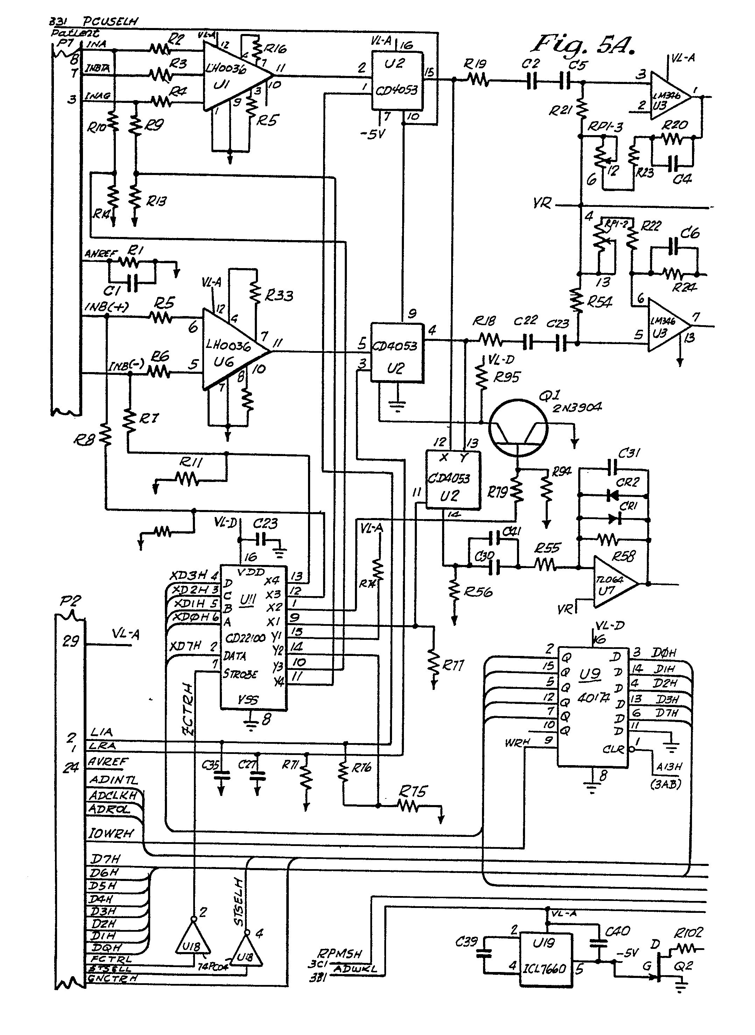

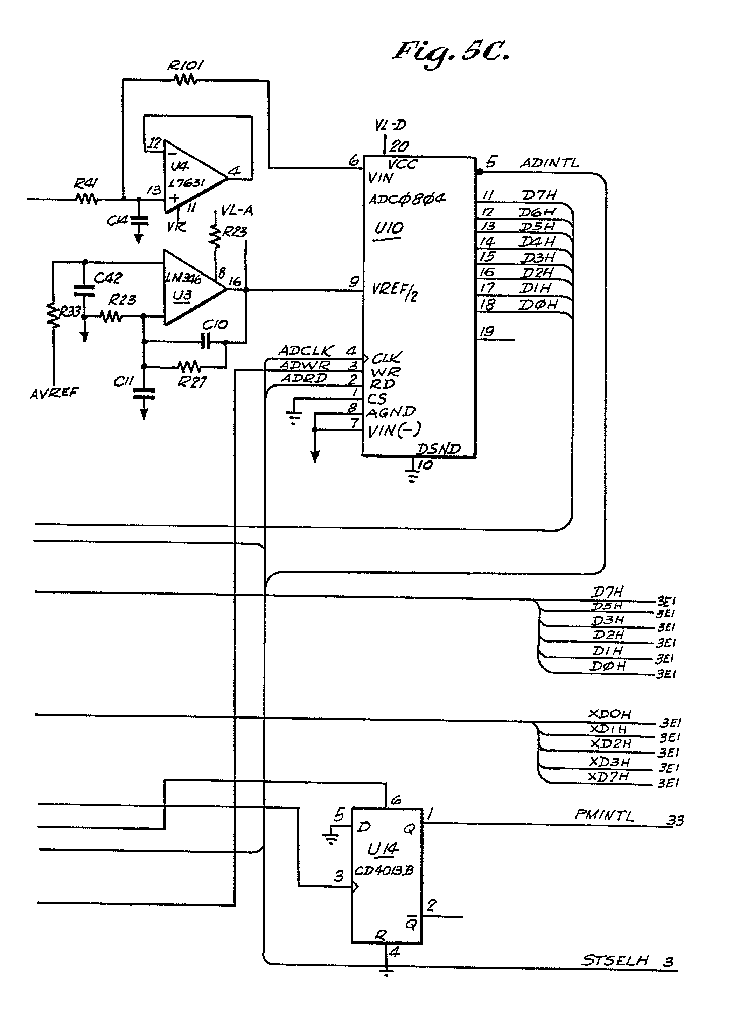

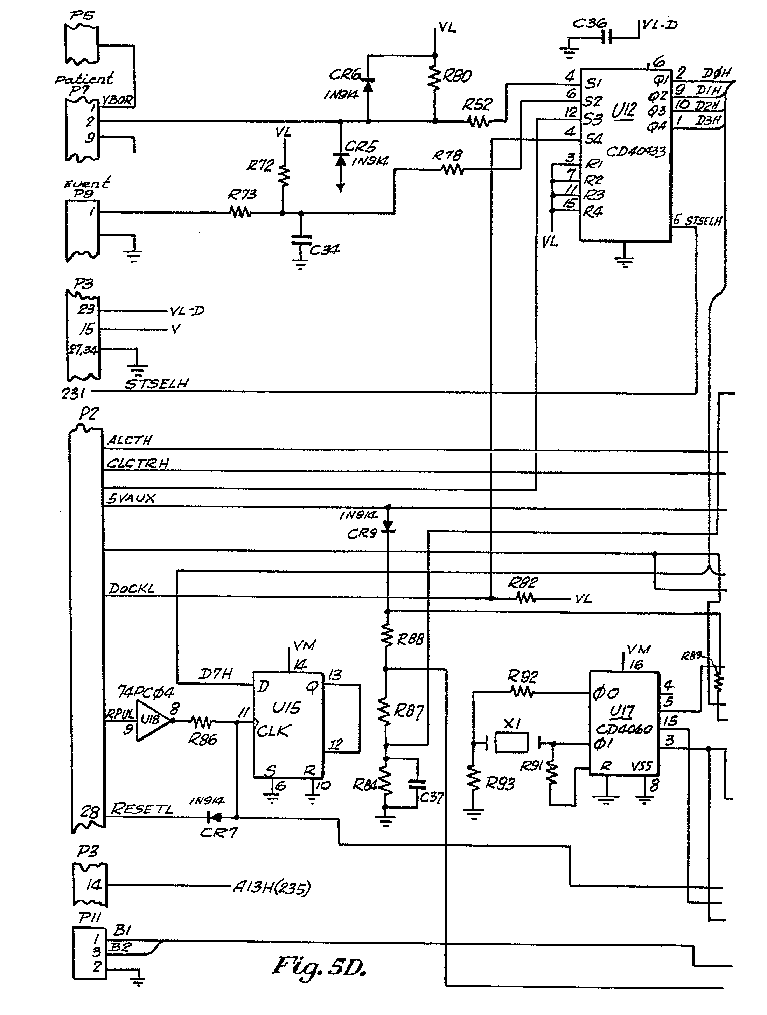

Referring now to the drawings the Two Channel Ambulatory Monitoring system monitors shown in Figures 1, analyzes and produces reports on patient ECG signals taken from a two lead electrode configuration, see Figure 2 and 5 A,B,C,D,E, and F. The monitoring and analysis is performed by a small, lightweight ambulatory computer system designated as the Ambulatory Monitoring Unit or AMU, see Figures 1 and 3. The operation of the AMU is initiated by the Procedure Control Unit or PCU 30 shown in Figure 2. The report from the Ambulatory Monitoring Procedure is formatted by the PCU and generated into a report using the Graphic Report Printer or GRP 30A.

Thus, the Two Channel Ambulatory Monitoring system consits of three basic components, the AMU, the PCU and the GRP, all of which are disclosed in detail in co-pending application Serial No. entitled Medical Monitor, filed January 15, 1981 incorporated herein by referekce as part of this specification. The ECG analysis performed by the AMU is designed to detect changes in rate, irregularity and shape of QRS complexes. Such changes are categorized into a number of QRS activity classifications: Rate ranges, single QRS and RR interval classifications, QRS sequence classifications, and major pattern changes.

The Ambulatory Monitoring Unit provides for pacemaker and pacemaker/cardiac activity classifications.

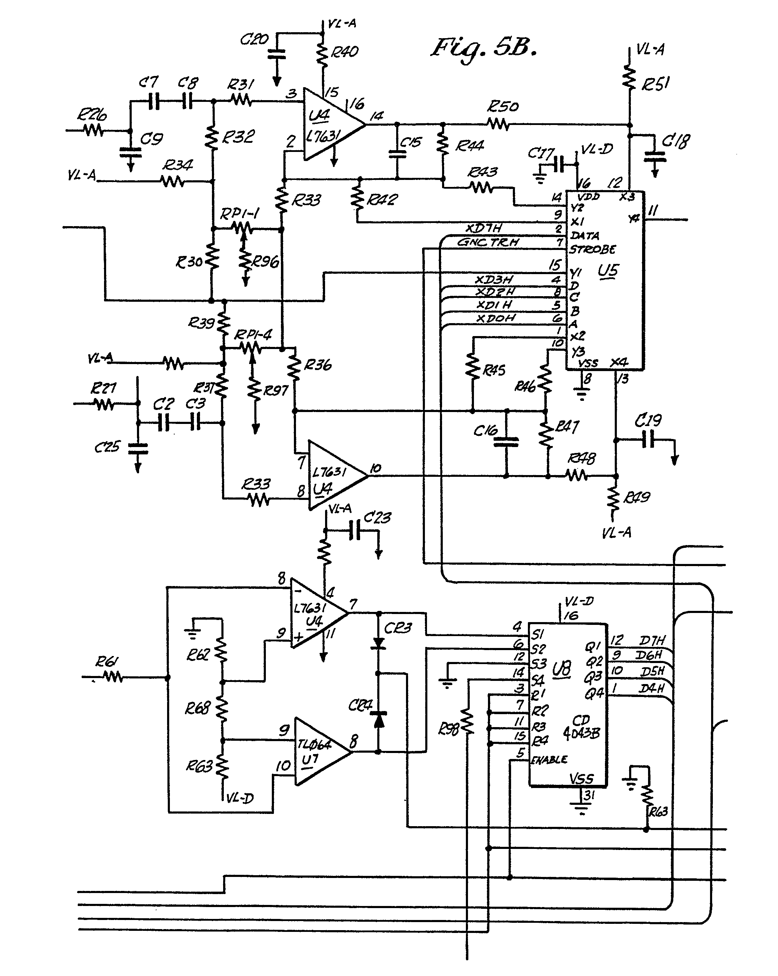

The user interaction with the system is accomplished through the Procedure Control Unit 30 shown in Figures 3 and 4 using a series of video screens. These screens contain prompting information and provide for user selection of system services. The user may define customized procedures for use with the Ambulatory Monitoring Unit. These customized procedures can subsequently be invoked when the Ambulatory Monitoring Unit is initialized. The user may specify the content of reports which are generated and may also review and edit report data on the video screen either before or subsequent to printed report generation.

The Ambulatory Monitoring Unit 10 makes use of two independent channels 200 and 202 of ECG data. Through the use impedance measuring equipment in the AMU, it is possible for the unit to determine that the patient leads are properly connected. In the event of poor connections or loose leads, the AMU can suspend analysis of ECG signals from a given channel. By verifying ECG activity in both channels the Ambulatory Monitoring Unit can achieve higher reliability of its QRS classifications than is possible through the use of a single channel.

The Ambulatory Monitoring Unit contains a liquid crystal time display 40 for patient convenience in maintaining an activity diary. The Ambulatory Monitoring Unit also contains a Patient Marker Button 32 for indications of patient symptoms. The Ambulatory Monitoring Unit logs depressions for the Patient Marker Button for patients in the physician's report. Additionally, the Ambulatory Monitoring Unit contains audio tone generator which is used for indicating to the patient that the AMU has terminated the monitoring procedure or that it is having difficulty in analyzing the signals received from the patient.

The Graphic Report Printer 30A is contained in an enclosure separate from the Procedure Control Unit 30. It produces printed pages which are cut to 8-1/2 x 11". The printing quality is such that it can accurately portray ECG signals.

The Procedure Control Unit provides a video screen 204 for prompting and data presentation to the user.  It provides a keyboard 206 for data entry and user control and response.  It also contains a docking port 84 for the Ambulatory Monitoring Unit as well as a receptacle 206 for the ECG cable connector.

The Ambulatory Monitoring Unit 10 referred to hereafter as the AMU is a battery operated, 24 hour ambulatory heart monitor.  It monitors the patients ECG activity via a dual channel 200 and 202 (Lead V5 and modified Lead 2) connection employing five patient leads.

The AMU as shwon in Figure 3 has a digital microprocessor and a 64K byte random access memory incorporated.  The microprocessing unit 24 (MPU) continuously analyzes the patients heart activity during the monitoring period. When abnormal activity is detected, the MPU stores data in memory 26 relating to the detected abnormality.

At the end of the monitoring period, the AMU 10 is "docked" in the Procedure Control Unit 30 at 24 referred to as PCU and a visual and/or printed report is generated.

The patient may alert the MPU of the occurrence of an unusual event by depressing the "Patient Symptom" button 32 on the unit.

The AMU 10 requires two 9 volt "transistor" alkaline batteries 12 for a full 24 hour procedure.  Average battery life is expected to be 36 hours or more.

After the 24 hour proceedure is completed the battery is still able to maintain the data in the memory for 5 to 7 days.

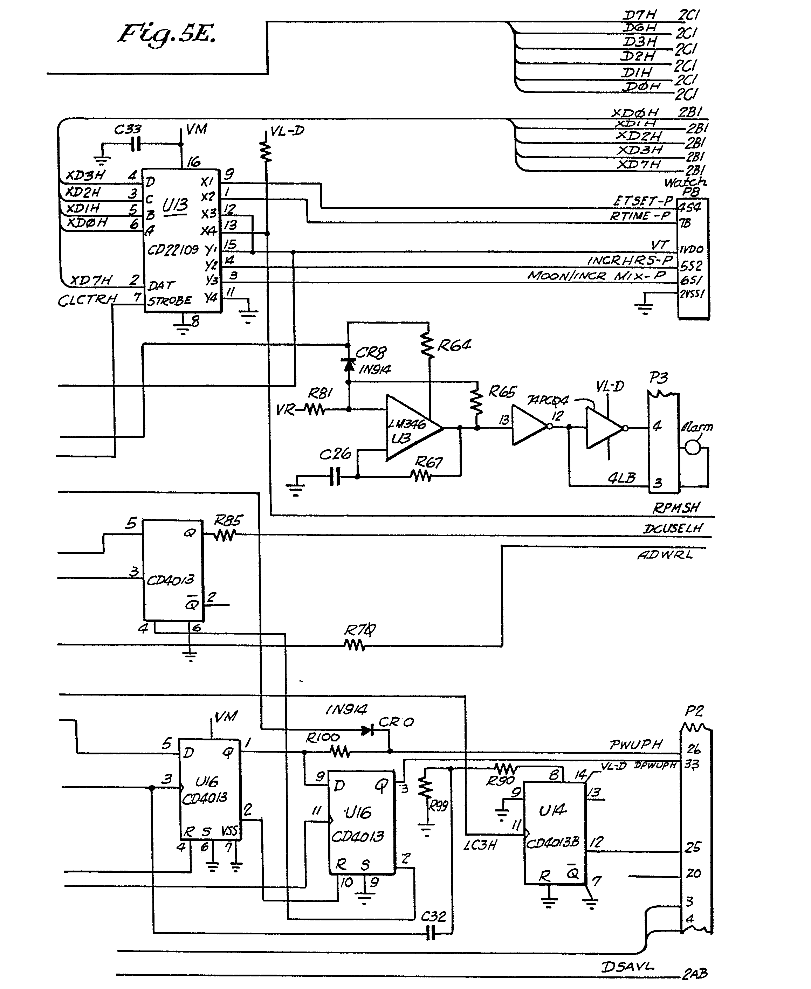

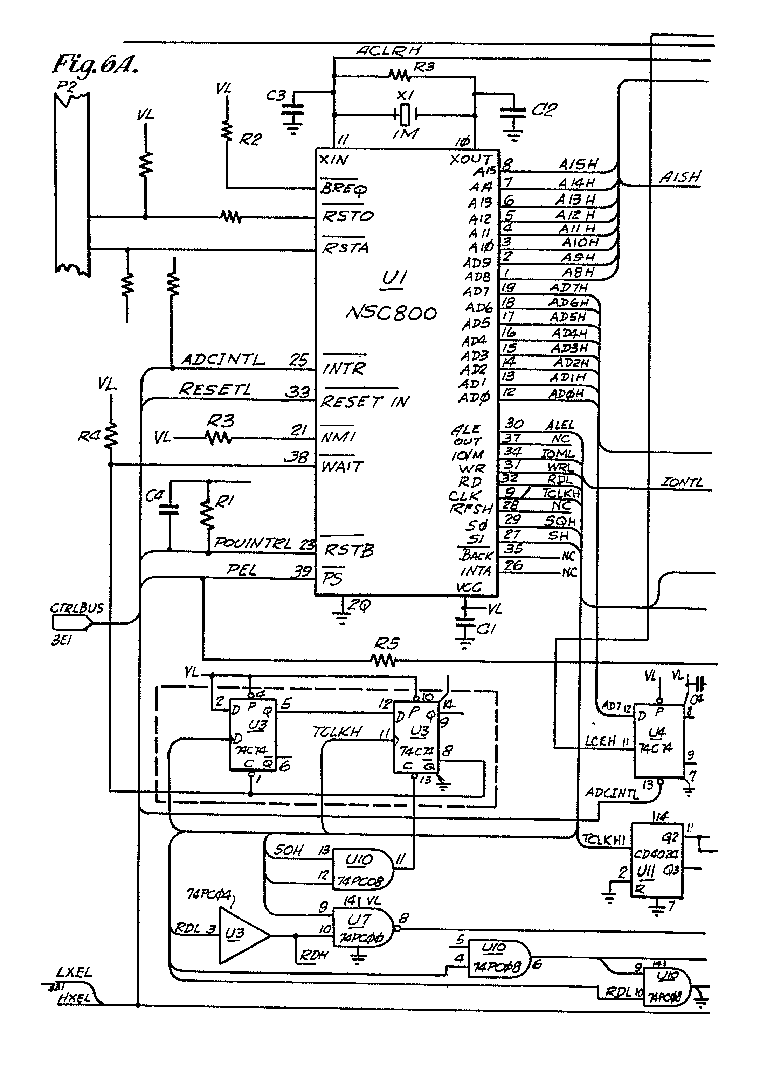

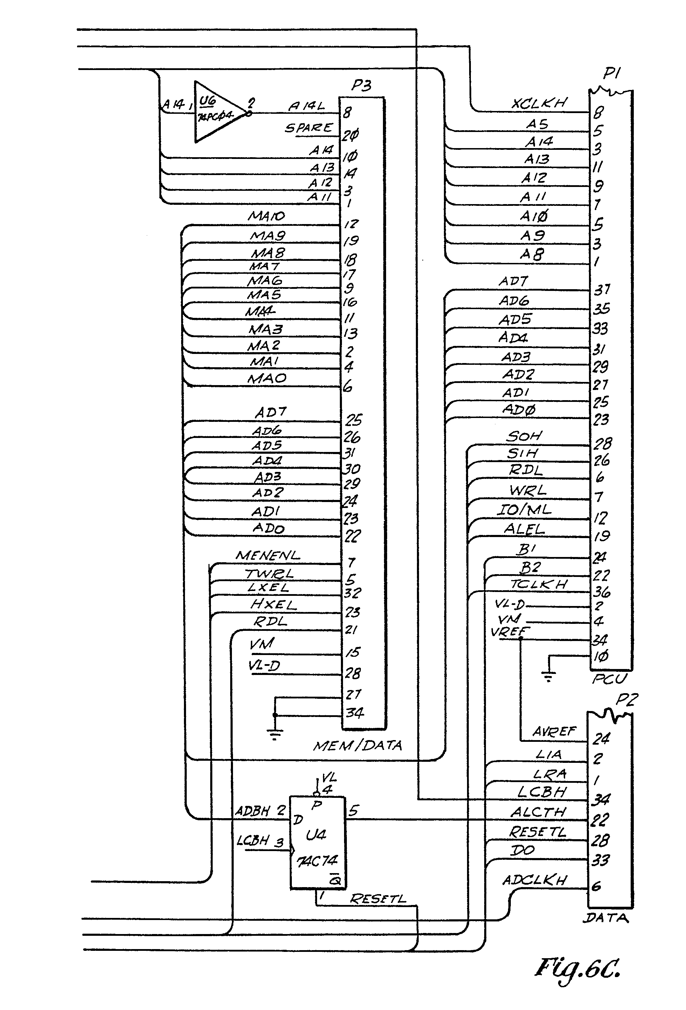

The controlling component is the NSC 800 MPU 24 shown in Figure 1 and 6A which directly accesses all of Memory 26 and PROM 50. The PROM 50 is accessible only when docked and powered by the PCU 30 shown in Figures 3 and 4. Prom, a personality Prom, for the AMU describes the model No. , Ser. No. and the revision level for the unit.   The MPU 24 communicates with the PCU via programmed I/O using the Data and Address busses and the MPU control lines. The Control Logic block generates the indicated control signals on command from the MPU. It also communicates device status (such as the "Patient Symptom" button) via the Data/Address bus to the MPU. The MPU includes as set forth in more detail herein a Memory, Multiply/Divide Unit, ECG Signal Processing means, Pacemaker Detector, Patient Lead Imbalance Test Logic, Patient Interface, and Power means.

The AMU 10 communicates with the PCU 30 (Procedure Control Unit) via a pin connector. The AMU docking and undocking procedures are controlled to provide a well controlled system.

The AMU 10 electronic package may consists of two 32K x 8 memory boards or other comparable memory board, an MPU board and a data board interconnected by a common 8 bit bidirectional address/ data bus, an address bus and several control signals. Figure 1 is the overall block diagram of the AMU and Figures 2, 3 and 4 are the block diagrams for the MPU, Memory and Data boards respectively.

The front panel Patient Connector of the PCU is cabled to this board which contains the optically coupled amplifiers to isolate and buffer the patient leads. These amplifiers connect to an analog mux which allows test signals (from the DAC on the PCU I/O Board) to be substituted for the live signal either as Lead 1, Lead 2, or both (common mode rejection ratio test). Provision is made to support the "Four Lead" patient connection with a second isolation amplifier.

The system includes means for detecting and controlling heart pacer spikes for use of the AMU on patients having implanted heart pacers.

This invention provides means for QRS classification by utilizing the following QRS classification criteria. The QRS classification criteria used by the AMU ECG analysis programs to provide QRS classifications as set forth hereinbelow.

DEFINITION OF TERMS

RRI            :  Time interval from one point of a QRS complex to the one point of the immediately preceding QRS complex

$RRI_c$        :  RRI for the QRS complex being classified

$RRI_p$        :  RRI for the previous complex

$RRI_n$        :  RRI for the next complex

$\overline{RRI}$        :  Average of eight successive RRI

$\overline{RRI}_c$        :  $\overline{RRI}$ which includes $RRI_c$ and the seven RRI preceding it

$\overline{RRI}_p$        :  $\overline{RRI}$ which includes $RRI_p$ and the seven RRI preceding it

$\overline{RRI}_n$        :  $\overline{RRI}$ which includes $RRI_n$ and the seven RRI which follow it

HR                          : Average QRS rate in beats/minute

$RRI_c$ is compensatory     : Preceding QRS complex is premature
                              and typical and $.91 < \dfrac{RRI_c + RRI_p}{2 \times \overline{RRI}p} < 1.09$

Initial, Typical QRS        : QRS identified during start up as
                              representing the patient's typical
                              QRS complex

$QRS_1$ is similar to $QRS_2$ :    $QRS_1$ has the same number and orientation of major legs as $QRS_2$ and the height of the major legs of $QRS_1$ are within 50% of the height of the corresponding waves of $QRS_2$

Typical descendant:    : A QRS similar to the initial typical QRS or similar to an average of the preceding eight (8) typical descendants

Typical QRS    : Typical descendant

Atypical QRS    : A QRS complex which is not similar to a Typical QRS

Baseline value    : The average of the four ECG values which immediately precede the I point of a typical QRS complex

Baseline Stability Criteria (BSC):

1. Baseline values for adjacent typical QRS complexes differ by less than 10% of the grid height.

2. The maximum and minimum baseline values differ by less than 15% of the grid height.

3. Baseline values differ from mid-grid by no more than 12% of the grid height.

Stable baseline    : Classification of an atypical QRS and all of the BSC are satisfied for 8 beats preceding and following it

Wandering baseline    : ONSET occurs when an atypical QRS is classified and one of the BSC is not satisfied and stable baseline existed for the previous atypical QRS.

OFFSET occurs 8 beats after all BSC are satisfied following ONSET.

No classifications are made from the ONSET to the OFFSET of wandering baseline.

**0080821**

A.  Asystole

a)  Time since previous QRS exceeds 2.5 seconds and either:

b)  next three QRS complexes are typical and the baseline is stable or

c)  7 more intervals of 2.5 seconds pass without a QRS being detected

B.  Rates

a)  For          complexes, hr $\leftarrow 60/\overline{RRI}_c$

C.  Single Beat Classifications

1.  Premature Typical $\geq 6$ per minute

The basic notion here is that approximately 15% change in RRI is
required in order for the change to be visually obvious.

The criteria for this classification are that either the RRI preceding
the candidate QRS is obviously premature (more than 15%) and there is
no rate increase present or that there is at least a 15% difference in
the length of the premature RRI and the succeeding RRI.

a)  the two preceding QRS are typical QRS and

b)  the preceding QRS is not Premature Typical and

c)  the reference of comparison for prematurity is $RRI_p$ unless it is
compensatory, in which case the reference is the average of the
two preceding RRI

and either

d) $RRI_c \leq .84 \times RRI_p$         (premature)

and

$RRI_n \geq .94 \times RRI_p$         (no rate increase)

or

e) $RRI_c \leq .84 \times RRI_n$         (visually obvious difference)

and

$RRI_c \leq .91 \times RRI_p$         (at least 9% premature)

and

$RRI_c + RRI_n \leq 2.09 \times RRI_p$     (compensatory)

and

f) 6 or more such classifications observed in the preceding 60 seconds.

2. Premature Typical

Same as 1 a), b), c), d), e) with

f) fewer than 6 such classifications observed in preceding 60 seconds.

3. Premature Atypical $\geq$ 6 per minute

a) Atypical QRS

and

b) $RRI_c < .875 \; \overline{RRI}_p$

and

c) 6 or more such classifications observed in preceding 60 seconds.

4. Premature Atypical

Same as 3 a), b) with c) fewer than 6 such classifications observed in the preceding 60 seconds.

5. Atypical $\geq$ 6 per minute

   a) Atypical QRS

   and

   b) $.875 < (RRI_c / \overline{RRI}_p) < 1.5$

   and

   c) preceded and followed by typical QRS or member of a sequence of not more than three atypical QRS complexes
      none of which are premature (i.e., all of which satisfy $RRI_c > .875\ \overline{RRI}_p$

   and

   d) more than six such classifications observed in preceding 60 seconds.

6. Atypical

   Same as 5 a), b), c) with d) fewer than six such classifications observed in the preceding 60
      seconds.

D. Prolonged RRI

   1. Prolonged RRI 50% to 87%

     a) $1.5 < (RRI_c / \overline{RRI}_p) \leq 1.875$       (one missing complex)

     and

     b) preceding QRS not premature typical or premature atypical QRS
     and

     c) $RRI_c < 2.5$ seconds       (not asystole)

     and

     d) not within 4 QRS complexes following a rate decrease pattern change
     and

     e) $1.125\ \overline{RRI}_n > \overline{RRI}_p$       (not preceding a rate decreased)

- 15 -

2. Prolonged RRI > 87%

  0080821

   a)  $RRI_c$ is not compensatory

   and

   b)  $RRI_c$ < 2.5 seconds                    (not asystole)

   and either

   c)  $1.875 < (RRI_c/\overline{RRI}_p) \leq 2.875$        (2 missing complexes)

      and

      d) and e) above

   or

   d)  $2.875 < (RRI_c/\overline{RRI}_p)$            (3 missing complexes)


E.   <u>Bigeminy (Onset)</u>

   1.  Typical

      Four typical QRS complexes in sequence with the first and third being
      premature typicals which satisfy:

      $$.91 < ((RRI_c + RRI_n)/2 \times \overline{RRI}_p) < 1.09$$

   2.  Atypical

      Four QRS's in sequence with the second and fourth being typical and
      the first and third being either premature atypical or premature
      atypical $\geq$ 6/minute.


F.   <u>Atypical Sequences</u>

   Model:  a.  X atypical QRS complexes in sequence preceded and followed by
              a typical QRS complex and b.  at least one of the X atypical complexes satisfies:

              $$RRI_c < .875 \times \overline{RRI}_p$$

   1.  Couplets:          X = 2

   2.  Triplets:          X = 3

6. <u>Pattern Change</u>

| | | | |
|---|---|---|---|
| 1. | Rate Increase | : | $.67 \times \overline{RRI}_c > \overline{RRI}_n$ |
| 2. | Rate Decrease | : | $1.5 \times \overline{RRI}_c < \overline{RRI}_n$ |
| 3. | Shape Increase | : | The first atypical QRS in a sequence of 4 or more atypical QRS which is preceded by a typical QRS |
| 4. | Shape Decrease | :· | A typical QRS preceded by 4 or more atypical QRS |
| 5. | Regularity Increase | : | Not operational |
| 6. | Regularity Decrease | : | Not operational |

The AMU measures the ST level of selected QRS complexes, it uses these measurements to determine a 16 B or beat. average for the ST level. The maximum & minimum average value summary are preserved for the physicians' report and . in addition the AMU will preserve rhythm strip s during the ambulatory monitoring procedure which show QRS complexes having the maximum and minimum average ST level which were observed during the procedure.

The ST level is computed only for typical QRS complexes and represents the difference between the average ECG voltage measured:

a) just prior to the onset of the typical QRS complex and;

(b) 80 ms after the offset of the typical QRS complex.

If the ST level for a given typical QRS complex differs significantly from the 16 B average than that QRS complex is not included in the 16 B average. The 16 QRS complexes use in the average must occur in a 36 B or beat sequence.

The ST level is positive if the ECG voltage 80 ms after the offset of the QRS complex is greater than the ECG voltage prior to the onset of the QRS complex.

This invention also provides means for ST level analysis by utilizing the following ST level analysis. The ST level analysis is used by the AMU ECG analysis programs disclosed in Appendix II to provide ST level as set forth hereinbelow.

The external functions, interfaces and performance of the Two Channel Ambulatory Monitoring System with pacemaker sensing is disclosed herein. This is an improvement to the Medical Monitor set forth in U.S. patent application Serial No. 208,051 filed January 15, 1981 which is incorporated herein by reference to make it a part of this specification.

The Two Channel Ambulatory Monitoring system monitors, analyzes and produces reports on patient ECG signals taken from a two lead electrode configuration. The monitoring and analysis is performed by a small, lightweight ambulatory computer system designated as the Ambulatory Monitoring Unit or AMU. The operation of the AMU is initiated by the Procedure Control Unit or PCU. The report from the Ambulatory Monitoring Procedure is formatted by the PCU and generated into a report using the Graphic Report Printer or GRP.

Thus, the Two Channel Ambulatory Monitoring system consists of three basic components, the AMU, the PCU and the GRP. The ECG analysis performed by the AMU is designed to detect changes in rate, irregularity and shape of QRS complexes. Such changes are categorized into a number of QRS activity classifications: Rate ranges, single QRS and RR interval classifications, QRS sequence classifications, and major pattern changes.

The Two Channel system is sensitive (under program control) to pacemaker stimulation pulses.

The Ambulatory Monitoring Unit provides for pacemaker and pacemaker/cardiac activity classifications.

The user interaction with the system is accomplished through the Procedure Control Unit using a series of video screens. These screens contain prompting information and provide for user selection of system services. The user may define customized procedures for use with the Ambulatory Monitoring Unit. These customized procedures can subsequently be invoked when the Ambulatory Monitoring Unit is initialized. The user may specify the content of reports which are generated and may also review and edit report data on the video screen before or subsequent to printed report generation.

The Ambulatory Monitoring Unit makes use of two independent channels of ECG data. Through the use of impedance measuring equipment in the AMU, it is possible for the unit to determine that the patient leads are properly connected. In the event of poor connections or loose leads, the AMU can suspend analysis of ECG signals from a given channel. By verifying ECG activity in both channels the Ambulatory Monitoring Unit can achieve higher reliability of its QRS classifications than is possible through the use of a single channel.

The Ambulatory Monitoring Unit contains a liquid crystal time display for patient convenience in maintaining an activity diary. The Ambulatory Monitoring Unit also contains a Patient Marker Button for indications of patient symptoms. The Ambulatory Monitoring Unit logs depressions of the Patient Marker Button for patients in the physician's report. Additionally, the Ambulatory Monitoring Unit contains audio tone generator which is used for indicating to the patient that the AMU has terminated the monitoring procedure or that it is having difficulty in analyzing the signals received from the patient.

The Graphic Report Printer is contained in an enclosure separate from the Procedure Control Unit. It produces printed pages which are cut to 8-1/2 x 11". The printing equality is such that it can accurately portray ECG signals.

The Procedure Control Unit provides a video screen for prompting and data presentation to the user. It provides a keyboard for data entry and user control and response. It also contains a docking port for the Ambulatory Monitoring Unit as well as a receptacle for the ECG cable connector.

The Two Channel Ambulatory Monitoring Unit will be capable of sustaining ambulatory monitoring and analysis for at least 24 hours. Following the ambulatory monitoring period of 24 hours, the Ambulatory Monitoring Unit will be capable of preserving ECG analysis data for a period of 72 hours. The Ambulatory Monitoring Unit receives ECG data from two independent ECG channels. These channels have independent program controlled sensitivity settings which provide three full scale voltage ranges as follows: $\pm$1.25 m, $\pm$2.5 m, $\pm$5 m. The Ambulatory Monitoring Unit samples the ECG signals from each channel at a rate of 250 samples per second. The sampling of these two channels is accomplished in an interlaced fashion. The Ambulatory Monitoring Unit detects QRS complexes at rates of up to 300 beats per minute. The Ambulatory Monitoring Unit classifies the detected QRS complexes which occur in the range of 0 to 190 beats per minute. The Ambulatory Monitoring Unit is powered by a self-contained power source consisting of two 9 volt batteries. On a program control basis the Ambulatory Monitoring Unit is capable of determining the integrity of the ECG signal circuit consisting of the ECG cable, the lead wires, the electrodes and the patient's physiology. The Ambulatory Monitoring Unit stores data which is sufficient to permit the reconstruction of ECG rhythm strips. The AMU can store at least the equivalent of twenty-four single channel rhythm strips each containing eight (8) seconds worth of ECG data. The Ambulatory Monitoring Unit stores data which is sufficient to permit the reconstruction of QRS type rhythm strips. The AMU contains sufficient storage to reconstruct 16 two second QRS type rhythm strips.

The Ambulatory Monitoring Unit detects the presence of stimulating pulses generated by electronic pacemakers. These stimulating pulses are characterized by pulse durations and amplitudes in the following ranges:

Amplitude:      1-500 millivolts

Duration:      1-2 milliseconds

These stimulation pulses are generally followed by recharge pulses of opposite polarity characterized by:

Amplitude:      5-250 millivolts

Duration:      20-100 milliseconds

The presence of the recharge pulses is not of interest to the ambulatory monitoring procedure. The actual shape and width of the stimulating pulses are not of interest to the ambulatory monitoring procedure. The existence and time of occurrence is of significance.

Through QRS detection and analysis logic the Ambulatory Monitoring Unit classifies ECG activity into the following activity types:

The QRS Average Rates may be as follows:

The following rate ranges are recognized by the AMU:

1. Asytole 0 - 25 BPM
2. Bradycardia 3 25 - 40 BPM
3. Bradycardia 2 40 - 50 BPM
4. Bradycardia 1 50 - 60 BPM
5. Normal 60-100 BPM
6. Tachycardia 1 100 - 150 BPM
7. Tachycardia 2 150 - 190 BPM
8. Tachycardia 3 190 BPM's and greater.

The Single Beat Classification may be as follows:

During procedure initiation, the patient's typical QRS complex is identified. During the ambulatory procedure, each detected QRS complex will be recognized as similar in shape to the patient's typical complex or dissimilar in shape to the patient's typical complex.

If the shape of an individual complex is dissimilar to the patient's typical complex, it will be recognized as an "Atypical" complex. If the complex is similar in shape to the patient's typical complex, the complex is recognized as "typical". If the R-R interval for a given complex is sufficiently less than the average R-R interval for the preceeding complexes, the complex is classified as premature. It is classified as typical, premature if the shape is typical and the complex is premature. It is classified as atypical, premature if the complex is classified as atypical and it is premature. If these premature complexes have been observed as frequently as 6/minute, then they are classified as typical premature or atypical premature occurring at a rate greater than or equal to 6/minute.

Consequently, the single beat classifications
for premature complexes are:

1.    Premature, typical, (less than 6/minute).

2.    Premature, typical, greater than or equal
to (GE) 6/minute.

3.    Premature, atypical, (less than 6/minute)

4.    Premature, atypical, GE 6/minute.

The QRS Sequence Classification may be as follows:

The ambulatory monitoring procedure will classify
multibeat sequences according to the following categories:

1.    Atypical bigeminal pattern (i.e., typical
QRS complexes alternating with atypical premature QRS
complexes).

2.    Typical bigeminal pattern (i.e., typical QRS
complexes alternating with premature typical QRS complexes).

3.    Atypical couplets (i.e., two atypical QRS
complexes in sequence preceeded and followed by typical
QRS complexes).

4.    Atypical triplets (i.e., three atypical QRS
complexes in sequence preceeded and followed by typical
QRS complexes).

The R-R interval for a given QRS complex may be
significantly longer than the average R-R interval for
the preceeding eight (8) complexes.  In this case the
ambulatory monitoring procedure will classify the complex
into one of the following categories:

1.    Pause (the measured R-R interval is less than
twice the average R-R interval).

2.    Missed beat(s) (the R-R interval is greater
than twice the average R-R interval but less than the
interval required for Asystole).

3.    Asystole (R-R interval greater than 2.5 seconds).

- 24 -                    0080821

The Major QRS Pattern Change Classifications may
be as follows:

The ambulatory monitoring procedure analyzes
adjacent, eight beat sequences of QRS complexes for
significant changes in rate, regularity and shape.
For each of rate, regularity and shape, the procedure
determines whether there was:

1.    No change.

2.    Increase.

3.    Decrease.

An increase (decrease) in regularity means that
the R-R intervals were more (less) uniform.  An increase
(decrease) in shape means the average width or area
of the QRS complex became greater (less).  An increase
(decrease) in rate means that the average R-R interval
became significantly shorter (longer).

By analyzing each of the three conditions (rate,
regularity and shape) into three possible categories
(no change, increase, decrease) the ambulatory .
monitoring procedure can recognize 27 types of possible
change patterns.  One of those patterns corresponds
to a no change condition for all three arrhythmia
patterns.  The remaining 26 change patterns of QRS ·
activity are considered to be significant by the
ambulatory monitoring procedure.  The occurrence of .
any one of these 26 patterns is classified as a Major
QRS Pattern Change.

The Pacemaker Related Classification may be as
follows:

For monitoring procedures which are sensitive
to the presence of pacemaker stimulating pulses, the
following activities are recognized:

- 25 -

1.  Pacemaker Activity Classifications

    A.   Short Escape Interval:
         Stimulation pulse occurs prematurely
         relative to preceeding QRS complex.
         This prematurity is measured with respect
         to the demand rate of the pacemaker.
         Such prematurity is consistent with:

         i.)   a premature QRS complex which occurs
               during the pacemaker's refractory
               period.
    or   ii.)  pacemaker sensing of "interference"
               signals and reversion to fixed rate
               pacing.
    or   iii.) sensing failure of a demand pacemaker.

    B.   Long Escape Interval:
         Stimulation pulse occurs late relative
         to the pacemaker demand rate and the
         previous QRS complex.  This is consistent
         with over sensitive demand pacemaker
         becoming inhibited by presence of noise
         or other extraneous or induced signals.

    C.   Failure to generate stimulation pulses.

2.  Pacemaker/Cardiac Activity Classifications

    A.   Cardiac Capture:
         Stimulation pulse followed by correctly
         timed and shaped QRS complex.

B.    Cardiac Non Capture:
No QRS complex following stimulation
pulse at proper interval.

C.    Pacer/QRS Fusion:
Stimulation pulse occurs during QRS complex.

During the ambulatory monitoring period, the monitoring procedure will collect the following summary data.

1.    Phenomena Summary.

2.    Major QRS Pattern Log.

3.    Hourly Summaries.

4.    Detailed Summaries.

5.    Patient Marker Log.

6.    R-R Interval Distributions.

The phenomena summary contains a summary of each of the QRS classifications which were observed during the ambulatory monitoring procedure.  This summary lists the name of the QRS classification and shows the number of such patterns or the number of episodes of such patterns.

The Major QRS pattern log identifies each AMU observation of a significant change in rate, rhythm or shape.  This log (for each observed major QRS change) contains:

1.    The time of day of the change in the ECG pattern.

2.    The duration of the atypical pattern, if it could be clearly determined by the AMU.

3.    The QRS rate in beats/minutes before and after the change.

4.    The QRS rhythm before and after the change.

5.    The average QRS width before and after the change.

6.    The identification of the ECG rhythm strip which displays the major atypical QRS pattern.

The AMU may not be able to retain the ECG rhythm strip for a particular atypical QRS pattern because of the occurrence of a higher priority pattern.

7.    An indication of which of rate, rhythm or width change was considered significant by the AMU.

The hourly summary data contains the time distribution of occurrence for each of a number of QRS classifications together with a partial ECG rhythm strip of the QRS activity.   The hourly summary data is sufficient to identify or create:

1.    The name of the QRS classification.

2.    The total number of observations for this classification during the ambulatory monitoring procedure.

3.    A histogram which shows the number of occurrences per hour of this QRS classification during the ambulatory monitoring procedure.

4.    A partial ECG rhythm strip which contains the atypical QRS pattern.

The AMU may not be able to preserve an ECG rhythm strip for each type of QRS activity which occurred during the ambulatory monitoring procedure.   In this situation the hourly summary data will contain all the previously mentioned information except for the partial ECG rhythm strip. ·

The classifications presented in hourly summaries are all classifications except:

1.    Rate classifications

2.    Major pattern change classifications

3.    Premature typical and atypical classification

· At the conclusion of each summary period the AMU calculates and records the following detailed summary data items:

1. For QRS rate, the maximum, average and minimum observed values for 8 beat sequences.

2. For atypical, premature complexes; the number of occurrences and the number of such complexes which occurred at a rate greater than or equal to 6/minute during that summary period.

3. For typical, premature complexes, the number of occurrences and the number of such complexes which occurred at a rate greater than or equal to 6/minute during that summary period.

4. For S-T level, the maximum average and minimum deflection from the estimated base line in millivolts.

5. For the time of analysis, the percentage of time lost due to the presence of noise and/or wandering base line in the patient's ECG signal.

6. For the time of analysis, the percentage of time lost due to the ECG cable being disconnected from the AMU.

The AMU maintains R-R interval distribution data for all complexes observed and for atypical premature complexes. It tallies the number of QRS complexes observed into one of a number of R-R interval durations. Each is approximately 25 milliseconds in duration (e.g., 200-224, 225-249, 250-274, etc.).

If the patient marker button was activated for the ambulatory procedure and if the patient depressed the patient marker button during the procedure, the AMU will record certain information.

The data consists of:

1. Time of day at which the button was depressed.

2. The identification of the highest priority QRS classification which was recognized in close time proximity to the depression of the patient marker button.

The user may specify how the AMU is to handle depression of the patient marker button by the setting of one of the procedure global parameters.

If the user specifies (via global parameters) that the patient marker button is non-operational, then no patient marker log data is kept. If the user specifies that the patient marker button is operational, then the AMU will record the time of each depression on the button. If the user indicates that more than zero (0) patient marker rhythm strips are to be preserved, then the AMU will also indicate the name of the observed QRS classifications in the patient marker log.

The AMU maintains the procedure definition parameters during the procedure period.

The AMU maintains the patient identification data during the procedure period.

The AMU maintains the physicians pre-procedure comments during the procedure period.

The AMU identifies QRS Activity according to the classifications of section 3.1.12. The occurrence of any classification except Rate 60 - 100 BPM or Cardiac Capture causes the AMU to consider preserving a rhythm strip data of the QRS complexes which caused the classification.

A rhythm strip data consists of:

1. The time of occurrence.

2. Average QRS rate.

3. The identification of the classification.

4. An indication of relationship to depression of the Patient Marker Button Depression.

5. Data to reconstruct the ECG representation.

6.    Data to identify the channel source of the
ECG representation.

7.    A measure of the "quality" of the ECG data.

The amount of ECG representation data kept is a
function of the classification of the QRS Activity:

1.    At least 4 QRS complexes for rate
classifications.

2.    At least 3 complexes before and after premature typicals and atypicals, couplets, triplets,
pauses, and missed beats.

3.    At least 2 QRS complexes before the onset
of bigeminal sequences.

4.    8 seconds for all other classifications.

In addition, the rhythm strip duration is limited
to one of the following values:

·4 seconds

5 seconds

6 seconds

7 seconds

8 seconds

The choice is the smallest duration required to
satisfy the 4 conditions specified above.

The procedure definition data may specify, for
any classification, the maximum number of rhythm strips
to be preserved.  In this case, the AMU preserves the
specified number of rhythm strips (assuming more than
this maximum number are observed) for each classification,
choosing the highest quality rhythm strips for preservation.

AMU stores approximately 200 seconds of ECG data.

A quality measure is assigned to each rhythm strip. Elements of quality are:

1. Noise
2. Rate
3. Prematurity
4. Extended RRI

A. The noise quality measurement will reflect the amount of noise and artifact in the rhythm strip.

B. The average QRS rate for the 16 complexes of the rhythm strip will be the rate quality measure.

C. For premature classifications, the ratio

$$R_P = \frac{PRRI}{ARRI}$$

is the prematurity quality measure where ARRI is the average RRI for the preceding 8 complexes and PRRI is the RRI for the premature complex.

D. Pause and Missed Beat Classifications:

The approximate ratio:

$$R_L = \frac{RRI}{ARRI}$$

Where: RRI is the measured R-R interval for the missed beat or pause.

ARRI is the average RRI for the proceeding 8 QRS complexes.

For QRS classifications other than Rate, Premature, Pause, Missed Beat, the AMU preserves the rhythm strips with the lower noise measurement.

For Rate classifications, the AMU gives preference to the rhythm strips with higher rates and/or lower noise measures.

For Premature classifications, the AMU gives preference to rhythm strips with smaller prematurity ratios $R_P$ and lower noise measures.

For Pause and Missed beat classifications, the AMU gives preference to larger rations of $R_L$ and smaller noise measures.

The AMU receives during procedure initiation a list of priorities of QRS classifications. The AMU uses this list to control the preservation of rhythm strips. Priorities of ECG classifications take precedence over ECG quantity specifications. Hence a rhythm strip may be displaced by one of higher priority classification if the maximum quantity of the higher priority classification has not been reached.

The priority scheme (subject to quantity and quality):

1. Preserves ECG rhythm strips with the QRS classifications of highest priority which were observed.

2. Prevents multiple occurrences of a given QRS classification from dominating the physician's report when QRS classifications have been observed.

3. Prevents multiple atypical QRS classifications which occur in any one hour time span from dominating the physician's report at the expense of lower priority QRS classifications which occur in other one hour time spans.

The AMU will maintain rhythm strips of only one channel of ECG data unless:

1. The user specified that both channels were to be preserved.

or

2. The analysis was not confirmed in both channels.

The AMU selects the channel to be presented if only one channel of data is displayed.

The Ambulatory Monitoring Unit referred to hereafter as the AMU is a battery operated, 24 hour ambulatory heart monitor. It monitors the patients ECG activity via a dual channel (Lead V5 and modified Lead 2) connection employing five patient leads.

The AMU has a digital microprocessor and a 64K byte random access memory incorporated. The microprocessing unit referred to as MPU continuously analyzes the patients heart activity during the monitoring period. When abnormal activity is detected, the MPU stores data in memory relating to the detected abnormality.

At the end of the monitoring period, the AMU is "docked" in the Procedure Control Unit referred to as PCU and a visual and/or printed report is generated.

.The patient may alert the MPU of the occurrence of an unusual event by depressing the "Patient Symptom" button on the unit.

The AMU requires two 9 volt "transistor" alkaline batteries for a full 24 hour procedure. Average battery life is expected to be 36 hours or more.

The AMU is 6.72 x 4.18 x 1.28 inches overall and weights 26 ounces. All electronics and power supply are contained in this envelope.

The case is a two piece injection molded permanently sealed unit. The case material is Noryl. The front panel (1.28 x 4.18 inches) contains the "Patient Symptom" button, the clock display and the patient connector. The rear panel houses the PCU interface connector which mates with the PCU docking station. A slide/snap door on one side of the AMU provides access to the two 9 volt batteries.

Figure 1 is a block diagram of the S-120B AMU. The controlling component is the NSC 800 MPU which directly accesses all of Memory and PROM. The PROM is accessible only when docked and powered by the PCU. The MPU communicates with the PCU via programmed I/O using the Data and Address busses and the MPU control lines. The Control Logic block generates the indicated control signals on command from the MPU. It also communicates device status (such as the "Patient Symptom" button) via the Data/Address bus to the MPU. The MPU operates on a 4 to 8 MHz clock which results in a memory cycle time of 2.0 to 1.0 microseconds.

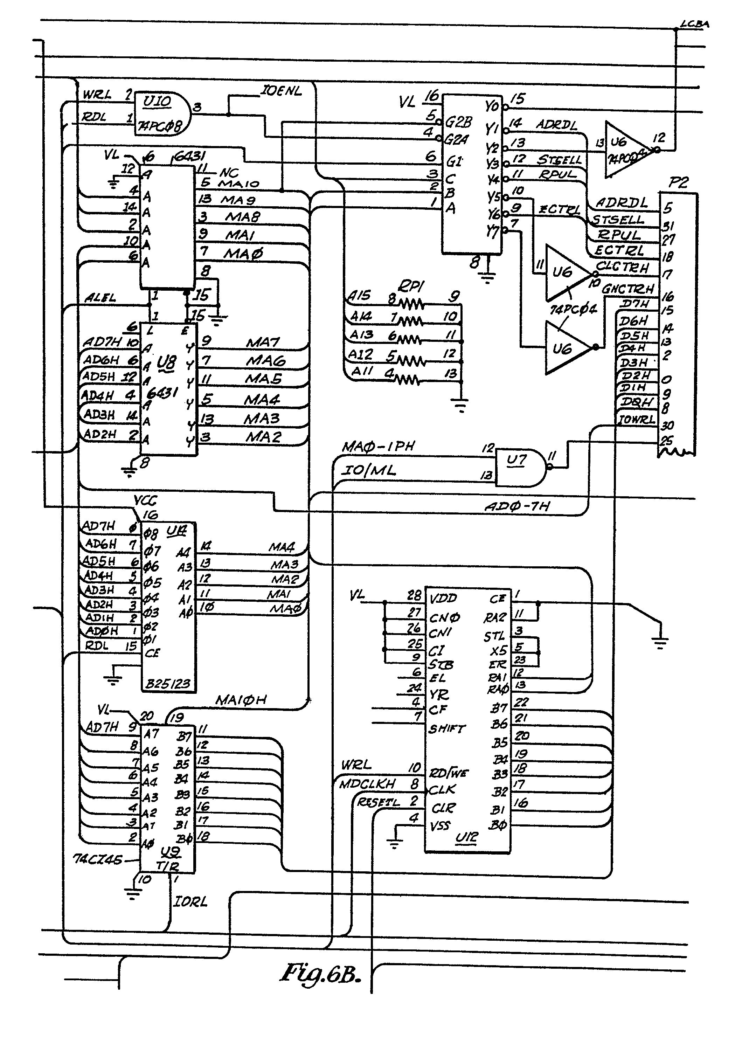

The MPU 24 is shown in detail in Figures 6A, 6B, 6C, 6D and 6E, it reads the 12 byte personality section c the PROM (only when the AMU is docked) by issuing a series cf memory read and move instructions beginning at memory address $0015 and ending at $001F. The least significant five bits of the address form the PROM address for this instruction. The PROM contains one HEX address 00-13 for Bootstrap loader data, see initial program load, and another HEX address 14-1F for Personality data. Addresses $00-$1F are also RAM addresses which can be written to by the MPU at any time. They can always be read by the MPU when the AMU is ambulatory. When docked, the RAM addresses can only be read by the CPU when the PCU controlled PROM enable bit is off.

The MPU I/O Functions are:

I/O PORT

| HEX ADDRESS | OUTPUT | INPUT |
|---|---|---|
| 00 | Write to MDU X Register | Read MDU X Register |
| 01 | Invalid | Read A/D |
| 02 | Load Control Byte | Invalid |
| 03 | Invalid | Read AMU Status |
| 04 | Invalid | Read PCU Status |
| 05 | Spare | Spare |
| 06 | Reset Output Ready | Spare |
| 07 | Write Data to PCU | Read PCU Data |
| 08 | Write to MDU Z Register | Read MDU Z Register |

```
I/O PORT
HEX ADDRESS      OUTPUT                        INPUT

     10        Write to MDU Y Register      Read MDU Y Register
     18        Write to MDU Control Reg.    Read MDU Status
     20        Reset Power Up *             Invalid
     21        Clock Control                Invalid
     22        Lead Sense Control           Invalid
     23        Gain Control                 Invalid
     25        ADC Ch A Data                Invalid
     2D        ADC Ch B Data                Invalid
```

at 20 the Data bit 7 is transferred to Data Save.   If data bit 7 is set high when port $20 is selected, the Data Save mode is entered.

Control Byte Bit Assignments are:

| BIT | ACTIVE STATE | FUNCTION |
| --- | --- | --- |
| 0 | — | Spare |
| 1 | — | Spare |
| 2 | — | Spare |
| 3 | 1 | Alarm Enable |
| 4 | — | Spare |
| 5 | — | Spare |
| 6 | 1 | Read PCU Analog Channels |
| 7 | 1 | Power Save |

MPU Status is as follows:

| | AMU STATUS | | PCU STATUS | |
| --- | --- | --- | --- | --- |
| Bit | (Input Port 03) | Active State | (Input Port 04) | Active State |
| 0 | Patient Connected | 0 | Output Buffer Empty | 1 |
| 1 | Symptom Button | 0 | AMU Output Ready | 1 |
| 2 | Low Battery | 0 | Spare | — |
| 3 | Docked | 0 | Spare | — |
| 4 | Data Save Status | 0 | Spare | — |
| 5 | Spare | — | Spare | — |
| 6 | Pacemaker + | 1 | Input Message Pending | 1 |
| 7 | Pacemaker - | 1 | Input Buffer Full | 1 |

The Clock Control POrt ($21) Data/Function Assignments are:

| Function | Xpoint(s) | Enable Byte Val. | Disable Byte Val. |
|---|---|---|---|
| Enable Time Set | X1Y1 | 80 | 00 |
| Reset Time (Note 3) | X2Y1 | 81 | 01 |
| Increment Hours (Note 2) | X1Y2 | 84 | 04 |
| Increment Minutes (Note 2) | X1Y3 | 88 | 08 |
| Crescent Symbol (note 1,2) | X3Y3 | 8A | 0A |
| Reset Pacemaker Data | X4Y4 | 8F | 0F |

Note that 1 requires the following alternating sequence to turn the crescent symbol on and off: 8F-0F-8F-0F, and 2: the increment minutes, increment hours, and crescent symbol pulses should have the following characteristics:

Pulse High Time: 250 Milliseconds minimum Pulse Low Time: 250 Milliseconds minimum Period: 500 Milliseconds minimum,

and 3: the reset pulse should have a minimum pulse high time of 10 ms.

Lead Sense Port ($22) Data/Function Assignments are:

| Function | Xpoint(s) | Enable Byte Val. | Disable Byte Val. |
|---|---|---|---|
| Test Lead 1 | X2Y3 | 89 | 09 |
| | X2Y1 | 81 | * |
| Test Lead 2 | X2Y4 | 8D | 0D |
| | X2Y1 | 81 | * |
| Test Lead 3 | X2Y1 | 82 | 02 |
| | X2Y1 | 81 | * |
| Test Lead 4 | X4Y1 | 83 | 03 |
| | X2Y1 | 81 | * |

| Function | Xpoint(s) | Enable Byte Val. | Disable Byte Val. |
|---|---|---|---|
| Select Pecg Ch A | X1Y1 | 00 | 80 |
| Select Pecg Ch B | X1Y1 | 80 | 00 |
| Analog Test L1, L3 | X3Y3 | 8A | 0A |
|  | X3Y2 | 86 | 06 |
| Analog Test L2, L4 | X4Y4 | 8F | 0F |
|  | X4Y2 | 87 | 07 |
| Analog Test L3 Only | X3Y2 | 86 | 06 |
| Analog Test L4 Only | X4Y2 | 87 | 07 |
| Baseline Protect | X2Y1 | 01 | 81 |

It should be noted that the Xpoint must be enabled at all times except when the baseline protect is enabled.

Channel/Gain Select Port ($23) Data Assignments are:

| Function | Xpoint(s) | Enable Byte Val. | Disable Byte Val. |
|---|---|---|---|
| Channel A to ADC Select | X3Y4 | 8E | 0E |
| Channel B to ADC Select | X4Y4 | 8F | 0F |
| Baseline Test | X1Y1 | 80 | 00 |
|  | X1Y4 | 8C | 0C |
| Channel A Gain X1/2 | – | – | – |
| Channel A Gain X1 | X1Y1 | 80 | 00 |
| Channel A Gain X2 | X1Y1 | 80 | 00 |
|  | X1Y2 | 84 | 04 |
| Channel B Gain X1/2 | – | – | – |
| Channel B Gain X1 | X2Y1 | 81 | 01 |
| Channel B Gain X2 | X2Y3 | 89 | 09 |

Initialization of I/O ports which contain the cross point arrays. All cross points within each array should initially be set to the open condition whenever the AMU is powered up.

MPU Interrupts are:

| Priority (1=highest) | Function | Restart Address* | Name | Active State |
|---|---|---|---|---|
| 1 | Spare | X'0066' | NMI | 0 |
| 2 | Pacemaker | X'003C' | RSTA | 0 |
| 3 | PCU Comm. | X'0034' | RSTB | 0 |
| 4 | 1024 Ms. | X'002C' | RSTC | 0 |
| 5 | A/D (2 MS) | X'0038' | INTR | 0 |

The restart address is the address where the first word of the interrupt service routine for the listed interrupt is located.

The maskable interrupt inputs are enabled under program control through the use of the interrupt control register and enable/disable interrupt instruction. The interrupt control register is an on-chip write only output port located at port address X 'BB'. Its contents are:

| BIT | NAME | FUNCTION |
|---|---|---|
| 3 | IEA | Interrupt Enable for RSTA (Pacemaker) |
| 2 | IEB | Interrupt Enable for RSTB (PCU) |
| 1 | IEC | Interrupt Enable for RSTC (1024 Ms.) |
| 0 | IEI | Interrupt Enable for INTR (A/D) |

Note that the NMI is the non-maskable interrupt. It is always active and its execution is independent of the MPU internal interrupt enable flip-flop. (NMI is not used on the Dual Channel AMU).

Each of the four external devices that can generate an interrupt request contain an interrupt request flip-flop that must be cleared once the interrupt is acknowledged by the MPU. The following table lists the action required to clear each of the specified interrupt request flip-flops:

| Interrupt FF | Action to Clear Interrupt Request |
|---|---|
| RSTA (Pacer) | Reset Pacemaker Status Latch |
| RSTB (PCU) | Read PCU·Data Port ($07) |
| RSTC (Timer) | Write to LCB Port ($02) |
| INTRA (A/D) | Read A/D Port ($01) |

The following table lists the number of T or timing states per cycle when the wait state generator is used. The wait state generator is used on MPU boards which have a CPU clock frequency of 6 MHz or higher.

| Cycle Type | Number of T States |
|---|---|
| Op Code Fetch | 5 |
| Memory Read/Write | 3 |
| I/O | 4 |

The following table lists the number of T states per cycle when the wait state generator is not used. The wait state generator is not used on MPU boards which have a CPU clock frequency of less than 6 MHz.

| Cycle Type | Number of T States |
|---|---|
| OP Code Fetch | 4 |
| Memory Read/Write | 3 |
| I/O | 4 |

T State Times Vs. Clock Frequency are:

| CPU Clock Frequency | T State Time |
|---|---|
| 8 MHz | 250 ns |
| 6 MHz | 333 ns |
| 5 MHz | 400 ns |
| 4 MHz | 500 ns |
| 4.096 MHz | 488 ns |

The AMU memory consists of 32 2K X 8 CMOS chips organized in a 64K X 8 array. This configuration minimizes power consumption in that only one chip is active at any one time.

Power consumption is further reduced by lowering the chip power level to +4 volts in the standby mode. (See Section 4.8.2).

The CDP 1855 is a Multiply/Divide Unit (MDU) which can do a 16 bit by 8 bit divide yielding an 8 bit result plus an 8 bit remainder. The multiply is an 8 bit by 8 bit operation with a 16 bit result. Depending on the CPU clock frequency and including I/O access times, 39 to 56 microseconds are required for the divide operation and 32 to 44 microseconds are required for the multiply operation.

The following sections provide a brief summary of MDU operations:

I/O Assignments are:

| I/O ADDRESS | MDU READ FUNCTION | MDU WRITE FUNCTION |
| --- | --- | --- |
| $00 | Read X Register | Write X Register |
| $08 | Read Z Register | Write Z Register |
| $10 | Read Y Register | Write Y Register |
| $18 | Read MDU Status | Write Control Register |

Control Register Bit Assignment Table is as follows:

| Bit 1 | Bit 0 | Function |
| --- | --- | --- |
| 0 | 1 | Multiply |
| 1 | 0 | Divide |
| 0 | 0 | No Op (other than operations specified by bits 2, 3 and 6) |
| 1 | 1 | Illegal |

Bit 2 = 1 = Reset Z register before operation

Bit 3 = 1 = Reset Y register before operation

Bit 4    1    Set number of cascaded MDU's = 0

Bit 5    1    Set number of cascaded MDU's = 0

Bit 6 = 1 = Reset sequence counters (reset prior
             to EACH series of reads or writes)

Bit 7    0    Sets shift rate = clock frequency


Status Byte Bit Assignments are:

Bit        7  6  5  4  3  2  1  0

Output     0  0  0  0  0  0  0  OVF

OVF = 1 if overflow (only valid after a divide command has been done).  If overflow occurs, the data in the X, Y and Z registers will be changed.


Register Contents are:

| Reg | Before Div. | After Div | Before Multip. | After Multp. |
|-----|-------------|-----------|----------------|--------------|
| X | Divisor | No Change | Multiplicand or Multiplier | No change |
| Y | Dividend,MSB | Remainder | | Product, MSB |
| Z | Dividend,LSB | Quotient | Multiplicand or Multiplier | Product,LSB |

ECG Signal Processing includes the Linear Amplifiers, A/D Converter and Pacemaker detector.

The Linear Amplifier for each channel has three gain levels independently setable under program control. Recovery time from a gain adjustment is less than 100 ms. The gain levels are controlled by the Channel/Gain Select I/O Port ($23).  This same I/O port controls whether the output of the channel A or B amplifier is connected to the A/D converter.  For dual channel applications, each channel should be read by the software on alternate 2 Ms. interrupts.

When the AMU is docked in the PCU, it can bypass its instrumentation amplifiers and monitor the isolated analog data from the PCU by setting control byte bit 06 high. When the AMU is ambulatory, it should set bit 06 low in order to monitor the patient leads. For diagnostic purposes, the AMU instrumentation amplifiers can be tested when the AMU is docked by closing the appropriate cross points in the Lead Sense port ($22) and setting control byte bit 06 high.

The amplifier bandpass is from .05 Hz to 100 Hz. The rolloff is 30 DB per octave above 100 Hz in order to prevent a pacemaker spike, if one is present, from reaching the A/D converter.

The A/D converter generates an 8 bit sample every 2 ms with a conversion time of 105 to 144 microseconds*. The first 8 channels of the 256 available in the 8 bit sample are not used by the A/D converter. Sample values 00 to 07 are not generated by the hardware and are reserved for system use. An analog input of +4.60 volts is the full scale input of the converter. The converter output will be $FF for an input voltage equal to or greater than the full scale value. The output of the converter will not fold back to zero if its full scale input is exceeded. The samples are loaded into memory by reading input port 01 after each 2 ms interrupt. (Input Port 01 should not be read until 32 Processor T states have elapsed to insure that the A/D interrupt is reset.

The exact conversion time depends on the CPU clock frequency. With a CPU clock frequency of 5 MHz, the conversion time will be 112 microseconds with a maximum variation of plus or minus 6 microseconds. With a CPU clock of 8 MHz, the conversion time will be 139 microseconds with a maximum variation of plus or minus 7 microseconds.

The Pacemaker Detector is used to detect the presence of pacemaker pulses in the range of 1.0 mv to 1.0 v with pulse widths in the range of 100 us to 2 ms. When a Pacemaker pulse of either polarity is detected, the Pacemaker Interrupt flip-flop will be set, causing a RSTA interrupt to be asserted. In addition, the appropriate polarity indicator bits in the status latch will be set. If a positive-going pulse transition is detected, bit 06 of the status word will be set, and if a negative transition is detected, bit 07 will be set. Upon detection of the leading edge of a pacemaker pulse, the pacemaker interrupt handler should enable the baseline protect function for a period of time in order to prevent the pacemaker pulse or the recharge pulse from overloading the ECG amplifiers. The amount of time that the baseline protect function is enabled is software-controlled akd is dependent upon the make, model and type of pacemaker being used. After the contents of the status latch have been read by the MPU, the MPU should assert the RPMSH command in order to clear the status latch. Whenever RPMSH is asserted, the Pacemaker Interrupt flip-flop will also be reset, clearing the RSTA interrupt request. It is important to note that the pacemaker status latch must be cleared immediately after the baseline protect function is disabled in order to prevent spurious setting of the status latch.

The Pacemaker Detector can be set up to monitor either channel A by writing $00 to port $22 or channel B by writing $80 to the same port.

The impedance of each of the four patient leads may be independently tested when the AMU is ambulatory. (A separate lead impedance measuring circuit is included in the PCU for use when the AMU is docked). The MPU should first set the pacemaker detector to monitor the channel corresponding to the lead(s) to be tested. Next, the MPU should close, then open, the corresponding cross point in port $22 for the patient lead to be tested. The cross point selected should be closed for 50 us which will cause the lead sense threshold to be set to approximately 12K ohms. If the patient lead impendance is greater than 12K ohms, bit 06 of the status latch will be set approximately 75 usec. after the lead sense pulse for the RL or LL leads is enabled and bit 7 will be set approximately 75 - 500 usec. (depends on impedance) after the lead sense pulse is disabled. When leads LA or RA are tested, bit 07 will be set 75 usec after the lead sense pulse is enabled, and bit 06 will be set 75 - 500 usec. (depends on impedance) after the lead sense pulse is disabled.

It is important to note that the patient lead impedance routine must be executed between pacemaker pulses in order to prevent the possibility of missing a pacemaker pulse.

The Patient Interface includes lead sensing, interface, audible alarm, time display, and symptom button. Connection of the patient leads is sensed by a shorting jumper in the connection which drives bit 0 of port 03 in the MPU. A low indicates that the patient leads are connected.

Patient Connector to Data Board P7 Interface is as follows:

| Signal Name | Mnemonic | Patient Connector Pin # | Data Board Pin # |
|---|---|---|---|
| Bootstrap Output | ANBTA | 1 | 7 |
| Patient Lead 2 (RA,-) | AN2A | 2 | 6 |
| Analog Reference (C) | ANREF | 3 | 5 |
| Patient Lead 3 (RL,+) | AN3A | 4 | 4 |
| Patient Lead 4 (LA,-) | AN4A | 5 | 3 |
| Patient Lead 1 (LL,+) | AN1A | 6 | 8 |
| Ground | GND | 7 | 9 |
| Spare | SPARE | 8 | 1 |
| Lead Sense Input | SENSEL | 9 | 2 |

The Audible Alarm is driven by bit 3 of port 02 (Control Byte). Whenever the bit is on (high), a continuous 4 KHz tone is sounded.

The Time Display contains a watch chip, 12 hour LCD display with AM/PM and crescent symbol indicators and a self-contained crystal controlled clock oscillator (accuracy within 30 seconds/24 hours).

Control of the Time Display is done by opening and closing certain cross point switches which are controlled by port $21. The Crescent Symbol is controlled by alternately opening and closing cross point X3Y3. The following sequence of data should be written to port $21 in order to turn the Crescent Symbol

on, then off: $8F-$0F-$8F-$0F. To enable the time set function, write $80 to port $21 which closes cross point X1Y1. While the cross point is closed, the MPU can increment hours or minutes by pulsing X1Y2 and X1Y3 respectively. Hours and minutes can be incremented simultaneously. When cross point X1Y1 is opened, the enable time set function is disabled and time will resume at zero seconds. To reset the time to 12:00 midnight, close, then open cross point X2Y1. Note that the clock is powered whenever the AMU is not in the "Data Save" or the "Inactive" modes.

Pressing the "Patient Symptom" button will cause bit 1 of input port 03 to be low and remain low for at least 1.5 seconds after the button is released. The program must test this bit at least once each second to insure that a depression is not missed.

The "Patient Symptom" status is not valid until 1.5 seconds after VL has stabilized. Therefore, the status is not valid during the Leadless mode and should be ignored when the AMU is in that mode.

The AMU is powered by two 9 volt "transistor" batteries connected in parallel and isolated by diodes. The diodes protect against damage caused by temporary reversal of polarity when the battery is installed.

While the AMU is functioning normally, the battery drain is approximately 25 ms with a CPU clock frequency of 4 MHz and approximately 43 ma with a CPU clock frequency of 8 MHz. This results in a life of about 18 or 10 hours per battery for a total of 36 or 20 hours with a CPU clock frequency of 4 MHz or 8 MHz respectively.

The AMU can, under program control, power
down all its control electronics including the MPU
and reduce the memory voltage to 4 volts. The only
functions remaining active are the Time Display and
a 1024 millisecond wake-up timer. This reduces the
battery drain to about 2.5 ma. At this level, battery
life is increased by a factor of 10 or 17 (depending
on ehe CPU clock frequency) and in addition, memory
data will be retained for fifty hours after the
batteries have dropped to the five volt level. The
MPU program begins a power down sequence by issuing
a "Reset Power Up" (RPU) function by writing to port
$60. The effect of RPU is modified by the state of bit.
7 of port $20. If bit 7 is high, the power down is
permanent and power is not restored until the AMU is
docked (Data Save Mode, see Section 7.3). If bit 7
is low, power is restored and the MPU restarted once
during each 1024 millisecond interval (Leadless
Mode). The restart operation causes the MPU program
counter to be set to location 0000, the contents of
the MPU I and R registers to be cleared and all interrupts
except the NMI interrupt to be disabled. In order to
prevent AMU memory write over, the stack pointer should
be initialized by the appropriate instruction beginning
at location $0000. It should be noted that program
execution always begins at location $0000 and that the
NMI interrupt is not used in the Dual Channel AMU.
The permanent power down mode is called the "Data Save"
condition and the temporary power down mode is called
the "Leadless" condition since it is normally invoked
whenever the patient leads have been disconnected.

A third power down mode, called "Power Save", can
be invoked by the CPU. The mode is entered by the CPU
setting bit 7 of the control byte to a high state. At the end of the current instruction cycle, the CPU will stop executing and will keep itself in the low power mode. During this time, all of the internal control states and register values of the CPU will be maintained. The "Power Save" mode is exited and the CPU returned to normal operation upon receipt of an interrupt from the A/D converter. During the "Power Save" mode, the total operating current of the AMU is reduced by about 75 percent.

Battery status is passed to the MPU on bit 3 of the AMU status port. When the battery voltage drops to 5.25 volts, it will cause the status bit to go low. The MPU should test this bit at least once every ten seconds. If a low battery condition is detected, the bit should be tested at least twice more before the AMU is powered down. This is necessary in order to insure that the decision to power down is not based on a momentary or transient low battery status indication. With a battery voltage below 5 volts, MPU operation becomes unreliable and the program must enter the "Data Save" condition within ten minutes.

The personality PROM is a high current device and hence is powered by the PCU only. The 5 volt auxiliary power line supplies power to the PROM and also overrides the "Leadless" or "Data Save" conditions in the AMU.

When batteries are first installed in the AMU, power remains shut off and the AMU is in the "Inactive" state. The AMU is activated by the PCU after a battery test is performed.

The instruction set and op codes of the NSC800 are identical to those of the Z80 microprocessor and the code is executed using the same number of T states as the Z80.

Software written for the Z80 will run on the NSC800 without change unless I/O location $BB is used. Another location should be assigned since location $BB is an on-chip write only register used for the interrupt mask. The NSC800 expanded interrupt capability is transparent to the user unless specifically invoked by the user software.

The status flags on the NSC800 are identical to those on the Z80. There is no difference between the positions of the individual bits in the flag register nor in the manner in which the flags are set or reset due to an arithmetic or logical operation. Testing of the flags is also the same.

The AMU communicates with the PCU (Procedure Control Unit) via a 41 pin connector.

## AMU/PCU INTERFACE

| Pin # | Signal Name | Mnemonic | Signal Type | Source AMU | PCU |
|---|---|---|---|---|---|
| 23 | Address/Data Bus 0 | AD0H | CMOS | X | X |
| 25 | Address/Data Bus 1 | AD1H | CMOS | X | X |
| 27 | Address/Data Bus 2 | AD2H | CMOS | X | X |
| 29 | Address/Data Bus 3 | AD3H | CMOS | X | X |
| 31 | Address/Data Bus 4 | AD4H | CMOS | X | X |
| 33 | Address/Data Bus 5 | AD5H | CMOS | X | X |
| 35 | Address/Data Bus 6 | AD6H | CMOS | X | X |
| 37 | Address/Data Bus 7 | AD7H | CMOS | X | X |
| 1 | Address But 8 | A8H | CMOS | X | |
| 3 | Address Bus 9 | A9H | CMOS | X | |
| 5 | Address Bus 10 | A10H | CMOS | X | |
| 7 | Address Bus 11 | A11H | CMOS | X | |
| 9 | Address Bus 12 | A12H | CMOS | X | |
| 11 | Address Bus 13 | A13H | CMOS | X | |
| 13 | Address Bus 14 | A14H | CMOS | X | |
| 15 | Address Bus 15 | A15H | CMOS | X | |
| 30 | Interrupt | INTROL | CMOS | | X |
| 17 | Write Strobe | WRL | CMOS | X | |
| 16 | Read Strobe | RDL | CMOS | X | |
| 12 | IO/Memory Select | IO/ML | CMOS | X | |
| 19 | Address Latch Enable | ALEL | CMOS | X | |
| 14 | Reset | RSTL | CMOS | | X |
| 18 | *Crystal Clock Out | XCLKH | CMOS | X | |
| 8 | *Power Save | PSL | CMOS | X | |
| 6 | PROM Enable | PROMENL | TTL | | X |
| 38 | AMU Present | AMUPRL | CMOS | X | |
| 26 | *Status S1 | S1H | CMOS | X | |
| 20 | *Status S0 | S0H | CMOS | X | |
| 39 | Lead-1 | L1 | ANALOG | | X |
| 41 | Lead-2 | L2 | ANALOG | | X |
| 36 | T Clock Out | TCLKH | CMOS | X | |
| 24 | Battery Voltage-1 | VB1 | POWER | X | |
| 22 | Battery Voltage-2 | VB2 | POWER | X | |
| 28 | Battery Voltage | VB | POWER | X | X |
| 4 | Memory Voltage | VM | POWER | X | |
| 2 | Logic Voltage | VL | POWER | X | |
| 21 | Auxiliary 5 Volts | VREF | POWER | | X |
| 34 | Reference Voltage | VREF | POWER | X | |
| 10 | Ground | GND | POWER | X | X |
| 40 | Ground | GND | POWER | X | X |
| 32 | Docked Status | DOCKL | CMOS | | X |

at Pin 18, 8, 26 and 20 the signals are not used by the PCU and are used for test only.

## MPU FUNCTIONS NOT ON AMU/PCU INTERFACE CONNECTOR

| Signal Name | Mnemonic | Signal Type |
|---|---|---|
| Wait | WAITL | CMOS |
| Reset Out | RSTOH | CMOS |
| Refresh | RFSHL | CMOS |
| Interrupt Acknow. | INTRAL | CMOS |
| Restart C | RSTCL | CMOS |
| Restart B | RSTBL | CMOS |
| Restart A | RSTAL | CMOS |
| Non-Maskable Interrupt | NMIL | CMOS |
| Bus Request | BREQL | CMOS |
| Bus Acknowledge | BACKL | CMOS |

The PCU holds all I/O lines in a high impedance state until the AMU is locked in place and powered up at the docking station. The maximum loading is 100K ohm and 20 pf regardless of whether the PCU is powered up or not. (The address, data and control buffers on the PCU AMU interface should not load the AMU I/O lines regardless of whether the PCU is powered or not in order to prevent "hanging of the AMU I/O lines in the event of a power failure in the PCU).

The presence of an AMU at the PCU Docking Station is sensed electrically through the interface connector. The PCU program then sets, via software control, "Docked" status to the AMU.

The patient connector must be physically disconnected before the PCU interface connector can be mated. Patient monitoring while docked is accomplished by plugging the patient leads into the patient connector on the PCU. The ECG signal is passed through an isolation amplifier to the AMU/PCU interface connector and into the AMU input terminals. The isolation amplifier is relatively wide band and linear so as to preserve the integrity of the patient ECG and Pacemaker signals. In addition, it is optically coupled to insure a maximum of 10 microamperes leakage at the patient connection. The amplifier has a gain of 25 which is compensated for by an attenuation of the same amount in the AMU.

The docked condition is entered from the "Leadless", "Data Save" or "Inactive" conditions. The AMU "Inactive" state is detected by the PCU hardware by sensing VM 3 volts. The "Data Save" condition is not sensed by the PCU hardware.

An inactive AMU docked at the PCU is first checked for battery condition. This is done under PCU program control by applying a 130 ma.current pulse for 500 ms to each battery while monitoring output voltage. If the output voltage drops below 8.25 volts, the battery will not provide sufficient energy to last for a full 24 hour monitoring period. The battery should be replaced.

The PCU activates an inactive AMU by driving Vm to 5 volts for 100 ms. This is followed by a load sequence.

The PCU supplies +12 volts to the AMU via the VB pin on the interface connector during the time the AMU is docked. This insures against loss of data in a low battery situation and prevents draining of the batteries while the AMU is docked.

The "Data Save" or "Leadless" condition in the AMU is overriden by application of 5 volt auxiliary power from the PCU. The PCU first monitors Vm to insure that the AMU is powered down (VM $<$ 4.3 volts) and then applies 5 volt auxiliary power.

A power failure in the PCU causes the AMU to be ejected when power has been restored to the PCU. If a "Data Save" condition existed prior to docking, the AMU reverts to that state. When power is restored, the AMU may be redocked without loss of data.

The "Data Save" condition is controlled by AMU program command only and requires initialization by the program on power up. Ehe Data Save status bit (04) of port 03 should be tested by the MPU and if it is low, the program should write 00 to port $20 (the RPU port) to remove the AMU from the "Data Save" condition. The above procedure must be done while the unit is docked in the PCU with AUX 5 volts being on. Note that even though AUX 5 volts is held high during the initialization procedure, ANYTIME THE CPU CAUSES A RPU

TO BE GENERATED, THE AMU WILL STILL MOMENTARILY POWER DOWN AND BEGIN EXECUTION AT LOCATION $0000 WHEN THE DELAYED POWER UP TIME HAS EXPIRED (1088 MS MAX).

The above procedure can also be used to remove the AMU from the "Data Save" condition for test purposes.

The AMU can be returned to the "Inactive" condition by the PCU pulsing VREF to zero volts for at least two seconds. Caution: this will cause all of the contents of the AMU memory to be lost.

The PCU initiates the load sequence by asserting "Reset". This signal forces any activity in the AMU to an abrupt halt by resetting the MPU. While holding "Reset" low, the PCU initiates its output DMA channel, sets its PROM enable bit, enables AUX 5, then releases "Reset". The AMU begins program execution at location $0000 causing the PROM to be enabled. The first 20 bytes of the PROM contain a primtive bootstrap loader which causes a more complex load routine from the PCU to be loaded into AMU memory. The remainder of the Program is loaded into AMU memory via the AMU Input Operation.

AMU input is DMA controlled in the PCU and programmed I/O in the AMU. The DMA priority is sufficiently high to insure that when the AMU inputs data at its maximum rate of 200K bytes/second, no data will be lost.

A transfer is initiated by the PCU setting the Input Message Pending bit (06) high and activating its output DMA channel which causes the Ikput Buffer Full bit (07) to be set and also causes the interrupt (RSTB) line to be set low.

The AMU MPU first tests the Input Buffer Full bit (07) of the PCU status port (04) to verify that the bit is high, indicating that the input buffer is full. The AMU MPU then causes input port 07 to be accessed, transferring the first byte of data from input port 07 to AMU memory. The selection of input port 07 causes the Interrupt flip-flop and the Input Buffer Full flip-flop in the PCU to be reset. (RSTB goes high and the Input Buffer Full lines goes low). The Input Buffer Full status bit is set high by the PCU when the next byte to be transferred has been loaded into the input buffer. (If the Input Buffer Full status bit has not been set by the time the AMU is ready to input another byte of data, the AMU MPU will remain in a wait loop and continue to test the status bit until it is set high, at which time the AMU will input the next data byte). After each data transfer or status test with Input Buffer empty, the AMU MPU also tests the Input Message Pending bit (6) of the PCU status port. When the last byte of data has been transferred, the PCU sets the Input Message Pending bit low, causing the AMU to terminate the input sequence.

AMU output is via an independent DMA channel in the PCU and program controlled in the AMU. The PCU DMA channel is capable of supporting a 200K byte/second data transfer rate.

The "Output Ready" status bit (AMU input port 04 bit 2) is controlled by the PCU and when high, indicates that the PCU DMA channel is armed and ready to accept output from the AMU. This status bit is tested by the AMU prior to initiating an output sequence.

The AMU issues an output command, causing the first byteof data to be transferred to the PCU via AMU output port 07. The selection of output port 07 causes the Output Buffer Empty flip-flop in the PCU to be reset. The Output Buffer Empty status bit (0) is set high by the PCU after the DMA transfer has been completed. (If the Output Buffer Empty status bit has not been set by the time the AMU is ready to output another byte of data, the AMU MPU will remain in a wait loop and continue to test the status bit until it is set high, at which time the AMU will output the next data byte). When the entire block of data has been transferred, the AMU sends "Reset Output Ready" by selecting output port 06 which cause the PCU to reset the "Output Ready" status bit, terminating the sequence. If the AMU attempts to send a longer block of data than the PCU DMA channel has been set up for, the additional data will be lost.

The digitized ECG data from the A/D converter is continuously monitored by the MPU. The Interleaved data, representing ECG channels A and B, are separated by the MPU and output to AMU ports $25 and $2D respectively. While the AMU is docked, the PCU continuously monitors the activity of these ports, captures the data and transfers it to the PCU under program control.

The AMU personality/bootstrap PROM is powered by the PCU via the 5 volt auxiliary power input. The PROM is read as a memory device by the AMU MPU after power has been supplied.

AMU Docking Procedure is as follows:

- DETECT DOCKED AMU VIA PCU "AMU PRESENT" INTERRUPT
- SUPPLY VB TO AMU
- SUPPLY POWER TO AMU INTERFACE
- DETERMINE IF AMU IS ACTIVATED
    - MEASURE VM
        - VM $<$ 3 VOLTS == $>>$ AMU NON-ACTIVATED
        - VM $>$ 3 VOLTS == $>>$ AMU ACTIVATED
- DETECT USED/BAD BATTERIES IN AMU
    - MEASURE VB1 & VB2
    - APPLY AMU BATTERY TEST LOAD
    - WAIT 500 MILLISECONDS
    - MEASURE VB1 & VB2 (DETECT USED/BAD BATTERIES)
        - VB1 and VB2 $>$ 8.3 VOLTS == $>>$ OK BATTERIES
        - VB1 OR VB2 $<$ 8.3 VOLTS == $>>$ USED BATTERIES
        - VB1 OR VB2 $<$ 6.5 VOLTS == $>>$ BAD BATTERIES
    - REMOVE TEST LOAD
- SET DOCKED STATUS TO AMU

IF AMU IS NON-ACTIVATED:

- STOP
  (AMU IS ACTIVATED LATER ONLY WHEN PROCEDURE IS INITIATED)

IF AMU IS ACTIVATED:

- WAIT 2.5 SECONDS
  (AMU WAKES UP & ENTERS DATA-SAVE)
- RAISE AUX 5
  (WAKE AMU UP FROM DATA-SAVE)
- WAIT 2.5 SECONDS
  (AMU PERFORMS WAKE-UP CHECKS)
- TALK NORMALLY TO AMU

IF AMU DOES NOT RESPOND CORRECTLY TO 1ST MESSAGE:

- FORCE 'INPUT READY' LOW
- FORCE 'AMU INTERRUPT' LOW
- FORCE 'OUTPUT READY' LOW
- DROP AUX 5
- WAIT 100 MILLISECONDS
  (ALLOW AUX 5 TO DROP)
- SET AMU RESET
- WAIT 1 MILLISECOND
- CLEAR AMU RESET
- WAIT 5 SECONDS
  (AMU WAKES UP & ENTERS DATA-SAVE)
- RAISE AUX 5
  (WAKE AMU UP FROM DATA-SAVE)

- WAIT 5 SECONDS
  (AMU PERFORMS WAKE-UP CHECKS)
- TALK NORMALLY TO AMU

IF AMU STILL DOES NOT RESPOND CORRECTLY TO NEXT MESSAGE:

- AMU IS NON-OPERATIONAL


        AMU ACTIVATION/LOADING is as follows:

- ACTIVATE AMU POWER
        - SUPPLY VB TO AMU
        - WAIT 10 MILLISECONDS
        - SET AMU RESET
        - SKIP VM PULSE IF AMU ALREADY ACTIVATED
                - MEASURE VM TO DETERMINE IF ACTIVATED
                        - IF VM > 3 VOLTS == >>   SKIP PULSE OF VM
                        - IF VM < 3 VOLTS == >>   CONTINUE
        - PULSE VM TO ACTIVATE AMU
                - SUPPLY VM TO AMU   ------------------/RETRY 20    /
                - WAIT 100 MILLISECONDS               /TIMES MAX.  /
                - DROP VM SUPPLY TO AMU               /AMU IS NON- /
                - WAIT 10 MILLISECONDS                /OPERATIONAL /
                - MEASURE VM TO DETERMINE IF ACTIVATED/IF EXCEEDED./
                        - IF VM < 3 VOLTS == >>   TRY AGAIN
                        - IF VM > 3 VOLTS == >>   CONTINUE
                - WAIT FOR VM TO STOP RISING < --------------------------.
                        - WAIT 100 MILLISECONDS< ----------------/
                        - MEASURE VM
                                - IF NEW VM > PREVIOUS VM == >>  ·LOOP -------/
                                - IF NEW VM NOT > PREVIOUS VM == >>   CONTINUE
- SUPPLY POWER TO AMU INTERFACE
- LOAD AMU SOFTWARE
        - SEND 'LOADER' MODULE TO AMU
                - SET. AMU RESET
                - ENABLE AMU PERSONALITY PROM
                        - SUPPLY AUX 5 TO AMU
                          (AMU.PERSONALITY PROM POWER)
                        - ENABLE THE AMU PERSONALITY PROM
                        - WAIT FOR 100 MILLISECONDS
                - SET UP DMA TO SEND LOADER TO AMU
                  (NOTE: NEITHER MODULE HEADER NOR CHECKS UM ARE SENT
                - RELEASE AMU RESET
                - WAIT UP TO 8 SECONDS FOR AMU TO INPUT 'LOADER'
                  (IF NOT INPUT IN 8 SECONDS, AMU IS NON-OPERATIONAL
                - WAIT UP TO 6 SECONDS FOR AMU TO SEND 1ST 'ACK'
                  (ACK CONSISTS OF DROPPING 'OUTPUT READY' & INDICATION
                  THAT THE AMU HAS INPUT THE 'LOADER')
                  (IF NOT ACK'ED IN 6 SECONDS, AMU IS NON-OPERATIONAL

- RAISE 'OUTPUT READY' AGAIN
- DISABLE THE AMU PERSONALITY PROM
- PREVENT AMU FROM POWERING DOWN DURING NEXT 1 SECOND
    - WAIT 1.25 SECONDS
        (AMU EXITS FROM DATA-SAVE MODE & MAY TRY TO
        POWER DOWN FOR UP TO 1+ SECOND IN THE PROCESS)
- WAIT UP TO 1.5 SECONDS FOR AMU TO SEND 2ND 'ACK'
    (ACK CONSISTS OF DROPPING 'OUTPUT READY' &
    INDICATES THAT THE AMU HAS CORRECTLY CHECKS UMMED
    THE 'LOADER')
    (IF NOT ACK'ED IN 1.5 SECONDS, AMU IS NON-OPERATIONAL
- REMOVE AUX 5 SUPPLY TO AMU
- WAIT 100 MILLISECONDS
- SEND 'MONITOR' SOFTWARE MODULE TO AMU
- SET UP DAM TO SEND MONITOR TO AMU
    (NOTE: MODULE HEADER NOT SENT, CHECKS UM IS SENT)
- WAIT UP TO 15 SECONDS TO INPUT 'MONITOR'
    (IF NOT INPUT IN 15 SECONDS, AMU IS NON-OPERATIONAL
- WAIT UP TO 6 SECONDS FOR AMU TO SEND 'ACK'
    (ACK CONSISTS OF DROPPING 'OUTPUT READY' &
    INDICATES THAT THE AMU CORRECTLY INPUT THE 'MONITOR
    (IF NOT ACK'ED IN 6 SECONDS, AMU IS NON-OPERATIONAL
- WAIT 1 SECOND
    (AMU PERFORMS INITIALIZATION)
- TALK NORMALLY TO AMU

AMU UN-DOCKING PROCEDURE

IF AMU IS OPERATIONAL:

- WAIT FOR AMU TO BE READY TO UNDOCK
    - SEND MESSAGE TO AMU INDICATING UNDOCK COMING
    - WAIT UP TO 2.5 SECONDS FOR AMU RESPONSE
        (RESPONSE INDICATES AMU IS READY TO BE UNDOCKED)

IN ALL CASES:

- PREPARE INTERFACE FOR NEXT AMU DOCKING
    - FORCE 'OUTPUT READY' LOW
    - FORCE 'AMU INTERRUPT' LOW
    - FORCE 'INPUT READY' LOW
    - CLEAR AMU RESET
    - DISABLE THE AMU PERSONALITY PROM
    - DROP DOCKED STATUS TO AMU
    - REMOVE AUX INTERFACE POWER
    - REMOVE AUX 5 SUPPLY TO AMU
    - REMOVE VM SUPPLY TO AMU
    - REMOVE VB SUPPLY TO AMU

```
- ATTEMPT AMU DEACTIVATION IF APPROPRIATE
  (IF AMU WAS NOT ACTIVATED AT DOCKING & NO PROCEDURE
  INITIATED)
        - PULL DOWN VM FOR 1 SECOND
- EJECT THE AMU
      - ENABLE AMU EJECT SOLENOID
              - WAIT UP TO .25 SECOND FOR AMU PRESENT
                  STATUS TO GO AWAY
              - DISABLE AMU EJECT SOLENOID
              - IF AMU NO LONGER PRESENT -----------------------.
              - WAIT .75 SECOND <
              - ENABLE AMU EJECT SOLENOID        LOOP MAXIMUM OF
              - WAIT UP TO .1 SECOND FOR          9 TIMES.IF AMU
                AMU PRESENT STATUS TO GO AWAY     STILL PRESENT,
              - DISABLE AMU EJECT SOLENOID        DISPLAY ERROR MSG.
              - IF AMU STILL PRESENT
              - WAIT 100 MILLISECONDS < -------------------------.
```

The AMU electronic package consists of two 32K x 8 memory boards, an MPU board and a data board interconnected by a common 8 bit bidirectional address/data bus, an address bus and several control signals. Figure 1 is the overall block diagram of the AMU and Figures 2, 3 and 4 are the block diagrams for the MPU, Memory and Data boards respectively.

The NSC800 is an 8 bit microprocessor that functions as the central processing unit. System functions such as vectored priority interrupts, power save control, clock generator and bus controller are incorporated on the device. The CPU is capable of addressing 64K bytes of memory and 256 I/O devices.

The NSC800 uses a multiplexed data bus. The address is split between the higher 8 bit address bus and the lower 8 bit address/data bus. During the first cycle the address is sent out. The lower 8 bits are latched into the peripherals by the Address Latch Enable (ALE). During the rest of the machine cycle, the data bus is used for memory of I/O data. Refer to Figures 5 and 6 for detailed timing information.

The microprocessor provides RDL, WRL and IO/ML signals for bus control. Bus status outputs SO and S1 indicate encoded information regarding the ensuing M cycle as follows:

| S1 | SO | State |
|----|----|-------|
| 0 | 0 | Halt |
| 0 | 1 | Write |
| 1 | 0 | Read |
| 1 | 1 | Opcode Fetch |

At the falling edge of ALE time during a memory reference cycle, the least significant byte of address and bits A8, A9, and A10 are stored in the memory address latches to provide ten bits of buffered address data for the memory array. At the falling edge of ALE time during an I/O reference cycle, the 8 bit port address is stored in bits 0-7 of the memory address latches. Address A8-A15 also contain the port address; hence, bits 8-10 of the latches contain the same data as bits 0-2 of the latches during an I/O cycle.

The 32 byte PROM is a bipolar device and can only be activated when the AMU is docked in the PCU. Both the power source and the enable for the PROM are supplied by the PCU. To activate the PROM, the PCU raises AUX 5 and will cause PROMENL to go low whenever the AMU CPU generates a memory read address in the range $00-$1F. This causes the high speed power switch to supply power to the PROM and to disable the AMU RAM. Since the chip select of the PROM is connected to RDL, the PROM will output data to the system bus any time power is supplied to it and RDL is low simultaneously. Note that regardless of the docked status of the AMU, the CPU always has write ccess to the memory space behir the PROM.

The Memory Hi/Lo Decoder is used to select which memory board is to be enabled at any given time. The memory enable is qualified by several status and control lines to ensure that activation of the RAM memory is properly coordinated with other system activity. The following signals provide the indicated functions within the decoder:

a. IO/M - Permits RAM memory to be enabled only during a memory reference cycle.

b. PWUPH - Prevents spurious enabling of the RAM memory when VL is powered down.

c. PROMENL - Prevents RAM memory from being accessed when the PROM is enabled.

d. DPWUPH - Prevents spurious enabling of the RAM memory when VL is turned on.

e. A15 - When low, permits memory address SO-S7FFF to be accessed and when high, permits address S8000-SFFFF to be accessed.

The wait state generator provides for the insertion of one wait state during an opcode fetch cycle causing an op code fetch cycle to consist of five T states instead of four. During an I/O cycle, one wait state in inserted automatically by the MPU, resulting in an I/O cycle of four T states. The op code fetch cycle wait state is generated when SOH and S1H are both high indicating than an op code fetch cycle is in progress. The wait state generator is not used in NPU boards which have a CPU clock frequency of 6 MHz or less.

The power supply consists of a high efficiency 100KHz, stepdown switching regulator. It converts the 9 volts from the batteries to 5 volts to power the AMU with an average conversion efficiency of 85 percent.

The power supply provides two 5 volt outputs, VM and VL. VM is the voltage which is used to power the memory array. It is 5 volts when the power enable (PWENH) control line is high indicating that the AMU is in the active mode. When the AMU is in either the temporary power down mode (Leadless) or the permanent power down (Data Save) mode, PWENH will be low and VM will be 4 volts reducing power consumption. If the battery output drops below 4 volts, VM will track the battery voltage down to 2 volts at which time VM will drop to zero volts. If this should occur, all data in the memory will be lost.

The VL supply derives its power from VM through a complimentary MOS power switch. When PWENH is high, VL is switched to VM causing VL to be about 0.1 volt below VM (or about 4.9 volts). When PWENH is low, VL is switched to ground to insure that VL remains at zero volts.

For test purposes, the VM voltage and hence the VL voltage can be lowered via the VREF input. A 0.1 volt change on VREF will cause a 0.4 volt change on VL and VM simultaneously. VREF is supplied by a stable 1.23 volt band gap zener diode.

The power control and power up initialization of the AMU are controlled by the signals listed below and serve the indicated function.

a. AUX 5 - Used as a power source for the PROM and to temporarily override the Leadless or Data Save power down modes when the AMU is docked in the PCU.

b. PWUPH - Provided by the power control flip flop on the data board. When high, it will cause VL to turn on and a power up initialization sequence to begin.

c. PWENH - The logical "OR" function of AUX 5 and PWUPH. When it goes high, it will cause VL to be turned on and a 64 ms delay period to be generated by the delay flip-flop on the Data Board. During the delay period, the CPU is held reset. When the delay terminates, RESTL will be released, the memory will be enabled by DPWUPH and the CPU will always begin program execution at location X'0000'. When PWENH goes low, it reduces power consumption by turning off VL and lowering VM.

d. RESETL - It can be generated as a result of PWENH going high or it can be generated by the PCU when the AMU is docked. If it is generated by the later method and if the PCU enables PROMENL and AUX 5, the MPU will cause the bootstrap loader to be executed.

e. PSL - Used to place the AMU in the Power Save mode, causing AMU power consumption to be reduced by a factor of four to one in this mode. All CPU registers, flags and status information are maintained during power save. The mode is entered by the MPU writing a high to Bit 7 of Port 02. The mode is exited upon receipt of an interrupt from the A/D converter.

The I/O decoder is a one of eight decoder with appropriate gating to allow it to recognize a CPU I/O cycle and to select the I/O port being addressed by the CPU. Refer above for I/O port assignments. The following inputs to the decoder serve the indicated functions:

a. IO/ML - When high, indicates that the CPU is currently executing an I/O cycle.

b. IOENL - This signal is low whenever the CPU executes a read or write cycle. It is the logical "OR" function of the inverted read and write strobes.

c. A13H - When low, allows selection of I/O ports $00 - $03. When high, it will cause ports $20 - 23 to be selected.

d. MA8-MA09 - The least significant two bits of the I/O port address when the CPU is executing an I/O cycle. They are used by the decoder to select the appropriate I/O port.

e.   MA10 - When low, allows selection of ports $00 -
$03 or ports $20 - $23.   When high, allows selection of
ports $04 - $07 or ports $24 - $27, which are PCU-
related ports.   Therefore, this signal provides the
necessary separation between AMU and PCU-related I/O
ports.

The Multiplier/Divider is an 8 bit RCA CDP1855
CMOS device which serves to provide the hardware divide
and multiply function in the AMU.   Refer to above for
further details.

The Bus Transceiver is used to provide bidirectional
beffering between the data bus on the data board and the
main system bus.   The direction control for the transceiver
is obtained from the I/O read (IORL) signal.   It will be
low-only when the CPU is executing an I/O read cycle at
which time it will cause the Bus Transceiver to send data
from the data board data bus to the system bus if MA10
is low.   On any other type of CPU cycle, IORL will be
high and the Bus Transceiver will transmit data from the
system bus to the data board data bus if MA10 is low.
When MA10 is high, the bus transceiver is disabled and
cannot send data in either direction.   The MA10 control
line is used to prevent the simultaneous driving of the
AMU system bus by both the PCU and the AMU during an AMU
I/O read cycle.

The Control Byte Register consists of devices used
to store the Control Byte data for the Alarm enable and
AMU/PCU analog select function and one half of a dual
"D" type flip-flop used to store the Power Save control
bit.   The bit assignments are listed above.

The low order nibble of the AMU Status Port
consists of a 4 bit bus buffer which is normally
strobed by the CPU once each second to provide information
regarding patient lead connected status, dock status, "Patient Symptom" button status and battery status. The bit assignments are listed above.

The high order nibble consists of a 4 bit tri-state RS latch used to store the status of the pacemaker detector and also the status of the Data Save flip-flop. The latch can be reset when the MPU asserts the RPMSH (Reset Pacemaker Status) signal. Refer to above for details.

The Oscillator/Frequency Divider is used to provide an accurate 2 millisecond convert command to the A/D converter. It also provides a 1024 millisecond "wake-up" command to the power control logic and a 64 millisecond signal used to establish the 64 millisecond Delayed Power Up signal. A tuning fork resonator provides a stable 16 KHz source for the frequency divider chain. . The Oscillator/Frequency Divider is a CMOS device which is powered by the power control logic to provide the self latching Data Save function.

The Power Control Logic provides the power up (PWUPH) command to the switching regulator on the MPU board. It consists of three flip flops, FF1 - FF3 (refer to Figure 4). In order to place the AMU in the temporary power down (Leadless) mode, the CPU will generate an I/O write cycle to port $20 with data bit 7 set low. The port $20 write operation causes FF1 to be reset which in turn causes PWUPH to go low, turning off VL and lowering VM to 4 volts. About one second later, a "wakeup" pulse is generated by the Frequency Divider which sets FF1 causing PWUP to go high. This causes VL to be returned to 5 volts and VM restored to 5 volts.

The AMU is placed in the permanent power down (Data Save) mode by the CPU generating an I/O write cycle to port $20 with data bit 7 set high. This causes the outputs of both FF1 and FF2 to go low. In the same manner as the "Leadless Mode", VL is shut off and VM lowered to 4 volts. However, no "wakeup" pulse will be generated by the Frequency Divider because its "D" input is obtained from the output of FF1 which has been set low. Therefore, the AMU will remain in the "Data Save" mode until it is overriden by the PCU.

The 64 millisecond Delayed Power Up signal is generated by FF3. The input of FF3 is the Power Up command, but it does not get clocked through FF3 until the 64 millisecond signal occurs.

The first stage of the ECG Signal Processing Chain consists of a low noise differential amplifier having a fixed gain of ten. Its output is capacitor coupled to a second amplifier having an adjustable gain which is nominally set to ten.

The output of the adjustable gain stage drives an amplifier stage which has fixed gain settings of 4.4, 8.8 and 17.6 which correspond to the standard ECG gain settings of X 1/2, X 1 and X 2 respectively. The gain setting is determined by closing the appropriate cross points within the Gain Control Port ($23). The programmabl· gain amplifier also has an offset adjustment which is used to set the baseline to 2.3 volts which corresponds to a digital code of $82 (one half of full scale).

Additional cross points within the Gain Control Port determine whether the output of the channel A or B programmable gain stages is applied to the A/D converter. Selection of the channels is under software control.

The A/D converter receives one conversion command each 2 ms and after each conversion, generates an interrupt to the CPU.  The CPU responds by selecting the A/D port (01) and reading the contents of the A/D data register and storing the data in memory for further processing.  Reading port 01 also causes the A/D interrupt to be reset.

The VREF voltage (1.23 volts) is increased to 2.3 volts by the reference amplifier which is used by the A/D converter to establish a full scale of 4.6 volts, corresponding to a digital code of $ff.  Mid scale is 2.3 volts, corresponding to a digital code of $82.

The Clock Control cross point array is used to control the various time set parameters of the watch chip and also controls the Reset Pacemaker Status latch command (RPMSH).  The control functions are implemented by the MPU causing the related cross points to be opened or closed as required.  Refer to above for a listing of the detailed data assignments.

The following diagram indicates how the connections to the array are made:

```
                                        X1      .
      0-----------0-----------0-----------0----- ET SET
      :           :           :           :
      :           :           :           :
      :           :           :           :
      :           :           :           :
      :           :           :           : X2
      0-----------0-----------0-----------0----- RESET TIME
      :           :           :           :
      :           :           :           :
      :           :           :           :
      :           :           :           : X3
      0-----------0-----------0-----------0----- VT
      :           :           :           :
      :           :           :           :
      :           :           :           :
      :           :           :           : X4
      0-----------0-----------0-----------0----- RPMSH
      :           :           :           :
  Y1  :      Y2   :      Y3   :      Y4   :
      :           :           :           :
      VT        INC HRS    INC MIN/      GND
      (1.5V)               CRESCENT
```

The Lead Sense Cross Point Array provides the baseline protect function and the pacemaker channel select function.  It also provides a diagnostic function, in that it provides the capability of testing the AMU instrumentation amplifiers when the AMU is docked in the PCU.

The following diagram indicates how the connections to the array are made:

```
                                              X1
   0-------------0------------0------------0----- PACER A/B
   :             :            :            :      SELECT
   :             :            :            :
   :             :            :            :
   :             :            :            :
   :             :            :          : X2
   0-------------0------------0------------0----- BASELINE
   :             :            :            :      PROTECT CTRL
   :             :            :            :
   :             :            :            :
   :             :            :          : X3
   0-------------0------------0------------0----- CHB  (+)
   :             :            :            :      (RL)
   :             :            :            :
   :             :            :            :
   :             :            :          : X4
   0-------------0------------0------------0----- CHB  (-)
   :             :            :            :      (LA)
  Y1 :          Y2 :         Y3 :         Y4 :
   :             :            :            :
   VL           PCU ANLG    CHA (+)      CHA (-)
                (L1 IN)     (LL)         (RA)
```

The Channel/Gain Select Cross Point Array provides the capability for the MPU to independently control the gain of each channel and to control which channel is applied to the A/D converter.

The following diagram indicates how the connections to the array are made:

```
                                          X1
0----------0----------0----------0----- CHA GAIN X1
:          :          :          :
:          :          :          :
:          :          :          :
:          :          :        : X2
0----------0----------0----------0----- CHB GAIN X1
:          :          :          :
:          :          :          :
:          :          :          :
:          :          :        : X3
0----------0----------0----------0----- CHA ANLG IN
:          :          :          :
:          :          :          :
:          :          :          :
:          :          :        : X4
0----------0----------0----------0----- CHB ANLG IN
:          :          :          :
Y1 :       Y2 :       Y3 :       Y4 :
:          :          :          :
VR         CHA        CHB         TO ADC
(2.3V)     GAIN X2    GAIN X2
```

Cross Point Array Hex Data Table:

| X Point | Enable<br>Byte Val | Disable<br>Byte Val |
|---------|--------------------|--------------------|
| X1Y1 | $80 | $00 |
| X2Y1 | $81 | $01 |
| X3Y1 | $82 | $02 |
| X4Y1 | $83 | $03 |
| X1Y2 | $84 | $04 |
| X2Y2 | $85 | $05 |
| X3Y2 | $86 | $06 |
| X4Y2 | $87 | $07 |
| X1Y3 | $88 | $08 |
| X2Y3 | $89 | $09 |
| X3Y3 | $8A | $0A |
| X3Y3 | $8B | $0B |
| X1Y4 | $8C | $0C |
| X2Y4 | $8D | $0D |
| X3Y4 | $8E | $0E |
| X4Y4 | $8F | $0F |

The input to the Pacemaker Detector is obtained from switch U2, which determines which AMU channel the detector is to monitor and also provides the baseline protect function. The input is applied to the first stage amplifier through a high pass filter to remove the ECG signal. The first stage amplifier is a limiter amplifier with a gain of ten. Its output is limited to 1 volt p-p maximum in order to prevent overlad of the next stage when large pacemaker pulses are present. The second stage is a differentiator with a gain of ten. It produces a positive pulse during the rising edge of the pacemaker pulse and a negative pulse during the falling edge of the pacemaker pulse. The bidirectional output of the second stage amplifier is converted to two separate outputs (one for each polarity) by a dual comparator. The comparator outputs provide the status information for the pacemaker status latch. In addition, the outputs are diode-or'ed to provide for the generation of the RSTA interrupt for the MPU whenever either edge of a pacemaker signal is detected.

The baseline protect function is used to prevent overload of the ECG amplifiers during the time a pacemaker pulse is present. When the leading edge of a pacemaker pulse is detected, the RSTA interrupt line of the MPU is asserted. The MPU then asserts the inhibit function of switch U2, which removes the input from the ECG amplifiers. The inhibit function is active for a period of time which is determined by the MPU program

The generation of the lead sense pulse and lead selection functions are controlled by the U11 cross point array.  To test the integrity of a given lead, the MPU first selects the pacemaker detector to monitor the channel corresponding to the lead to be tested. The MPU then enables the appropriate cross point within the U11 array for 50 usec.  This causes a 0.5 ua, 50 usec current pulse to be applied to the selected patient lead.  If the patient lead impedance is greater than approximately 12K ohms, a pulse of sufficient amplitude will be generated such that it will cause the pacemaker detector to be triggered.  The MPU then tests the pacemaker status bits of the AMU Status port.  If the bits corresponding to the tested lead are set, this indicates to the MPU that the tested lead is open.

The Memory Array consists of two nearly identical circuit boards each one containing 32K bytes of memory, memory decoders and a bidirectional bus transceiver. The only difference between the two boards is a jumper pin which must be installed in the appropriate location to make the memory board respond to either the lower or upper 32K bytes of address range.

The 32K Memory Array consists of sixteen 2K x 8 CMOS static random access memory devices.  The address and data lines of all sixteen devices are wired in parallel.  Only one device may be accessed at a time reducing power consumption.

The Decoder is used to control which memory device in the array may be activated.  Only one decoder may be active at any given time and only one of the eight outputs of the active decoder may be selected at any given time thus allowing only one memory device in the array to be selected at any given time.  Upper/lower array select lines HXEL and LXEL along with address

lines A11-A13, A14H and A14L are used to determine which decoder and its associated output are to be selected.

The MEMENL ("or" function of RD and WR) control signal which is applied to each decoder is used to allow selection of the memory array only during the time read or write is active thus preventing bus contention and reducing power consumption.

The bidirectional bus transceiver is used to buffer the data bus on each memory card from the main CPU system bus. The transceiver is activated only during a memory read/write or op-code fetch cycle. It is inactive during an I/O cycle. The direction control of the transceiver is obtained from the read signal. Only when the transceiver is selected and read is low, can the transceiver send data from the memory array to the main system bus. When read is high, data is sent from the system bus to the selected memory array.

Input Dynamic Range of ECG Sigkals of this system provides an improved system.

Range of Linear Operations of Input Signal is +/- 5 MV min. The Slew Rate Change is 320 MV/sec max. The DC Offset Voltage Range is +/- 350 MV min. The Allowed Variation of Amplitude with DC Offset is +/- 5% max. The Accuracy of Input Signal Reproduction is as follows. The Overall Error for Signals is +/- .05 to +/- 5 MV: Target +/-10% or +/-50 UV max. The Upper Cutoff Frequency (3 dB) is 100 Hz min. The Allowable Overshoot to Step Response is 10% max. The Decay Time Constane After Step Input is 3 sec. min. The Lower Cutoff Frequency (3 dB) is 0.05 Hz max. The Gain Control and Stability incudes the following. The Error is +/- 3% max. The Gain Drift Initial is 0.2%/minute max. The Gain Drift, Total in One Hour is 0.5% max. In reference to base line, the baseline Drift Rate, referred to Input is 20 uv/sec. max. The Total Baseline Drift, Referred to Input is +/- 40 uv max. The Recovery Time After Gain Change is (To within +/- 4 ADC channels of center): 30 msec max. The Recovery Time After Exit from Leadless & Data Save Modes may be to within +/- 4 ADC channels of center) :2 minutes max. The Baseline Value vs. Gain is $81 +/- 8 ADC Channels for any gain setting. As to Input Impedance, the Impedance at 10 Hz is 20 megohms min. The Impedance at 100 Hz is 17 megohms min. The Input Bias Current is 25 Nanoamperes Max. As to the Common Mode Rejection, the Rejection at 30 Hz is 74 dB min. The Rejection at 60 Hz is 74 dB min. The Rejection at 90 Hz is 68 dB min. The Pacemaker Pulse Feedthrough in ECG Channels may be +/- 5 ADC Channels max for any time period exceeding 4 ms. The Internal Noise, Referred to Input 50 uv P-P max.

In regard to Data Conversion, the Sampling Rate is 250 samples/sec. min. The Resolution is 8 bits min. as to the Internal Clocks, the Microprocessor Clock is 4.096 Mhz +/- 0.1%. The Sample Clock is 250 Hz +/- 0.1%. The Wake-Up Timer is 1024 ms +/- 0.1%. As to the Protection From Defibrillation, the maximum voltage is 2500 DC Peak. The Maximum Energy is 100 Joules. The Maximum Cumulative Number of Discharges is 10. As to the Patient Protection, the Fault Current is 50 Microamperes Max. As to the Pacemaker Detector, the Amplitude Range of Pulse Detection is 1.0 mv to 1.0 v. The Slew Rate Change is no limit. The Pulse Width Range of Pulse Dection is 100 us to 2 ms. The Refractory Period is 25 ms typical, software programmable. The Upper Cutoff Frequency is 16 Khz. The Lower Cutoff Frequency is 100 Hz. As to the Lead Sense Detector, the Open Leads Threshold may be 12K Ohms. The Threshold Variation is +/- 25% Max. As to the Reliability, the MTBF is good. The MTTR is good.

A bootstrap output is required to improve common mode rejection when a patient lead impedance imbalance exists. This signal shall be equal in magnitude to the common mode voltage of the L1/L2 lead pair. It is used to drive the shield of the patient cable.

A separate isolation amplifier shall be used for the Pacemaker Snapshot Interface. It shall be AC-coupled with a low frequency 3db roll-off of 30 Hz. It shall have two PCU-controlled gain settings: X1 and X10. Its gain shall be set to make full use of the dynamic range of the opto-isolator (74 db). It shall be capable of being switched, under PCU program control, to monitor either channel A or B.

In order to reduce the dynamic range requirements of the opto-isolators, a circuit shall be included which can remove most of the patient electrode offset from the baseline. This circuit shall not increase the recovery time due to pacemaker overload and the recovery time due to long-term overload shall not exceed 1 minute.

The Analog Multiplexer controls whether the test signals or the outputs from the isolation amplifiers are applied to the AMU.

The Mux should have the capability of handling signals between +10 and -10 volts. The large signal range is necessary due to the fact that the isolation amplifier will amplify the electrode offset voltage (+/- 350 Mv. max.) by its DC gain (8), producing an ECG signal of +/- 2.5 volts max. riding on a DC offset of +/- 3 volts max.

The front panel Patient Connector of the PCU is cabled to this board which contains the optically coupled amplifiers to isolate and buffer the patient leads. These amplifiers connect to an analog mux which allows test signals (from the DAC on the PCU I/O Board) to be substituted for the live signal either as Lead 1, Lead 2, or both (common mode rejection ratio test). Provision is made to support the "Four Lead" patient connection with a second isolation amplifier. The Available Circuit Board Area is no more than 50 square inches.

The front-end electronics section is physically separated from the non-isolated electronics to minimize the stray capacitance from the two sections and provide isolation.

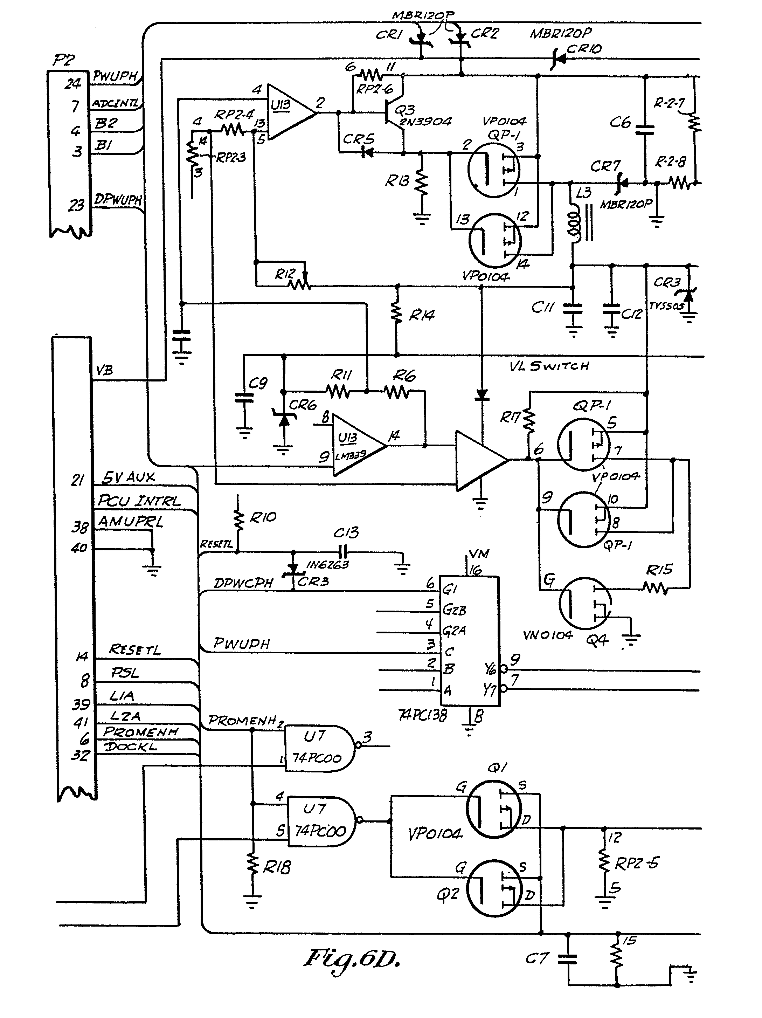

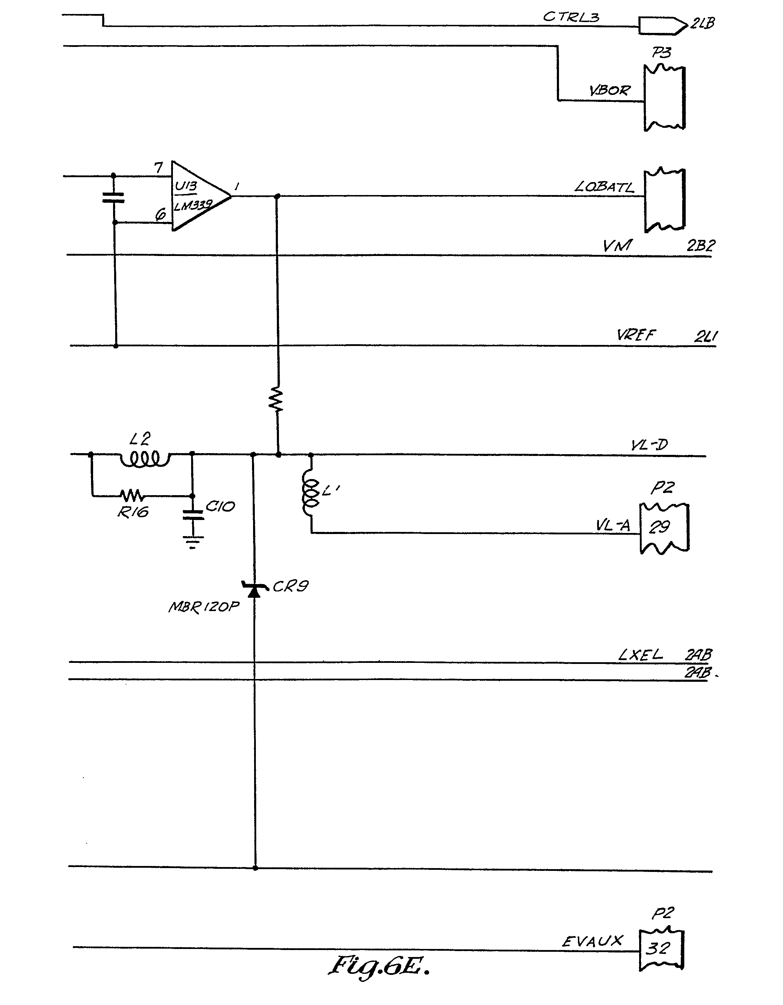

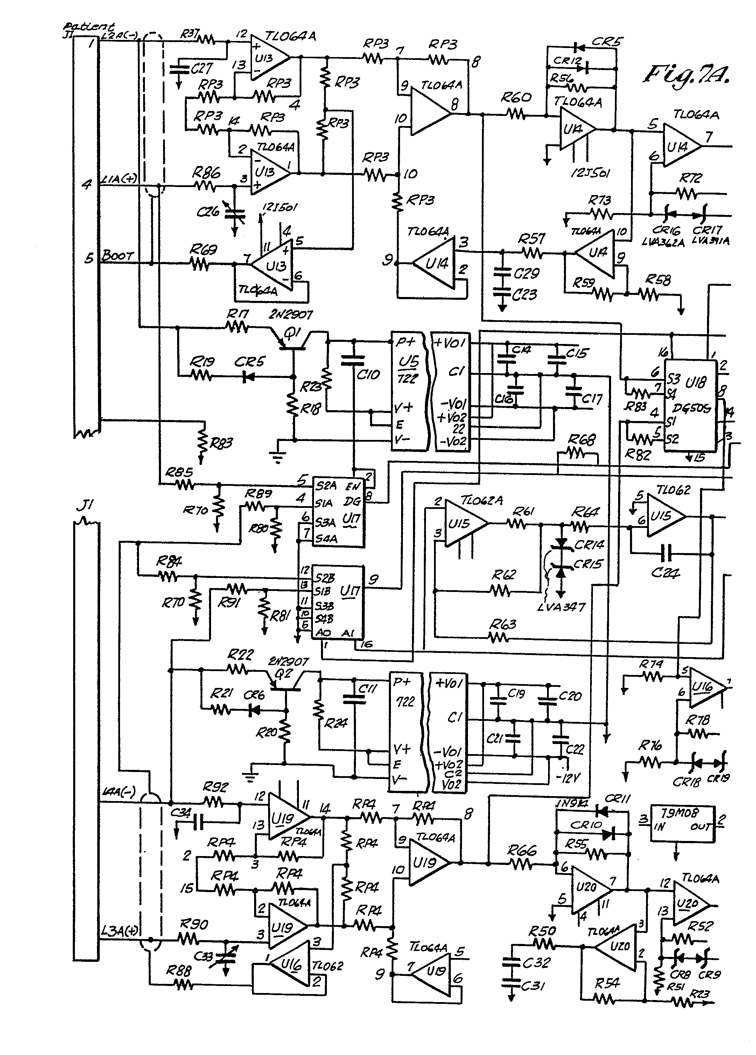

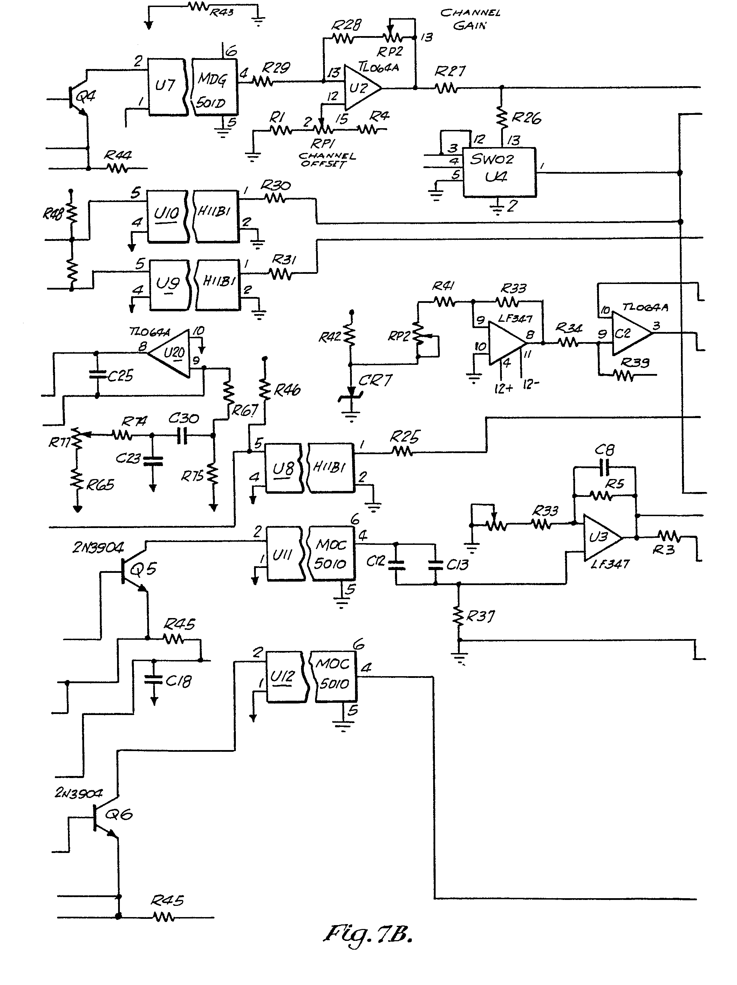

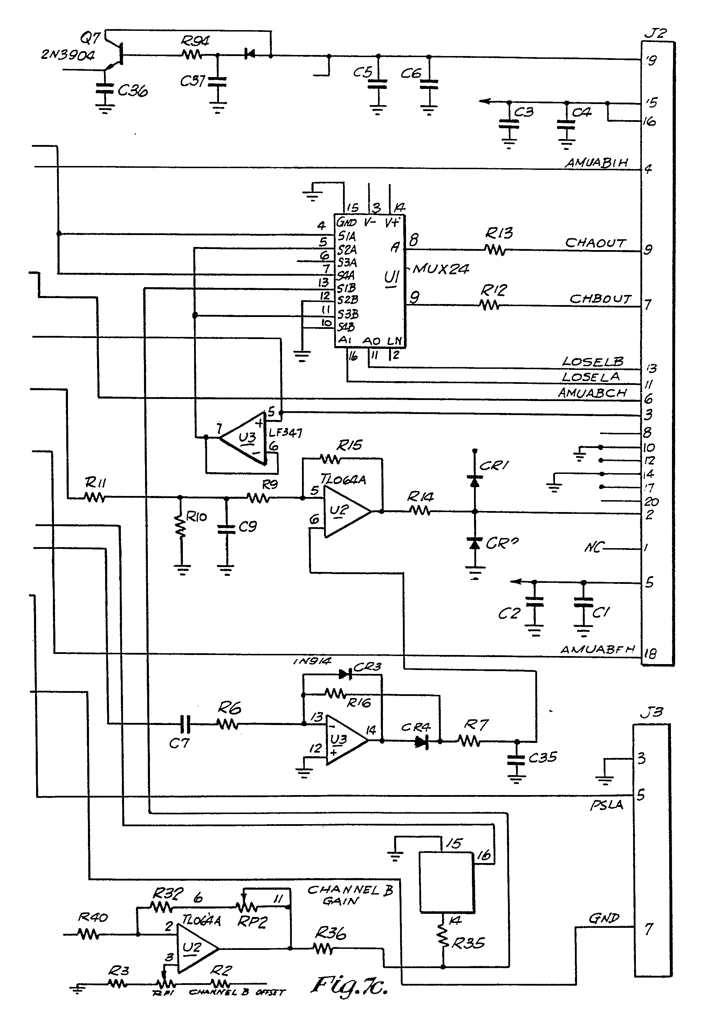

Figure 7 is a block diagram of the Dual Channel Analog Interface Board. Each pair of patient leads has its own independent optically coupled isolation amplifier with a common isolated power supply.

The isolation amplifiers shall be designed to pass both ECG and pacemaker signals with minimum distortion. It is especially important that they have good slew rate capability with a short settling time after a step input. If feedback type amplifiers are used, it is important that they be properly compensated to eliminate overshoot and ringing. This also applies to the modulator section of the isolation amplifiers.

The interstage coupling must be DC in order to keep the recovery time from a pacemaker overload to less than 15 microseconds.

Mux Functions are:

| Control Value | Function |
|---|---|
| 00 | Connect PCU Analog A to AMU Channel A |
|  | Connect PCU Analog B to AMU Channel B |
| 01 | Connect ground to AMU Channel B |
|  | Connect test signal to AMU Channel A. |
| 02 | Connect test signal to AMU Channels A and B. |
| 03 | Connect PCU Analog A to AMU Channel A; Connect ground to AMU Channel B. |

The capability to measure the impedance of each pair of patient leads shall be included. This function shall be a PCU menu item and shall be under software control. The impendance of each pair of leads shall be displayed on the CRT screen and if the value is above a pre-determined threshold, a flashing message on the screen shall so indicate; however this shall not prevent the initiation of the procedure.

The technique to measure the impedance of the leads shall use a 1 Khz, 10 ua p-p sinewave impressed across the lead pair to be measured. The differential voltage applied to the instrumentation amplifier will be directly proportional to the patient impedance. The output of the instrumentation amplifder shall be amplified by the isolated pacemaker channel. The output of the pacemaker channel shall be applied to an AC to DC converter. The output of the converter shall be a DC voltage which is directly proportional to lead impedance. This voltage shall be applied to one input of a comparator; the other input of the comparator shall be connected to the PCU DAC. The DAC shall be set up to output an incremented DC voltage. The output of the comparator shall be tested after each increment and if the comparator output is true, it indicates that the DAC output matches the impedance value.

The 1 Khz current generator shall be enabled only when the PCU is making an impedance measurement; it shall be disabled at all other times to ensure that it does not interfere with the pacemaker snapshot function. Note that this technique does not require that an AMU be docked in the PCU for impedance measurement purposes. The PCU software shall convert this value to the actual value of the impedance and display it on the CRT screen.

The capability to verify that the amplitude of the pacemaker pulse is sufficient to insure reliable detection by the AMU shall be included. The requirement can be implemented by including a software-controlled 2:1 analog attenuator between the amplifier outputs and the mux. During initialization, the attenuator should momentarily be activated. The operation of the AMU pacemaker detection logic should then be verified by the PCU. If the pacemaker detection logic continues to function, it means that the pacemaker pulse has at least twice the amplitude necessary for detection by the AMU.

Patient Connector (J1) Pin Functions are:

| Pin No. | Signal Name | Mnemonic |
|---------|-------------|----------|
| 1 | Patient Lead 2 (-) | L2A |
| 2 | Patient Lead 3 (+) | L3A |
| 3 | No Connection | - |
| 4 | Patient Lead 1 (+) | L1A |
| 5 | Bootstrap | Boot |
| 6 | No Connection | - |
| 7 | Analog Reference | ANREF |
| 8 | Patient Lead 4 (-) | L4A |
| 9 | Ground | GND |

Differential Connector (J2) Pin Functions are:

| Pin # | Signal Name | Mnemonic |
|-------|-------------|----------|
| 1 | No Connection | |
| 2 | Z Flag Out | $\overline{Z}$flag |
| 3 | D/A Converter Out | DACINA |
| 4 | Pacemaker Atten. Ctrl | AMUAB1H |
| 5 | +5 Volts | +5 |
| 6 | Pacemaker A/B Sel | AMUAB2H |
| 7 | Channel B Out | CHBOUTA |
| 8 | Ground | GND |
| 9 | Channel A Out | CHBOUTA |
| 10 | Ground | GND |
| 11 | Mux Control 0 | LDSELAH |
| 12 | Ground | GND |
| 13 | Mux Control 1 | LDSELBH |
| 14 | Ground | GND |
| 15 | -12 Volts | -12V |
| 16 | -12 Volts | -12V |
| 17 | Ground | GND |
| 18 | Z enable | AMUAB3H |
| 19 | +12 Volts | +12V |
| 20 | Ground | GND |

Pacemaker Snapshot (J3) Connector are:

| Pin # | Signal Name | Mnemonic |
|-------|-------------|----------|
| 1 | No connection | — |
| 2 | No connection | — |
| 3 | Ground | GND |
| 4 | No connection | — |
| 5 | Pacer Snap Out | PSLA |
| 6 | No connection | — |
| 7 | Ground | GND |
| 8 | No connection | — |
| 9 | No connection | — |
| 10 | No connection | — |

In regard to the Input Dynamic Range, the Range of Linear Operations of Input Signal (ECG) is +/- 5 MV min. The Slew Rate Change, ECG is 320 MV/sec max. The Range of Linear Operations of Input Signal (Pacemaker) +/- 1.0 MV to +/- 1.0 V. The Slew Rate Change, Pacemaker Pulse has No Limit. The DC Offset Voltage Range is +/- 350 MV min. The Allowed Variation of Amplitude with DC Offset is +/- 5% max.

In regard to the Accuracy of Input Signal Reproduction, the Overall Error for Signals is +/- .05 to +/- 5 MV: Target +/-2% or +/-25 UV max. The Upper Cutoff Frequency (3 db) is 30 KHz min. The Allowable Overshoot to Step Response is 5% max. The Settling time after step input is To 0.1% of final value within 1 usec. The Decay Time Constant After Step Input is 3 sec. min. The Lower Cutoff Frequency (3 dB) is 0.05 Hz max. In regard to Gain Control and Stability, the Error is +/- 3% max. The Gain Drift Initial is 0.2%/minute max. The Gain Drift, Total in One Hour is 0.5%. In regard to the Baseline, the Baseline Drift Rate, Referred to Input is 20 uv/sec. max. The Total Baseline Drift, Referred to Input is 50 uv max. The Baseline Recovery Time After Pacemaker Overload is 15 usec. max. The Baseline Recovery Time After Long-Term Overload is 45 seconds max. In regard to Input Impedance, the Impedance at 10 Hz is 20 megohms min. The Impedance at 100 Hz is 17 megohms min. The Input Current is 25 nanoamperes max. As to Common Mode Rejection, the Rejection at 30 Hz is 70 dB min and the Rejection at 60 Hz is 70 dB min. The Rejection at 120 Hz is 70 dB min. In regard to the Internal Noise, Referred to Input it is 50 uv P-P max. In regard to Protection From Defibrillation, the Maximum Voltage is 5000 Volts DC. The Maximum Energy is 400 Joules. The Maximum Cumulative Number of Discharges has No limit. The Patient is protected by providing DC to 1 KHz Risk Current Limit and 5 Microamperes Rms Max at 120 Volts Rms. The Fault Current is 50 Microamperes Max. The Patient Impedance Range of Measurement is 500 to 200,000 ohms. The Measurement Error is +/- 25% maximum,

or +/- 1k ohms whichever is greater.  The Resolution is 100 ohms up to 20k ohms, 1 k ohms for readings greater than 20k.

This board has the capability of interfacing both the single and dual channel AMU's to the PCU.  When a single channel AMU is docked with the PCU, the PCU software should set the channel B mux to output zero volts to AMU Lead 2.  (For dual channel units, Lead 2 becomes channel B).

TABLE

CPU TIMING PARAMETERS, TYPICAL VALUES

| MNEMONIC | PARAMETER | TYPICAL VALUE (NS) | |
|----------|-----------|--------------------|--------------------|
| | | (FETCH-R/W) | (I/O) |
| TDALA | ALE to A(8-15) valid | 20 | --- |
| TDALAD | ALE to AD(0-7) valid | 40 | --- |
| TDALRD | Read high to ALE high | -20 | --- |
| TDCAD | T2 high to data | 160 | 160 |
| TDCALH | T clock to ALE high | 50 | --- |
| TDCALL | T clock low to ALE low | 30 | --- |
| TDCRDH | T clock low to Read high | 50 | 60 |
| TDCRDL | T clock high to Read low | 70 | 70 |
| TDCWRH | T clock low to Write high | 50 | 50 |
| TDCWRL | T clock low to Write low | 40 | 40 |
| THRD | Read high to Data hold | 260 | 290 |
| THRDAD | Read high to AD(0-7) active | 40 | --- |
| THRDADH | Read high to AD(0-7) active | 300 | --- |
| THWD | Write high to Data hold | 310 | 300 |
| TSWD | Write set up time | 180 | 190 |
| TWALE | ALE pulse width | 230 | 220 |
| TWCLK | T clock width | 488 | 488 |
| TWRDIO | Read width, I/O cycle | --- | 710 |
| TWRDM | Read width, Memory Read cycle | 710 | --- |
| TWRDO | Read width, OP-Code Fetch cycle | 480 | --- |
| TWWR | Write width | 500 | 980 |

It should be noted that the following signals are applicable in Figures 5A, 5B, 5C, 5D and 5E and Figures 6A, 6B, 6C, 6D and 6E.

P1

| Pin | Signal |
|-----|--------|
| 1 | A8 |
| 2 | VL |
| 3 | A9 |
| 4 | VM |
| 5 | A110 |
| 6 | PROMENH |
| 7 | A11 |
| 8 | PSL |
| 9 | A12 |
| 10 | GND |
| 11 | A13 |
| 12 | IO/ML |
| 13 | A14 |
| 14 | RESETL |
| 15 | A15 |
| 16 | RDL |
| 17 | WRL |
| 18 | XCLKH |
| 19 | ALEL |
| 20 | SOH |
| 21 | 5VAUX |
| 22 | B2 |
| 23 | ADO |
| 24 | B1 |
| 25 | AD1 |
| 26 | S1H |
| 27 | AD2 |
| 28 | VB |
| 29 | AD3 |
| 30 | PCUINTRL |
| 31 | AD4 |
| 32 | DOCKL |
| 33 | AD5 |
| 34 | VREF |
| 35 | AD6 |
| 36 | TCLKH |
| 37 | AD7 |
| 38 | AMUPRL |
| 39 | LIA |
| 40 | GND |
| 41 | L2A |

P2

| Pin | Signal |
|-----|--------|
| 1 | L2A |
| 2 | L1A |
| 3 | B1 |
| 4 | B2 |
| 5 | ADRDL |
| 6 | ADCLKH |
| 7 | ADCINTOL |
| 8 | DOH |
| 9 | D1H |
| 10 | D2H |
| 11 | D3H |
| 12 | D4H |
| 13 | D5H |
| 14 | D6H |
| 15 | D7H |
| 16 | GNCTRH |
| 17 | CLCTRH |
| 18 | ZCTRL |
| 19 | ISECL |
| 20 | RSTCL |
| 21 | LOBATL |
| 22 | ·ALCTH |
| 23 | DPWUPH |
| 24 | AVREF |
| 25 | IOWRH |
| 26 | PWUPH |
| 27 | RPUL |
| 28 | RESETL |
| 29 | VL-A |
| 30 | AD7H |
| 31 | STSELL |
| 32 | 5VAUX |
| 33 | DOCKL |
| 34 | LCBH |

P3

| Pin | Signal |
|-----|--------|
| 1 | A11 |
| 2 | MA2 |
| 3 | A12 |
| 4 | MA1 |

## P3 Cont'd

| Pin | Signal |
|-----|--------|
| 5 | MWRL |
| 6 | MAO |
| 7 | MEMENL |
| 8 | A14L |
| 9 | MA6 |
| 10 | A14 |
| 11 | MA4 |
| 12 | MA10 |
| 13 | MA3 |
| 14 | A13 |
| 15 | VM |
| 16 | MA5 |
| 17 | MA7 |
| 18 | MA8 |
| 19 | MA9 |
| 20 | VBOR |
| 21 | RDL |
| 22 | ADO |
| 23 | AD1 |
| 24 | AD2 |
| 25 | AD7 |
| 26 | AD6 |
| 27 | GND |
| 28 | VL-D |
| 29 | AD3 |
| 30 | AD4 |
| 31 | AD5 |
| 32 | LXEL |
| 33 | HXEL |
| 34 | GND |

## P7 PATIENT

| Pin | Signal |
|-----|--------|
| 1 | VBOR |
| 2 | PAT CONN |
| 3 | INB- |
| 4 | INB+ |
| 5 | ANREF |
| 6 | INA- |
| 7 | INBTA |
| 8 | INA+ |
| 9 | GND |

P8 WATCH

Pin   Signal

1     VT
2     GND
3
4     ETSET-P
5     INCRHRS-P
6     MOONINCRMIN-P
7     R-TIME-P

P9 EVENT

Pin   Signal

1     EVENT SW
2     GND
3     ALARM
4     ALARM+

P11 BATTERY

Pin   Signal

1     B1
2     GND
3     B2

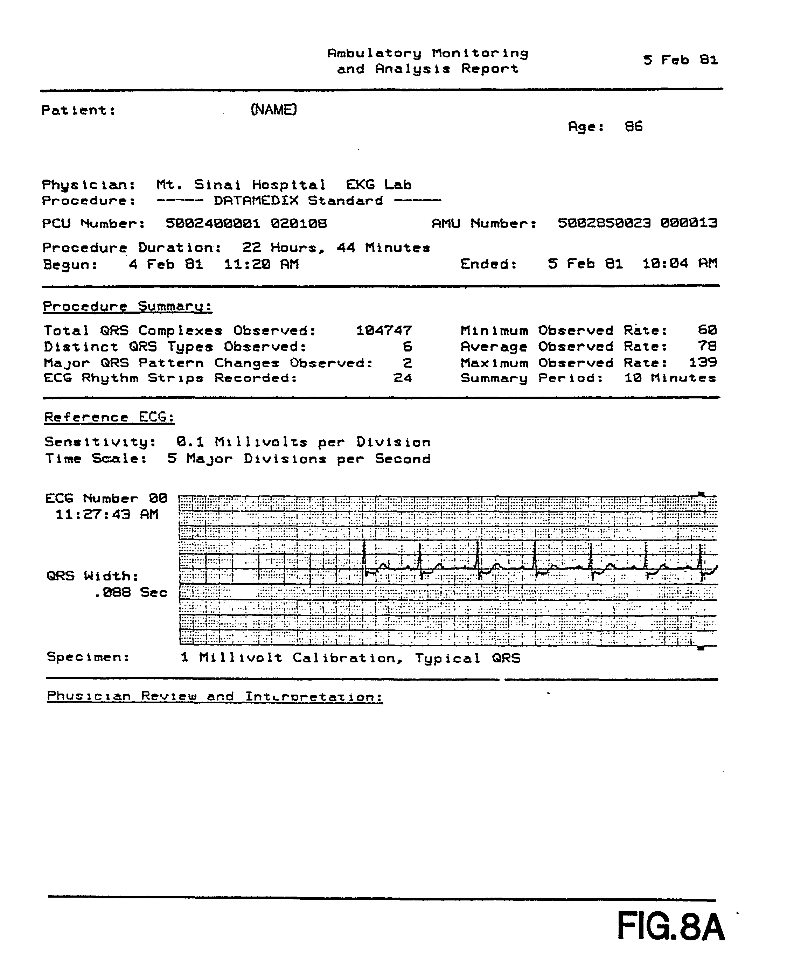

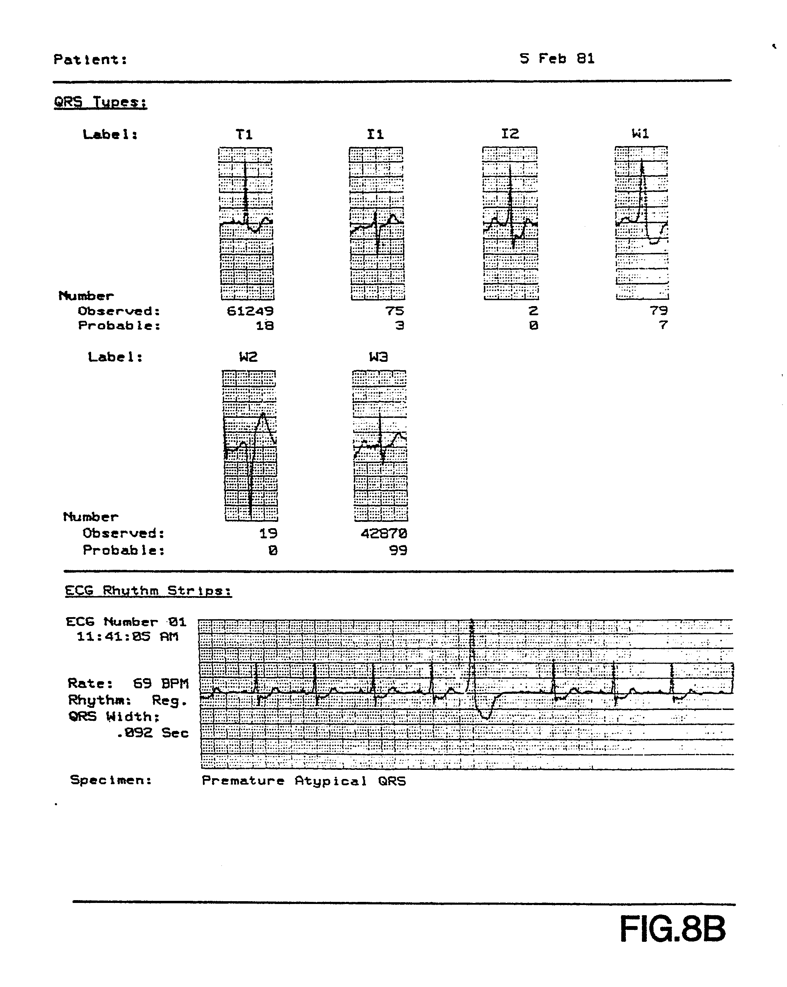

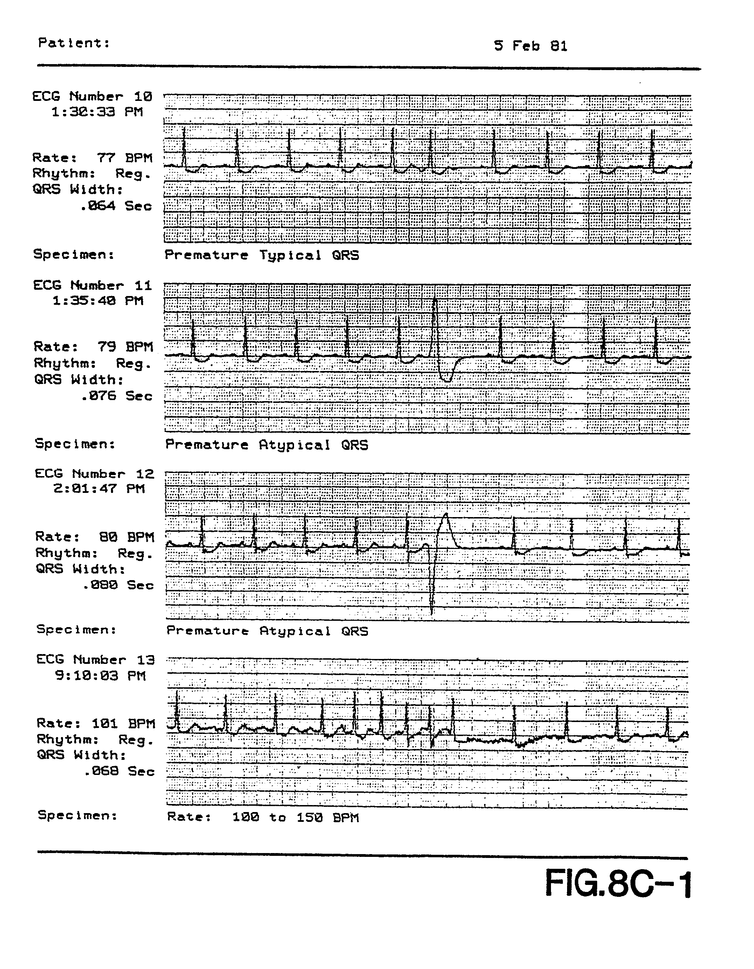

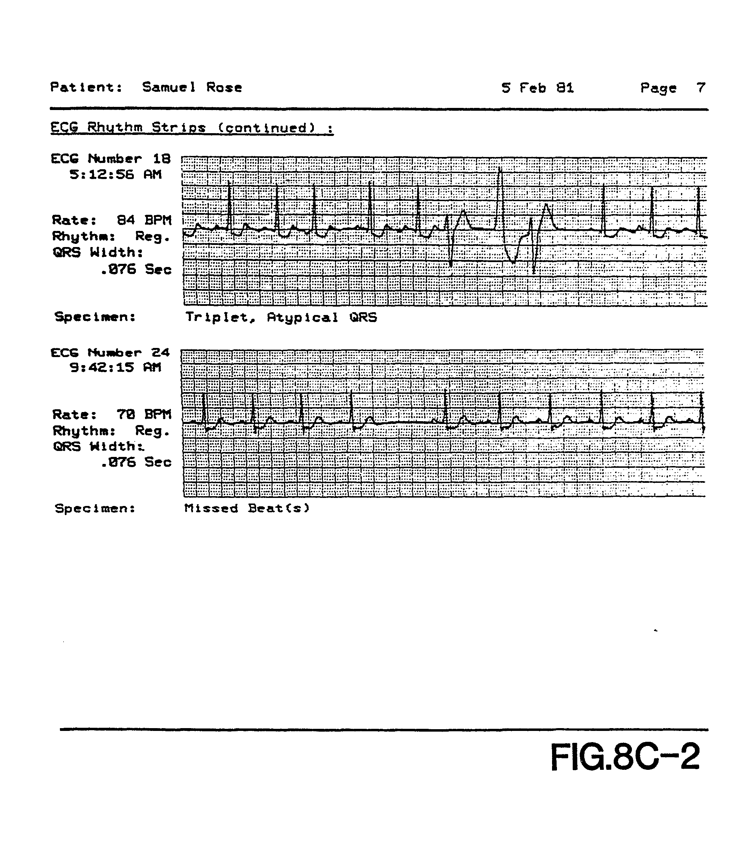

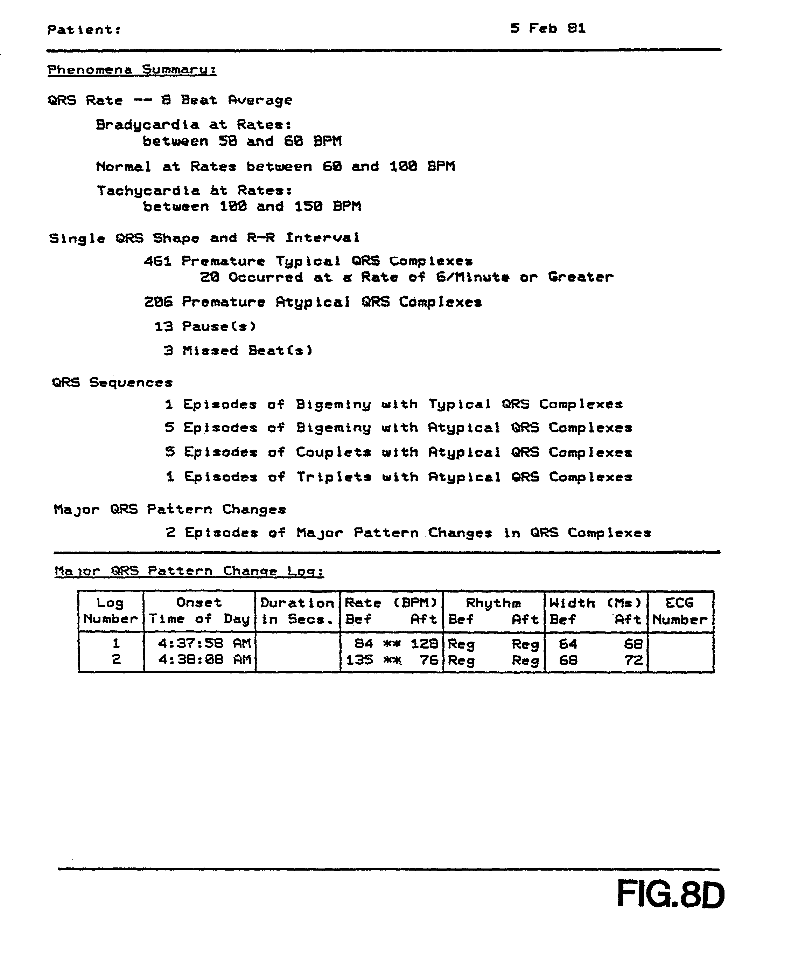

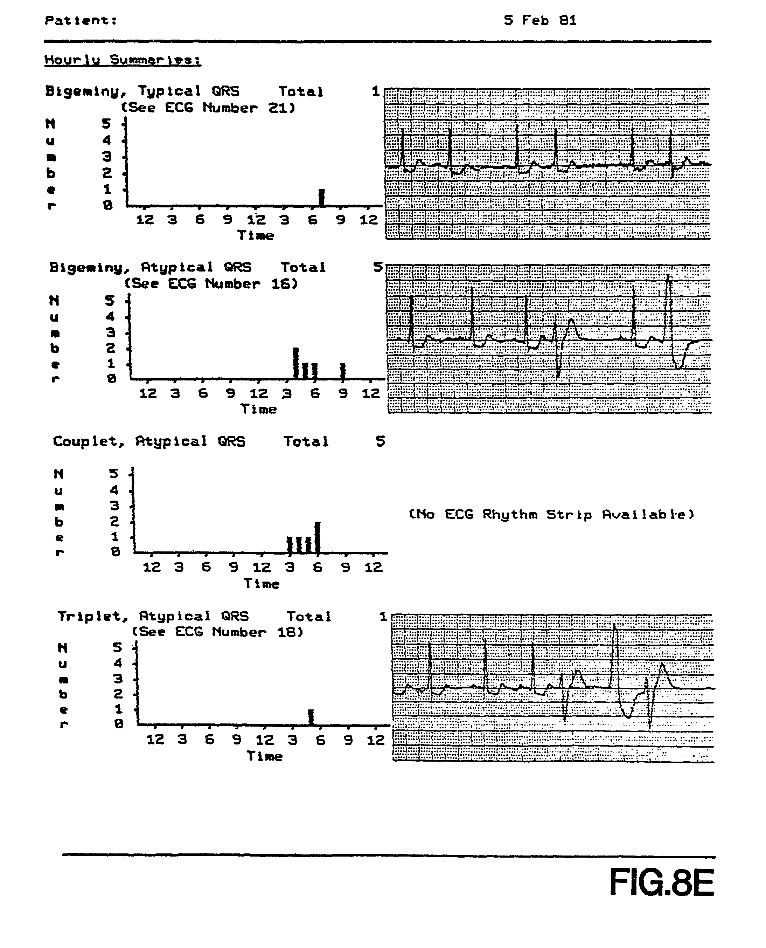

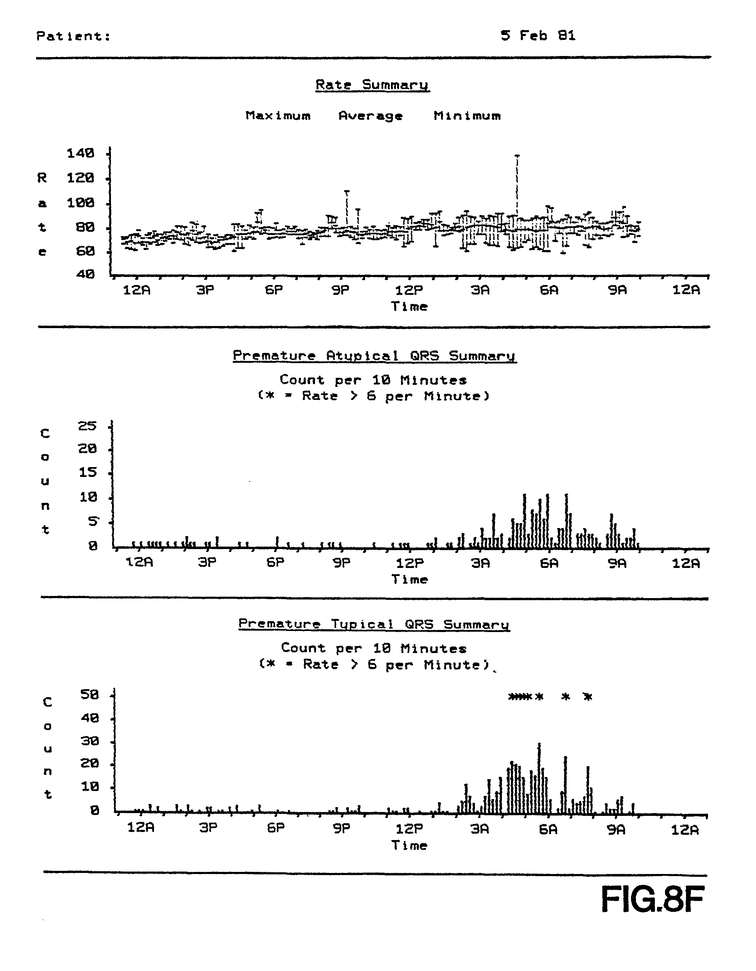

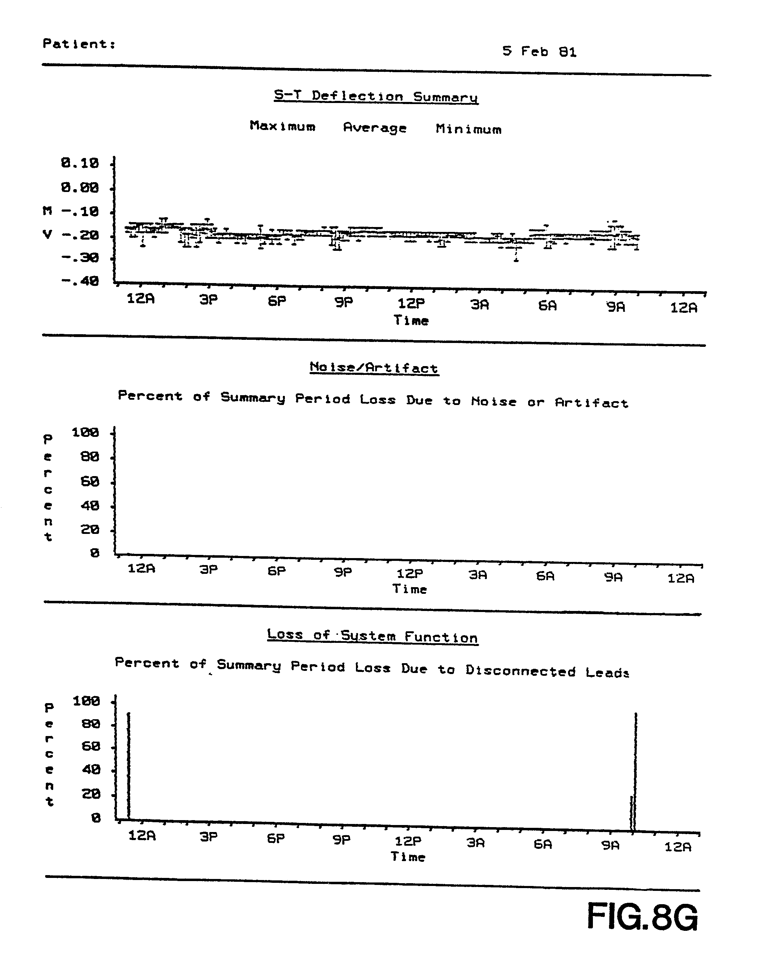

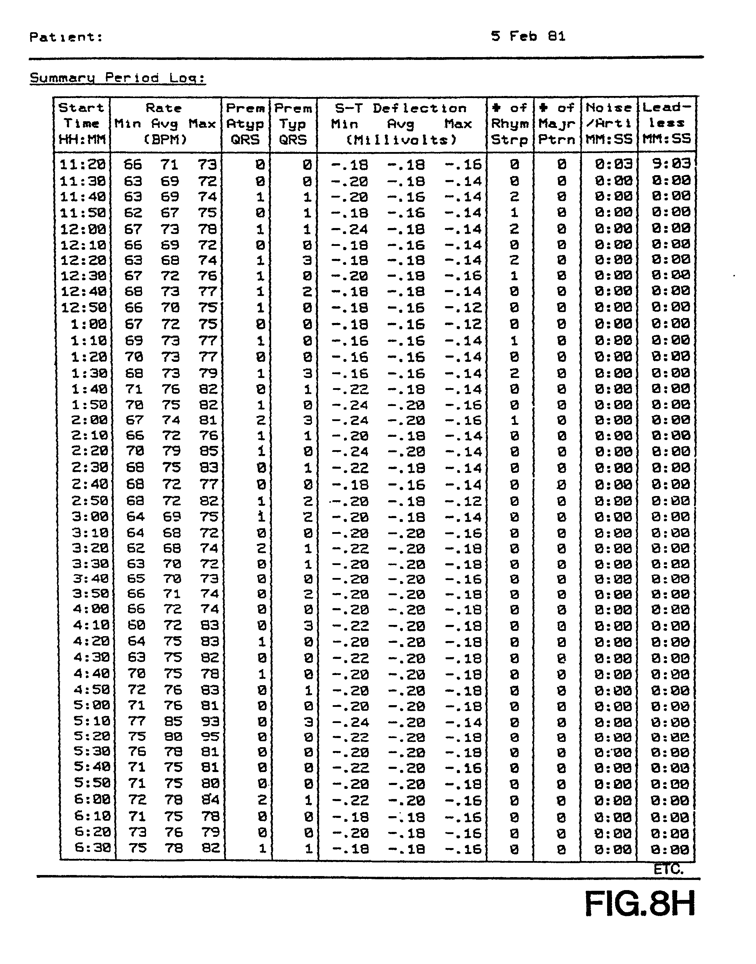

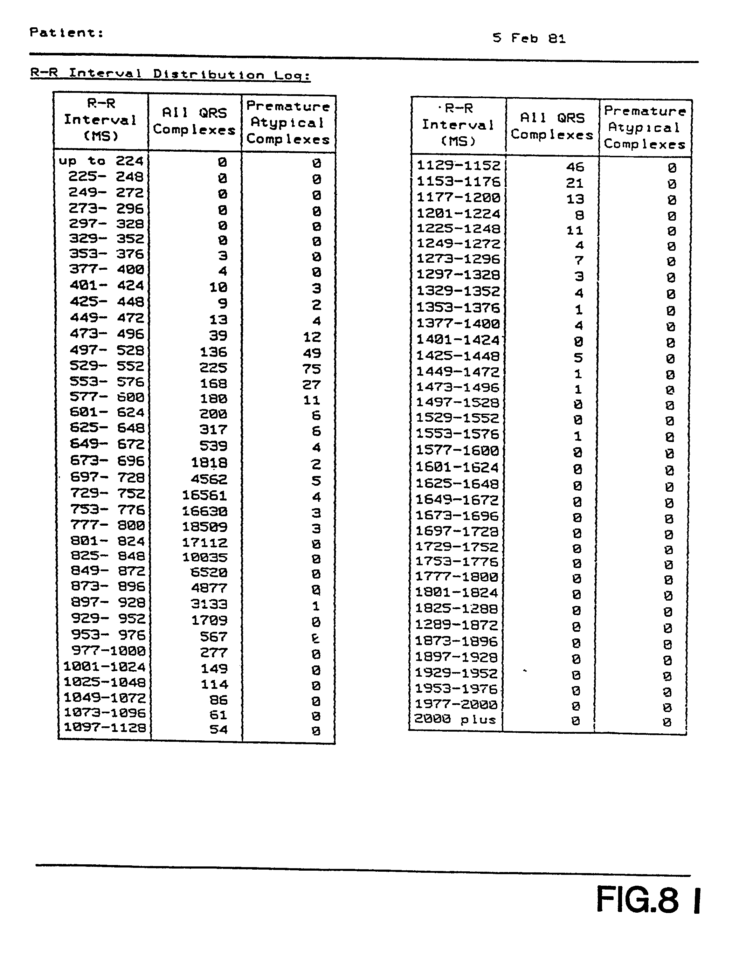

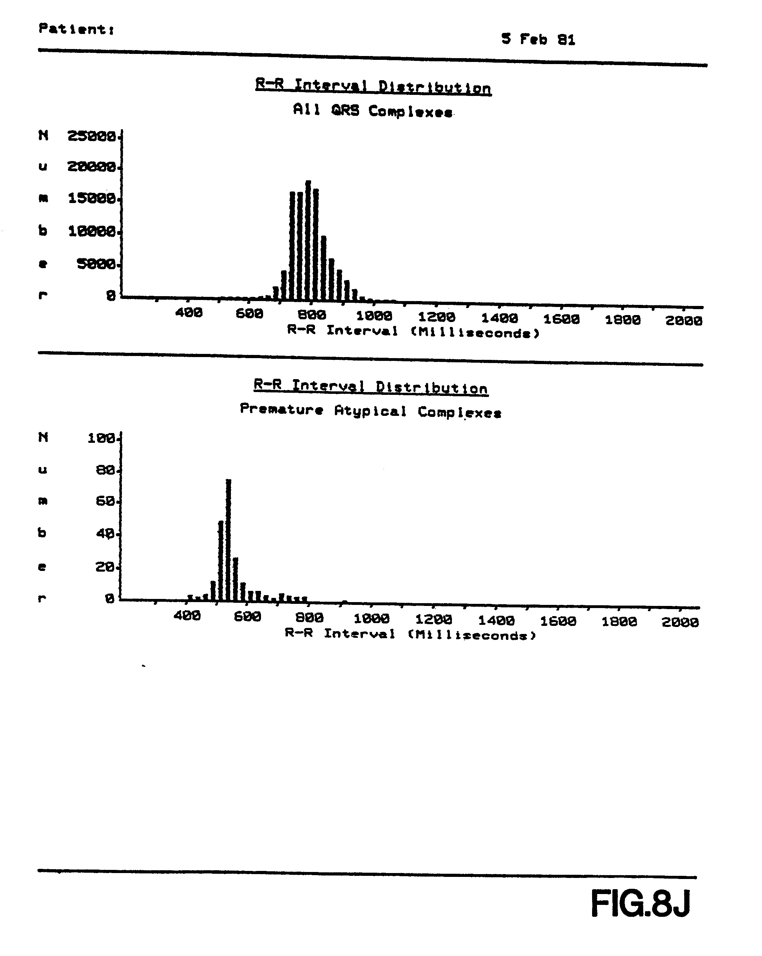

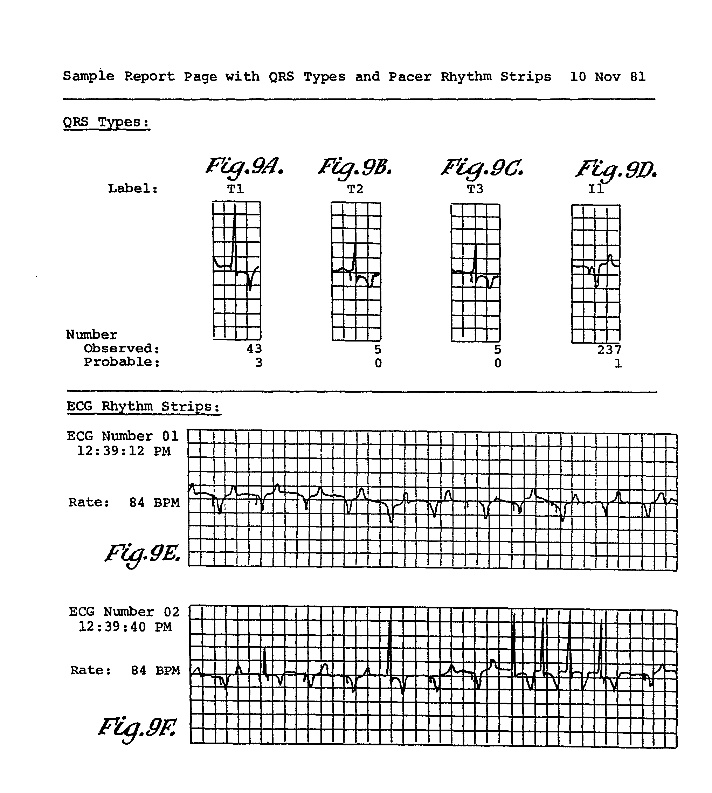

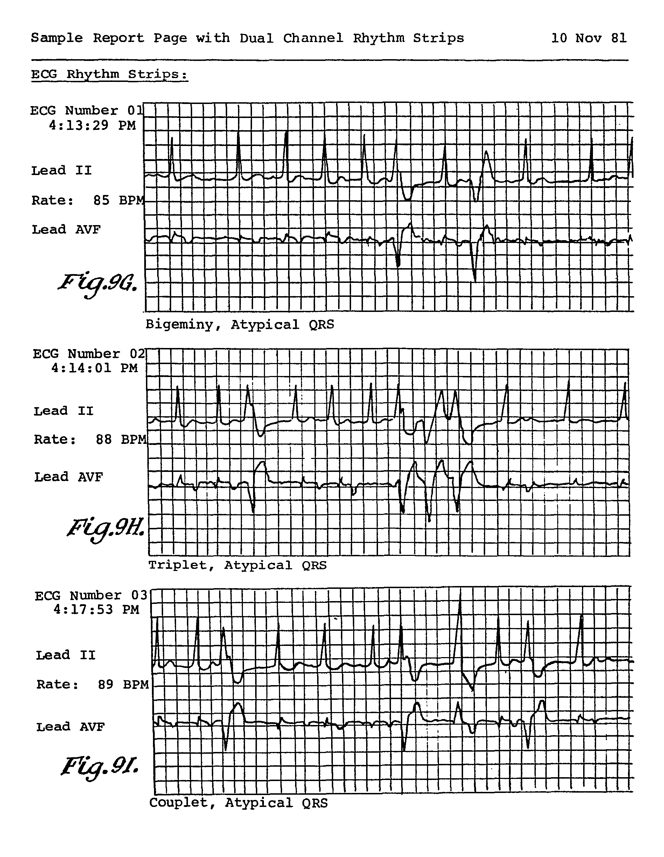

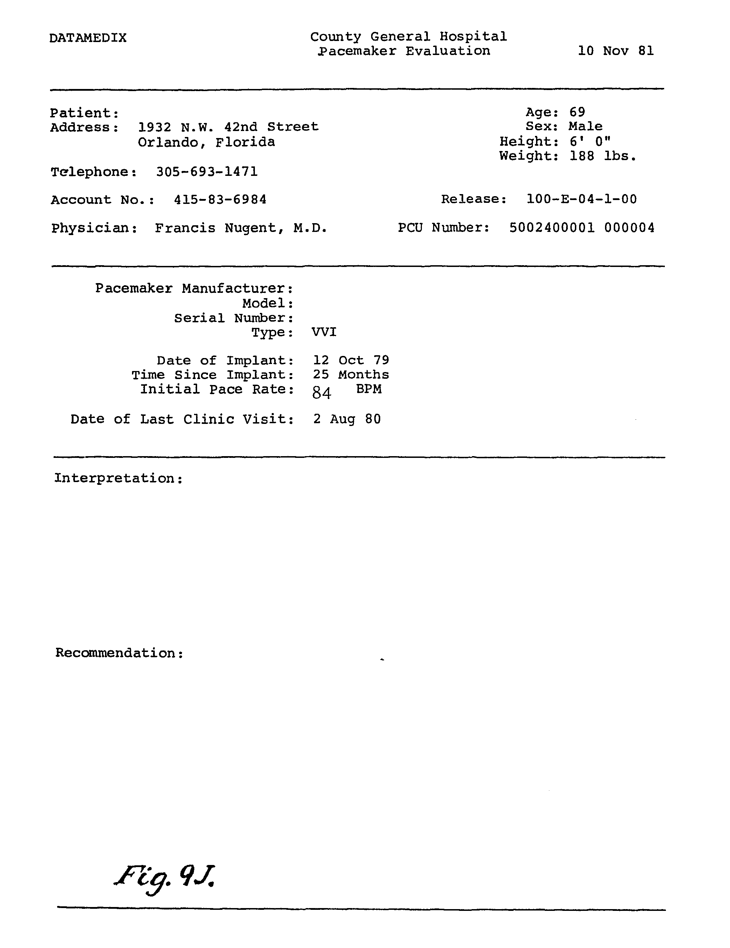

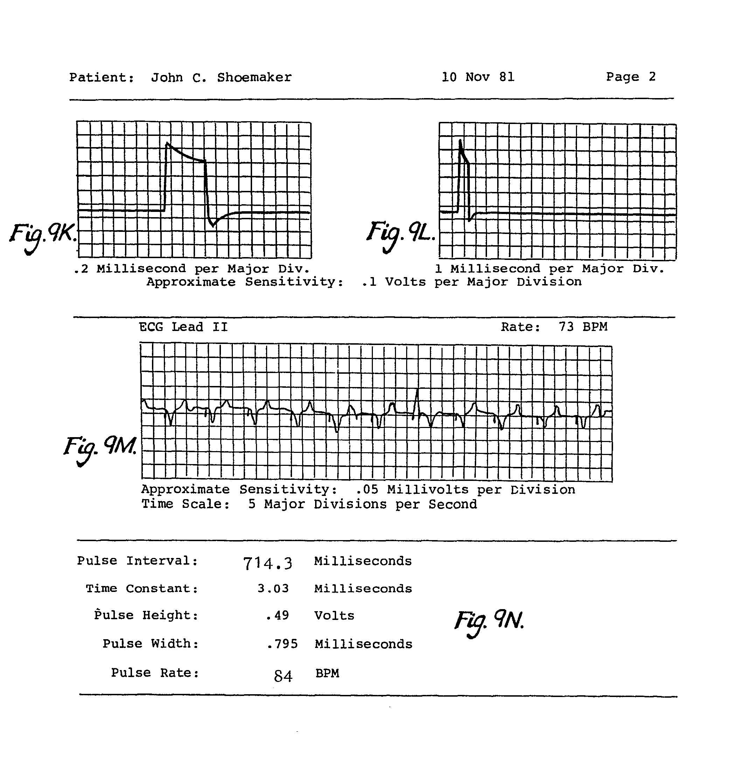

The use of the dual channel AMU 10, PCU 30 and the printer 30a provides data when operated as a single channel as shown in Figure 8. When the AMU 10 and PCU 30 and printer 30A is operated in the dual channel mode the printer provides output data as shown in Figures 9.

The instant invention has been shown and described herein in what is considered to be the most practical and preferred embodiment. It is recognized, however, that departures may be made therefrom within the scope of the invention and that obvious modifications will occur to a person skilled in the art.

The Two Channel Ambulatory Monitoring system monitors, analyzes and produces reports on patient ECG signals taken from a two lead electrode configuration. The monitoring and analysis is performed by a small, lightweight ambulatory computer system designated as the Ambulatory Monitoring Unit or AMU. The operation of the AMU is initiated by the Procedure Control Unit or PCU. The report from the Ambulatory Monitoring Procedure is formatted by the PCU and generated into a report using the Graphic Report Printer or GRP.

Thus, the Two Channel Ambulatory Monitoring system consits of three basic components, the AMU, the PCU and the GRP. The ECG analysis performed by the AMU is designed to detect changes in rate, irregularity and shape of QRS complexes. Such changes are categorized into a number of QRS activity classifications: Rate ranges, single QRS and RR interval classifications, QRS sequence classifications, and major pattern changes.

The Two Channel system is sensitive (under program control) to pacemaker stimulation pulses.

The Ambulatory Monitoring Unit provides for pacemaker and pacemaker/cardiac activity classifications.

The user interaction with the system is accomplished through the Procedure Control Unit using a series of video screens. These screens contain prompting information and provide for user selection of system services. The user may define customized procedures for use with the Ambulatory Monitoring Unit. These customized procedures can subsequently be invoked when the Ambulatory Monitoring Unit is initialized. The user may specify the content of reports which are generated and may also review and edit report data on the video screen either before or subsequent to printed report generation.

The Ambulatory Monitoring Unit makes use of two independent channels of ECG data.  Through the use of impedance measuring equipment in the AMU, it is possible for the unit to determine that the patient leads are properly connected.  In the event of poor connections or loose leads, the AMU can suspend analysis of ECG signals from a given channel.  By verifying ECG activity in both channels the Ambulatory Monitoring Unit can achieve higher reliability of its QRS classifications than is possible through the use of a single channel.

The Ambulatory Monitoring Unit contains a liquid crystal time display for patient convenience in maintaining an activity diary.  The Ambulatory Monitoring Unit also contains a Patient Marker Button for indications of patient symptoms.  The Ambulatory Monitoring Unit logs depressions for the Patient Marker Button for patients in the physician's report.  Additionally, the Ambulatory Monitoring Unit contains audio tone generator which is used for indicating to the patient that the AMU has terminated the monitoring procedure or that it is having difficulty in analyzing the signals received from the patient.

The Graphic Report Printer is contained in an enclosure separate from the Procedure Control Unit. It produces printed pages which are cut to 8-1/2 x 11". The printing quality is such that it can accurately portray ECG signals.

The Procedure Control Unit provides a video screen for prompting and data presentation to the user. It provides a keyboard for data entry and user control and response. It also contains a docking port for the Ambulatory Monitoring Unit as well as a receptacle for the ECG cable connector.

The Ambulatory Monitoring Unit referred to hereafter as the AMU is a battery operated, 24 hour ambulatory heart monitor. It monitors the patients ECG activity via a dual channel (Lead V5 and modified Lead 2) connection employing five patient leads.

The AMU has a digital microprocessor and a 64K byte random access memory incorporated. The microprocessing unit (MPU) continuously analyzes the patients heart activity during the monitoring period. When abnormal activity is detected, the MPU stores data in memory relating to the detected abnormality.

At the end of the monitoring period, the AMU is "docked" in the Procedure Control Unit referred to as PCU and a visual and/or printed report is generated.

. The patient may alert the MPU of the occurrence of an unusual event by depressing the "Patient Symptom" button on the unit.

The AMU requires two 9 volt "transistor" alkaline batteries for a full 24 hour procedure. Average battery life is expected to be 36 hours or more.

Reference is directed to European Patent Application No. 81 901091.9 (International Publication No. WO 81/02832) entitled "MEDICAL MONITOR".

The AMU is 6.72 x 4.18 x 1.28 inches overall and weighs 26 ounces. All electronics and power supply are contained in this envelope.

The case is a two piece injection molded sealed unit. The case material is Noryl. The front panel (1.28 x 4.18 inches) contains the "Patient Symptom" button, the clock display and the patient connector. The rear panel houses the PCU interface connector which mates with the PCU docking station. A slide/snap door on one side of the AMU provides access to the two 9 volt batteries.

The controlling component is the NSC 800 MPU which directly accesses all of Memory and PROM. The PROM is accessible only when docked and powered by the PCU. The MPU communicates with the PCU via programmed I/O using the Data and Address busses and the MPU control lines. The Control Logic block generates the indicated control signals on command from the MPU. It also communicates device status (such as the "Patient Symptom" button) via the Data/Address bus to the MPU. The MPU operates on a 4 to 8 MHz clock which results in a memory cycle time of 2.0 to 1.0 microseconds. The MPU includes as set forth in more detail below a Memory, Multiply/Divide Unit, ECG Signal Processing means, Pacemaker Detector, Patient Lead Imbalance Test Logic, Patient Interface, and Power means.

The instruction set and op codes of the NSC800 are identical to those of the Z80 microprocessor and the code is executed using the same number of T states as the Z80. Software written for the Z80 will run on the NSC800 without change unless I/O location SBB is used. Another location should be assigned since location SBB is an on-chip write only register used for the interrupt mask. The NSC800 expanded interrupt cability is transparent to the user unless specifically invoked by the user software.

The status flags on the NSC800 are identical to those on the Z80. There is no difference between the positions of the individual bits in the flag register nor in the manner in which the flags are set or reset due to an arithmetic or logical operation. Testing of the flags is also the same.

The AMU communicates with the PCU (Procedure Control Unit) via a 41 pin connector. The AMU docking and undocking procedures are controlled to provide a well controlled system.

The AMU electronic package consists of two 32K x 8 memory boards, an MPU board and a data board interconnected by a common 8 bit bidirectional address/ data bus, an address bus and several control signals. Figure 1 is the overall block diagram of the AMU and Figures 2, 3 and 4 are the block diagrams for the MPU, Memory and Data boards respectively.

The front panel Patient Connector of the PCU is cabled to this board which contains the optically coupled amplifiers to isolate and buffer the patient leads. These amplifiers connect to an analog mux which allows test signals (from the DAC on the PCU I/O Board) to be substituted for the live signal either as Lead 1, Lead 2, or both (common mode rejection ratio test). Provision is made to support the "Four Lead" patient connection with a second isolation amplifier.

The system includes means for detecting and controlling heart pacer spikes for use of the AMU on patients having implanted heart pacers.

It is an object of this invention to provide a dual channel AMU to provide more complete accurate output data.

It is another object of this invention to provide an ambulatory ECG monitoring unit for real time QRS classification by QRS classification criteria means.

It is a further object of this invention to provide an ambulatory ECG monitoring unit for real time ST line analysis.

A further object of this invention to provide an AMU operable on heart pacer patients and for detecting and controlling the detected heart pacer spikes for providing better analysis and providing heart pacer data for further analysis.

## CLAIMS

1. A dual channel patient monitoring system for monitoring ECG signals of an ambulatory patient comprising: an ambulatory container connectable to a patient, a monitoring means connected to said container, said monitoring means for monitoring of ambulatory patient output signals, real-time analysis of said signals and storage of analyzed signals for indepth reporting of said monitored and analyzed ambulatory patient output signals, said monitoring means includes power means for supplying power to said monitoring means, acquisition and conditioning means for acquiring and conditioning the ambulatory patient output signals to provide conditioned output signals, said acquisition and conditioning means connected to said power means, said acquisition and conditioning means including dual channel connection means, connected to the ambulatory patient for providing improved analysis and output data, detection and management means for detecting and managing the conditioned output signals for identification, said detection and managment means connected to said acquisition and conditioning means, identification means for identifying particular conditioned output signals, said identification means means connected to said detection and management means, pattern classification means for classifying particular patterns and change in the particular conditioned output signals in real-time, said pattern classification means connected to said identification means, storage means for storing the classified patterns and changes, said storage means connected to said pattern classification means; output means for providng output signals from said storage means, said output means connected to said storage means.

2.  A dual channel patient monitoring system as set
    forth in Claim 1, wherein:
        said detecting and managing means includes a
    heart pacer spike detection and control means for
    detecting and controlling the detected heart pacer spikes
    for providing a monitoring system for an ambulatory
    patient.

3.  A patient monitoring system for monitoring ECG
    signals of an ambulatory patient comprising: an
    ambulatory container connectable to a patient
    a monitoring means connected to said container, said
    monitoring means for monitoring of ambulatory patient
    output signals, real-time analysis of said signals
    and storage of analyzed signals for indepth reporting
    of said monitored and analyzed ambulatory patient
    output signals, said monitoring means includes
    power means for supplying power to said
    monitoring means, acquisition and conditioning
    means for acquiring and conditioning the
    ambulatory patient output signals to provide
    conditioned output signals, said acquisition
    and conditioning means connected to said power means,
    connectable to the ambulatory patient,
    detection and management means for detecting
    and managing the conditioned output signals
    for identification, said detection and
    management means connected to said acquisition
    and conditioning means, identification means
    for identifying particular conditioned output
    signals, said identification means connected to said
    detection and management means, pattern classification
    means for classifying particular patterns and
    change in the particular conditioned output signals
    in real-time, said pattern classification means connected to said pattern classification means; said pattern classification means including QRS classification criteria means for providing real time QRS classificati output, said QRS classification criteria means connected to said identification means, and said storage menas, and output means for providing output signals from said storage means, said output means connected to said storage means.

4. A patient monitoring system as set forth in Claim 2, wherein:
said pattern classification includes ST level analysis means for ST level output, said ST level analysis means connected to said identification means and said storage means.

5. What is shown and described herein.

Fig. 1.

L.L.

L.A.

R.L.
Ref.

R.F.

*Fig. 2.*

204    206    30

84

206

10

*Fig. 3.*

Patient
Lead
Connections

10

AMU

A.C. Power

Procedure
Report
Printer

30a

82

Patient
Isolated
AMU Analog
Interface
Fig.7.

Docking
Receptacle — 84

AMU
Digital
Interface — 80

System
Diagnostics
Display

Key Board

86

A.C.
Power

Power
Supply

+5V.
+12V
−12V

Input
Output
Logic

Program
Memory

96

System
Diagnostics
Memory

Micro
Processor

88

CRT
Display

Display
Memories

Graphics

Grid

Alpha
Numerics

System Bus       90      92

Fig.4.

Fig. 5A.

Fig. 5B.

*Fig.5C.*

Fig. 5D.

Fig.5E.

0080821

8/31

Fig.6A.

Fig.6B.

0080821

*Fig.6C.*

0080821

Fig.6D.

0080821

CTRL3 ▷ 2LB

P3

VBOR

U13
LM339

7

6

1

LOBATL

VM          2B2

VREF        2L1

VL-D

L2

R16    C10

L'

P2

VL-A    29

CR9
MBR120P

LXEL    2AB
         2AB

P2

EVAUX    32

*Fig.6E.*

Fig. 7A.

0080821

Fig. 7B.

Fig.7c.

Ambulatory Monitoring          5 Feb 81
and Analysis Report

Patient:                (NAME)
                                        Age:  86

Physician:  Mt. Sinai Hospital  EKG Lab
Procedure:  ----- DATAMEDIX Standard -----

PCU Number:  5002400001 020108      AMU Number:  5002850023 000013

Procedure Duration:  22 Hours, 44 Minutes
Begun:   4 Feb 81  11:20 AM          Ended:   5 Feb 81  10:04 AM

Procedure Summary:

Total QRS Complexes Observed:     104747    Minimum Observed Rate:    60
Distinct QRS Types Observed:           6    Average Observed Rate:    78
Major QRS Pattern Changes Observed:    2    Maximum Observed Rate:   139
ECG Rhythm Strips Recorded:           24    Summary Period:  10 Minutes

Reference ECG:

Sensitivity:  0.1 Millivolts per Division
Time Scale:  5 Major Divisions per Second

ECG Number 00
  11:27:43 AM

QRS Width:
    .088 Sec

Specimen:      1 Millivolt Calibration, Typical QRS

Physician Review and Interpretation:

FIG.8A

Patient:                                 5 Feb 81

QRS Types:

| Label: | T1 | I1 | I2 | W1 |
|---|---|---|---|---|

| Number | | | | |
|---|---|---|---|---|
| Observed: | 61249 | 75 | 2 | 79 |
| Probable: | 18 | 3 | 0 | 7 |

| Label: | W2 | W3 |
|---|---|---|

| Number | | |
|---|---|---|
| Observed: | 19 | 42870 |
| Probable: | 0 | 99 |

ECG Rhythm Strips:

ECG Number 01
  11:41:05 AM

Rate: 69 BPM
Rhythm: Reg.
QRS Width:
    .092 Sec

Specimen:     Premature Atypical QRS

FIG.8B

Patient:                                                                5 Feb 81

ECG Number 10
  1:30:33 PM


Rate:  77 BPM
Rhythm:  Reg.
QRS Width:
     .064 Sec

Specimen:        Premature Typical QRS

ECG Number 11
  1:35:40 PM


Rate:  79 BPM
Rhythm:  Reg.
QRS Width:
     .076 Sec

Specimen:        Premature Atypical QRS

ECG Number 12
  2:01:47 PM


Rate:  80 BPM
Rhythm:  Reg.
QRS Width:
     .080 Sec

Specimen:        Premature Atypical QRS

ECG Number 13
  9:10:03 PM


Rate: 101 BPM
Rhythm:  Reg.
QRS Width:
     .068 Sec

Specimen:        Rate:  100 to 150 BPM

# FIG.8C-1

Patient: Samuel Rose                    5 Feb 81        Page 7

ECG Rhythm Strips (continued) :

ECG Number 18
  5:12:56 AM

Rate:  84 BPM
Rhythm:  Reg.
QRS Width:
    .076 Sec

Specimen:    Triplet, Atypical QRS

ECG Number 24
  9:42:15 AM

Rate:  70 BPM
Rhythm:  Reg.
QRS Width:
    .076 Sec

Specimen:    Missed Beat(s)

# FIG.8C-2

Patient:                                              5 Feb 81

---

Phenomena Summary:

QRS Rate -- 8 Beat Average

    Bradycardia at Rates:
        between 50 and 60 BPM

    Normal at Rates between 60 and 100 BPM

    Tachycardia at Rates:
        between 100 and 150 BPM

Single QRS Shape and R-R Interval

        461 Premature Typical QRS Complexes
           20 Occurred at a Rate of 6/Minute or Greater

        206 Premature Atypical QRS Complexes

        13 Pause(s)

        3 Missed Beat(s)

QRS Sequences

        1 Episodes of Bigeminy with Typical QRS Complexes

        5 Episodes of Bigeminy with Atypical QRS Complexes

        5 Episodes of Couplets with Atypical QRS Complexes

        1 Episodes of Triplets with Atypical QRS Complexes

Major QRS Pattern Changes

        2 Episodes of Major Pattern Changes in QRS Complexes

---

Major QRS Pattern Change Log:

| Log Number | Onset Time of Day | Duration in Secs. | Rate (BPM) Bef | Aft | Rhythm Bef | Aft | Width (Ms) Bef | Aft | ECG Number |
|---|---|---|---|---|---|---|---|---|---|
| 1 | 4:37:58 AM | | 84 ** | 128 | Reg | Reg | 64 | 68 | |
| 2 | 4:38:08 AM | | 135 ** | 76 | Reg | Reg | 68 | 72 | |

FIG.8D

Patient:

5 Feb 81

Hourly Summaries:

Bigeminy, Typical QRS    Total    1
(See ECG Number 21)

Bigeminy, Atypical QRS    Total    5
(See ECG Number 16)

Couplet, Atypical QRS    Total    5

(No ECG Rhythm Strip Available)

Triplet, Atypical QRS    Total    1
(See ECG Number 18)

FIG.8E

Patient: 5 Feb 81

## Rate Summary

Maximum    Average    Minimum

## Premature Atypical QRS Summary

Count per 10 Minutes
(* = Rate > 6 per Minute)

## Premature Typical QRS Summary

Count per 10 Minutes
(* = Rate > 6 per Minute)

**FIG.8F**

Patient:                                                     5 Feb 81

## S-T Deflection Summary

Maximum    Average    Minimum

## Noise/Artifact

Percent of Summary Period Loss Due to Noise or Artifact

## Loss of System Function

Percent of Summary Period Loss Due to Disconnected Leads

**FIG.8G**

Patient:                                      5 Feb 81

Summary Period Log:

| Start Time HH:MM | Rate Min Avg Max (BPM) | | | Prem Atyp QRS | Prem Typ QRS | S-T Deflection Min Avg Max (Millivolts) | | | # of Rhym Strp | # of Majr Ptrn | Noise /Arti MM:SS | Lead-less MM:SS |
|---|---|---|---|---|---|---|---|---|---|---|---|---|
| 11:20 | 66 | 71 | 73 | 0 | 0 | -.18 | -.18 | -.16 | 0 | 0 | 0:03 | 9:03 |
| 11:30 | 63 | 69 | 72 | 0 | 0 | -.20 | -.18 | -.14 | 0 | 0 | 0:00 | 0:00 |
| 11:40 | 63 | 69 | 74 | 1 | 1 | -.20 | -.16 | -.14 | 2 | 0 | 0:00 | 0:00 |
| 11:50 | 62 | 67 | 75 | 0 | 1 | -.18 | -.16 | -.14 | 1 | 0 | 0:00 | 0:00 |
| 12:00 | 67 | 73 | 78 | 1 | 1 | -.24 | -.18 | -.14 | 2 | 0 | 0:00 | 0:00 |
| 12:10 | 66 | 69 | 72 | 0 | 0 | -.18 | -.16 | -.14 | 0 | 0 | 0:00 | 0:00 |
| 12:20 | 63 | 68 | 74 | 1 | 3 | -.18 | -.18 | -.14 | 2 | 0 | 0:00 | 0:00 |
| 12:30 | 67 | 72 | 76 | 1 | 0 | -.20 | -.18 | -.16 | 1 | 0 | 0:00 | 0:00 |
| 12:40 | 68 | 73 | 77 | 1 | 2 | -.18 | -.18 | -.14 | 0 | 0 | 0:00 | 0:00 |
| 12:50 | 66 | 70 | 75 | 1 | 0 | -.18 | -.16 | -.12 | 0 | 0 | 0:00 | 0:00 |
| 1:00 | 67 | 72 | 75 | 0 | 0 | -.18 | -.16 | -.12 | 0 | 0 | 0:00 | 0:00 |
| 1:10 | 69 | 73 | 77 | 1 | 0 | -.16 | -.16 | -.14 | 1 | 0 | 0:00 | 0:00 |
| 1:20 | 70 | 73 | 77 | 0 | 0 | -.16 | -.16 | -.14 | 0 | 0 | 0:00 | 0:00 |
| 1:30 | 68 | 73 | 79 | 1 | 3 | -.16 | -.16 | -.14 | 2 | 0 | 0:00 | 0:00 |
| 1:40 | 71 | 76 | 82 | 0 | 1 | -.22 | -.18 | -.14 | 0 | 0 | 0:00 | 0:00 |
| 1:50 | 70 | 75 | 82 | 1 | 0 | -.24 | -.20 | -.16 | 0 | 0 | 0:00 | 0:00 |
| 2:00 | 67 | 74 | 81 | 2 | 3 | -.24 | -.20 | -.16 | 1 | 0 | 0:00 | 0:00 |
| 2:10 | 66 | 72 | 76 | 1 | 1 | -.20 | -.18 | -.14 | 0 | 0 | 0:00 | 0:00 |
| 2:20 | 70 | 79 | 85 | 1 | 0 | -.24 | -.20 | -.14 | 0 | 0 | 0:00 | 0:00 |
| 2:30 | 68 | 75 | 83 | 0 | 1 | -.22 | -.18 | -.14 | 0 | 0 | 0:00 | 0:00 |
| 2:40 | 68 | 72 | 77 | 0 | 0 | -.18 | -.16 | -.14 | 0 | 0 | 0:00 | 0:00 |
| 2:50 | 68 | 72 | 82 | 1 | 2 | -.20 | -.18 | -.12 | 0 | 0 | 0:00 | 0:00 |
| 3:00 | 64 | 69 | 75 | 1 | 2 | -.20 | -.18 | -.14 | 0 | 0 | 0:00 | 0:00 |
| 3:10 | 64 | 68 | 72 | 0 | 0 | -.20 | -.20 | -.16 | 0 | 0 | 0:00 | 0:00 |
| 3:20 | 62 | 68 | 74 | 2 | 1 | -.22 | -.20 | -.18 | 0 | 0 | 0:00 | 0:00 |
| 3:30 | 63 | 70 | 72 | 0 | 1 | -.20 | -.20 | -.18 | 0 | 0 | 0:00 | 0:00 |
| 3:40 | 65 | 70 | 73 | 0 | 0 | -.20 | -.20 | -.16 | 0 | 0 | 0:00 | 0:00 |
| 3:50 | 66 | 71 | 74 | 0 | 2 | -.20 | -.20 | -.18 | 0 | 0 | 0:00 | 0:00 |
| 4:00 | 66 | 72 | 74 | 0 | 0 | -.20 | -.20 | -.18 | 0 | 0 | 0:00 | 0:00 |
| 4:10 | 60 | 72 | 83 | 0 | 3 | -.22 | -.20 | -.18 | 0 | 0 | 0:00 | 0:00 |
| 4:20 | 64 | 75 | 83 | 1 | 0 | -.20 | -.20 | -.18 | 0 | 0 | 0:00 | 0:00 |
| 4:30 | 63 | 75 | 82 | 0 | 0 | -.22 | -.20 | -.18 | 0 | 0 | 0:00 | 0:00 |
| 4:40 | 70 | 75 | 78 | 1 | 0 | -.20 | -.20 | -.18 | 0 | 0 | 0:00 | 0:00 |
| 4:50 | 72 | 76 | 83 | 0 | 1 | -.20 | -.20 | -.18 | 0 | 0 | 0:00 | 0:00 |
| 5:00 | 71 | 76 | 81 | 0 | 0 | -.20 | -.20 | -.18 | 0 | 0 | 0:00 | 0:00 |
| 5:10 | 77 | 85 | 93 | 0 | 3 | -.24 | -.20 | -.14 | 0 | 0 | 0:00 | 0:00 |
| 5:20 | 75 | 80 | 95 | 0 | 0 | -.22 | -.20 | -.18 | 0 | 0 | 0:00 | 0:00 |
| 5:30 | 76 | 78 | 81 | 0 | 0 | -.20 | -.20 | -.18 | 0 | 0 | 0:00 | 0:00 |
| 5:40 | 71 | 75 | 81 | 0 | 0 | -.22 | -.20 | -.16 | 0 | 0 | 0:00 | 0:00 |
| 5:50 | 71 | 75 | 80 | 0 | 0 | -.20 | -.20 | -.18 | 0 | 0 | 0:00 | 0:00 |
| 6:00 | 72 | 78 | 84 | 2 | 1 | -.22 | -.20 | -.16 | 0 | 0 | 0:00 | 0:00 |
| 6:10 | 71 | 75 | 78 | 0 | 0 | -.18 | -.18 | -.16 | 0 | 0 | 0:00 | 0:00 |
| 6:20 | 73 | 76 | 79 | 0 | 0 | -.20 | -.18 | -.16 | 0 | 0 | 0:00 | 0:00 |
| 6:30 | 75 | 78 | 82 | 1 | 1 | -.18 | -.18 | -.16 | 0 | 0 | 0:00 | 0:00 |

ETC.

FIG.8H

Patient: 5 Feb 81

R-R Interval Distribution Log:

| R-R Interval (MS) | All QRS Complexes | Premature Atypical Complexes | ·R-R Interval (MS) | All QRS Complexes | Premature Atypical Complexes |
|---|---|---|---|---|---|
| up to 224 | 0 | 0 | 1129-1152 | 46 | 0 |
| 225- 248 | 0 | 0 | 1153-1176 | 21 | 0 |
| 249- 272 | 0 | 0 | 1177-1200 | 13 | 0 |
| 273- 296 | 0 | 0 | 1201-1224 | 8 | 0 |
| 297- 328 | 0 | 0 | 1225-1248 | 11 | 0 |
| 329- 352 | 0 | 0 | 1249-1272 | 4 | 0 |
| 353- 376 | 3 | 0 | 1273-1296 | 7 | 0 |
| 377- 400 | 4 | 0 | 1297-1328 | 3 | 0 |
| 401- 424 | 10 | 3 | 1329-1352 | 4 | 0 |
| 425- 448 | 9 | 2 | 1353-1376 | 1 | 0 |
| 449- 472 | 13 | 4 | 1377-1400 | 4 | 0 |
| 473- 496 | 39 | 12 | 1401-1424 | 0 | 0 |
| 497- 528 | 136 | 49 | 1425-1448 | 5 | 0 |
| 529- 552 | 225 | 75 | 1449-1472 | 1 | 0 |
| 553- 576 | 168 | 27 | 1473-1496 | 1 | 0 |
| 577- 600 | 180 | 11 | 1497-1528 | 0 | 0 |
| 601- 624 | 200 | 6 | 1529-1552 | 0 | 0 |
| 625- 648 | 317 | 6 | 1553-1576 | 1 | 0 |
| 649- 672 | 539 | 4 | 1577-1600 | 0 | 0 |
| 673- 696 | 1818 | 2 | 1601-1624 | 0 | 0 |
| 697- 728 | 4562 | 5 | 1625-1648 | 0 | 0 |
| 729- 752 | 16561 | 4 | 1649-1672 | 0 | 0 |
| 753- 776 | 16630 | 3 | 1673-1696 | 0 | 0 |
| 777- 800 | 18509 | 3 | 1697-1728 | 0 | 0 |
| 801- 824 | 17112 | 0 | 1729-1752 | 0 | 0 |
| 825- 848 | 10035 | 0 | 1753-1776 | 0 | 0 |
| 849- 872 | 6520 | 0 | 1777-1800 | 0 | 0 |
| 873- 896 | 4877 | 0 | 1801-1824 | 0 | 0 |
| 897- 928 | 3133 | 1 | 1825-1288 | 0 | 0 |
| 929- 952 | 1709 | 0 | 1289-1872 | 0 | 0 |
| 953- 976 | 567 | 2 | 1873-1896 | 0 | 0 |
| 977-1000 | 277 | 0 | 1897-1928 | 0 | 0 |
| 1001-1024 | 149 | 0 | 1929-1952 | 0 | 0 |
| 1025-1048 | 114 | 0 | 1953-1976 | 0 | 0 |
| 1049-1072 | 86 | 0 | 1977-2000 | 0 | 0 |
| 1073-1096 | 61 | 0 | 2000 plus | 0 | 0 |
| 1097-1128 | 54 | 0 | | | |

# FIG.8 I

Patient:                                                                    5 Feb 81

R-R Interval Distribution
All QRS Complexes

R-R Interval (Milliseconds)

R-R Interval Distribution
Premature Atypical Complexes

R-R Interval (Milliseconds)

**FIG.8J**

Sample Report Page with QRS Types and Pacer Rhythm Strips   10 Nov 81

QRS Types:

|  | *Fig.9A.* | *Fig.9B.* | *Fig.9C.* | *Fig.9D.* |
|---|---|---|---|---|
| Label: | T1 | T2 | T3 | I1 |

Number
Observed:        43          5          5          237
Probable:         3          0          0            1

ECG Rhythm Strips:

ECG Number 01
  12:39:12 PM

Rate:   84 BPM

*Fig.9E.*

ECG Number 02
  12:39:40 PM

Rate:   84 BPM

*Fig.9F.*

Sample Report Page with Dual Channel Rhythm Strips        10 Nov 81

ECG Rhythm Strips:

ECG Number 01
4:13:29 PM

Lead II

Rate:   85 BPM

Lead AVF

*Fig.9G.*

Bigeminy, Atypical QRS

ECG Number 02
4:14:01 PM

Lead II

Rate:   88 BPM

Lead AVF

*Fig.9H.*

Triplet, Atypical QRS

ECG Number 03
4:17:53 PM

Lead II

Rate:   89 BPM

Lead AVF

*Fig.9I.*

Couplet, Atypical QRS

DATAMEDIX                     County General Hospital
                             Pacemaker Evaluation        10 Nov 81

---

Patient:                                          Age: 69
Address:   1932 N.W. 42nd Street                  Sex: Male
           Orlando, Florida                       Height: 6' 0"
                                                  Weight: 188 lbs.
Telephone:   305-693-1471

Account No.:   415-83-6984              Release:   100-E-04-1-00

Physician:  Francis Nugent, M.D.    PCU Number:   5002400001 000004

---

       Pacemaker Manufacturer:
                        Model:
                Serial Number:
                         Type:  VVI

            Date of Implant:  12 Oct 79
         Time Since Implant:  25 Months
            Initial Pace Rate:  84    BPM

   Date of Last Clinic Visit:  2 Aug 80

---

Interpretation:

Recommendation:

*Fig. 9J.*

---

Patient: John C. Shoemaker          10 Nov 81          Page 2

**Fig.9K.**

.2 Millisecond per Major Div.
          Approximate Sensitivity:

**Fig.9L.**

1 Millisecond per Major Div.
.1 Volts per Major Division

ECG Lead II                                    Rate:  73 BPM

**Fig.9M.**

Approximate Sensitivity:   .05 Millivolts per Division
Time Scale:  5 Major Divisions per Second

| | | |
|---|---|---|
| Pulse Interval: | 714.3 | Milliseconds |
| Time Constant: | 3.03 | Milliseconds |
| Pulse Height: | .49 | Volts |
| Pulse Width: | .795 | Milliseconds |
| Pulse Rate: | 84 | BPM |

**Fig. 9N.**